(19) Europäisches Patentamt
European Patent Office
Office européen des brevets

(11) EP 3 334 828 B1

(12) EUROPEAN PATENT SPECIFICATION

(45) Date of publication and mention of the grant of the patent:
**28.10.2020 Bulletin 2020/44**

(21) Application number: **16750759.9**

(22) Date of filing: **04.08.2016**

(51) Int Cl.:
***C12N 15/63*** *(2006.01)*

(86) International application number:
**PCT/EP2016/068702**

(87) International publication number:
**WO 2017/025447 (16.02.2017 Gazette 2017/07)**

# (54) METHOD OF INCREASING THE REPLICATION OF A CIRCULAR DNA MOLECULE

VERFAHREN ZUR ERHÖHUNG DER REPLIKATION EINES ZIRKULÄREN DNA-MOLEKÜLS

PROCÉDÉ D'AUGMENTATION DE LA RÉPLICATION D'UNE MOLÉCULE D'ADN CIRCULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.08.2015 PCT/EP2015/068398**

(43) Date of publication of application:
**20.06.2018 Bulletin 2018/25**

(73) Proprietor: **CureVac Real Estate GmbH**
**72076 Tübingen (DE)**

(72) Inventor: **WILLIAMS, Jim**
**72076 Tübingen (DE)**

(74) Representative: **Lasar, Andrea Gisela**
**Maiwald Patentanwalts- und Rechtsanwaltsgesellschaft mbH**
**Elisenhof**
**Elisenstraße 3**
**80335 München (DE)**

(56) References cited:
**CN-A- 104 278 055**

- **DUBIN MANU J ET AL: "A modified Gateway cloning strategy for overexpressing tagged proteins in plants", PLANT METHODS, BIOMED CENTRAL, LONDON, GB, vol. 4, no. 1, 22 January 2008 (2008-01-22), page 3, XP021039381, ISSN: 1746-4811**
- **"pCMV6-Entry Vector (Myc-DDK Tagged)", Amsbio , October 2009 (2009-10), XP002763069, Retrieved from the Internet: URL:http://www.amsbio.com/datasheets/pCMV6-Entry%20vector.pdf**
- **ZIBERT A ET AL: "INFECTIOUS FOOT-AND-MOUTH DISEASE VIRUS DERIVED FROM A CLONED FULL-LENGTH COMPLEMENTARY DNA", JOURNAL OF VIROLOGY, vol. 64, no. 6, 1990, pages 2467-2473, XP002763070, ISSN: 0022-538X**
- **LAHIJANI R ET AL: "High-yield production of pBR322-derived plasmids intended for human gene therapy by employing a temperature-controllable point mutation", HUMAN GENE THERAPY, MARY ANN LIEBERT, NEW YORK ,NY, US, vol. 7, no. 7, 20 October 1996 (1996-10-20), pages 1971-1980, XP002120738, ISSN: 1043-0342**
- **ANDREW G. BERT ET AL: "Generation of an Improved Luciferase Reporter Gene Plasmid That Employs a Novel Mechanism for High-Copy Replication", PLASMID, vol. 44, no. 2, 1 September 2000 (2000-09-01), pages 173-182, XP055223651, US ISSN: 0147-619X, DOI: 10.1006/plas.2000.1474**
- **GRABHERR R ET AL: "Impact of targeted vector design on ColE1 plasmid replication", TRENDS IN BIOTECHNOLOGY, ELSEVIER PUBLICATIONS, CAMBRIDGE, GB, vol. 20, no. 6, 1 June 2002 (2002-06-01), pages 257-260, XP004352764, ISSN: 0167-7799, DOI: 10.1016/S0167-7799(02)01950-9**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- WELLS J M ET AL: "IMPROVED CLONING VECTORS AND TRANSFORMATION PROCEDURE FOR LACTOCOCCUS LACTIS", JOURNAL OF APPLIED BACTERIOLOGY, BLACKWELL PUBLISHING LTD., OXFORD, GB, vol. 74, no. 6, 1 January 1993 (1993-01-01), pages 629-636, XP008005890, ISSN: 0021-8847
- TOMIZAWA ET AL: "Control of ColE1 plasmid replication: Binding of RNA I to RNA II and inhibition of primer formation", CELL, CELL PRESS, US, vol. 47, no. 1, 10 October 1986 (1986-10-10), pages 89-97, XP023883036, ISSN: 0092-8674, DOI: 10.1016/0092-8674(86)90369-7 [retrieved on 1986-10-10]
- Susan E Conrad ET AL: "volume 6 Number 101979 Nucleic Acids Research Characterization of an improved in vitro DNA replication system for Escherichia coli plasmids", , 1 January 1979 (1979-01-01), XP055223462, Retrieved from the Internet: URL:http://nar.oxfordjournals.org/content/6/10/3289.full.pdf [retrieved on 2015-10-26]
- SOLAR DEL G ET AL: "Replication and control of circular bacterial plasmids", MICROBIOLOGY AND MOLECULAR BIOLOGY REVIEWS, AMERICAN SOCIETY FOR MICROBIOLOGY, US, vol. 62, no. 2, 1 June 1998 (1998-06-01), pages 434-464, XP002231301, ISSN: 1092-2172
- Addgene: "Addgene: pCRII-TOPO CMV-cGFP-bGH Poly(A) Sense", , 1 January 2012 (2012-01-01), XP055599908, Retrieved from the Internet: URL:http://www.addgene.org/46835/ [retrieved on 2019-06-26]
- Addgene: "Addgene pCRII-TOPO CMV-cGFP-bGH Poly(A) Sense - Sequence Analyzer", , 1 January 2012 (2012-01-01), XP055599859, Retrieved from the Internet: URL:https://www.addgene.org/browse/sequence/66680/ [retrieved on 2019-06-26]
- J. E. Wilusz ET AL: "A triple helix stabilizes the 3' ends of long noncoding RNAs that lack poly(A) tails", Genes & Development, vol. 26, no. 21, 16 October 2012 (2012-10-16), pages 2392-2407, XP055194808, ISSN: 0890-9369, DOI: 10.1101/gad.204438.112

## Description

### Field of the invention

The present invention relates to a covalently closed circular recombinant DNA molecule comprising a bacterial origin of replication, a promoter for a DNA-dependent RNA polemerase and an insert comprising a homopolymeric region, wherein the homopolymeric region comprises at least one homopolymeric sequence comprising a stretch of at least 15 identical nucleotides and is located at a distance of least 500 bp from the bacterial origin of replication in the direction of replication, wherein the insert comprising a homopolymeric region is oriented so that the direction of transcription of the insert is the same as the direction of replication of the origin of replication. The invention further relates to the use of the covalently closed circular recombinant DNA molecule for increasing the yield during fermentation.

### Background of the invention

In gene therapy and many other therapeutically relevant biochemical and biotechnological applications the use of nucleic acids for therapeutic and diagnostic purposes is of major importance. As an example, rapid progress has occurred in recent years in the field of gene therapy and promising results have been achieved. Nucleic acids are therefore regarded as important tools for gene therapy and prophylactic and therapeutic vaccination against infectious and malignant diseases. For example messenger RNA amplified from tumor cells was transfected into dendritic cells to induce tumor immunity (Boczkowski et al. (2000) Cancer Research 60, 1028-1034).

Therapeutic ribonucleic acid (RNA) molecules represent a promising class of drugs. RNA-based therapeutics include mRNA molecules encoding antigens for use as vaccines (Fotin-Mleczek et al. (2012) J. Gene Med. 14(6): 428-439). In addition, it is envisioned to use RNA molecules for replacement therapies, e.g. for providing missing proteins such as growth factors or enzymes to patients (Kariko et al. (2012) Mol. Ther. 20(5): 948-953; Kormann et al. (2012) Nat. Biotechnol. 29(2): 154-157). Furthermore, the therapeutic use of non-coding immunostimulatory RNA molecules (Heidenreich et al. Int J Cancer. 2014 Dec 21. doi: 10.1002/ijc.29402.) and other non-coding RNAs such as microRNAs and long non-coding RNAs is considered (Esteller (2011) Nat. Rev. Genet. 15(12): 861-74).

RNA-based therapeutics exhibit some superior properties over DNA cell transfection. As generally known, transfection of DNA molecules may lead to serious problems. E.g. application of DNA molecules bears the risk that the DNA integrates into the host genome. Integration of foreign DNA into the host genome can have an influence on expression of the host genes and possibly triggers expression of an oncogene or destruction of a tumor suppressor gene. Furthermore, a gene - and therefore the gene product - which is essential to the host may be inactivated by integration of the foreign DNA into the coding region of this gene. There may be a particular danger if integration of the DNA takes place into a gene which is involved in regulation of cell growth. Nevertheless, DNA still represents an important tool, even though some risks are associated with the application of DNA. These risks do not occur, if RNA, particularly mRNA, is used instead of DNA. An advantage of using RNA rather than DNA is that no virus-derived promoter element has to be administered *in vivo* and no integration into the genome may occur. Furthermore, the RNA does not have to cross the barrier to the nucleus.

In gene therapy approaches, typically DNA is used even though RNA is also known in recent developments. Importantly, in all these gene therapy approaches mRNA functions as messenger for the sequence information of the encoded protein, irrespectively if DNA, viral RNA or mRNA is used. In general RNA is considered an unstable molecule: RNases are ubiquitous and notoriously difficult to inactivate. Furthermore, RNA is also chemically more labile than DNA. Thus, it is perhaps surprising that the "default state" of an mRNA in a eukaryotic cell is characterized by a relative stability and specific signals are required to accelerate the decay of individual mRNAs. The main reason for this finding appears to be that mRNA decay within cells is catalyzed almost exclusively by exonucleases. However, the ends of eukaryotic mRNAs are protected against these enzymes by specific terminal structures and their associated proteins: a m7GpppN CAP at the 5' end and typically a poly(A) sequence at the 3' end. Removal of these two terminal modifications is thus considered rate limiting for mRNA decay. Although a stabilizing element has been characterized in the 3' UTR of the alpha-globin mRNA, RNA sequences affecting turnover of eukaryotic mRNAs typically act as a promoter of decay usually by accelerating deadenylation (reviewed in Meyer, S., C. Temme, et *a/*. (2004), Crit Rev Biochem Mol Biol 39(4): 197-126.).

Dubin et al., "A modified gateway cloning strategy for overexpressing tagged proteins in plants", Plant methods, Biomed Central, London, GB, Vol. 4, No 1, 22 January 2008 discloses the adaptation of recombinant cloning methods including the Gateway system to plant transformation vectors to facilitate the creation of overexpression, tagging and silencing vectors on a large scale.

Amsbio markets the pCMV6-entry vector (My-DDK tagged) with the details indicated in the corresponding datasheet.

Zibert et al., "Infectious foot-and-mouth disease virus derived from a cloned full-length cDNA", Journal of virology, Vol 64., No. 6, 1990 discloses the construction of a full-length cDNA plasmid of foot-and-mouth disease virus.

Lahijani et al., "High-yield production of pBR322-derived plasmids intended for human gene therapy by employing a termperature-controllable point mutation", Human gene therapy, May Ann Liebert, New York, NY, US, Vol. 7, No. 7, 20 October 1996 discloses the conversion of a low-copy-number pBR322-derived plasmid (VCL1005) to a high-copy-number plasmid (VCL1005/A).

Bert et al., "Generation of an improved luciferase reporter gene plasmid that employs a novel mechanism for high-copy replication", Plasmid, Vol. 44, No. 2, 1 September 2000 discloses the engineering of the reporter gene plasmid pXPG by incorporating a novel high-copy origin of replication and a modified luciferase gene into a pXP1-derived vector.

Grabherr and Bayer, "Impact of targeted vector design on Co/E1 plasmid replication", Trends in biotechnology, Elsevier publications, Cambridge, GB, Vol. 20, No. 6, 1 June 2002 discloses general information on high copy number plasmids when used in particular for the recombinant expression of proteins.

Wells et al., "Improved cloning vectors and transformation procedure for Lactococcus lactis", Journal of applied bacteriology, Blackwell Publishing LTD. Vol. 74, No. 6, 1 January 1993 discloses the cloning the E.coli pUC replicon into specific shuttle vectors in order to achieve a broad host range.

Tomizawa, "Control of ColEl plasmid replication: binding of RNA I to RNA II and inhibition of primer formation", Cell, Vol. 47, No. 1, 10 October 1986 discloses that the binding rate of RNA I to RNA II is crucial to regulation, and thus plasmid replication control.

Conrad and Campbell, "Characterization of an improved in vitro DNA replication system for Escherichia coli plasmids", Nucleic Acids Research, Vol. 6, No. 10, 1 January 1979 discloses the characterization of a modified *in vitro* replication system, where endogenous DNA is removed.

Solar et al., "Replication and control of circular bacterial plasmids", Microbiology and molecular biology reviews, Vol. 62, No. 2, 1 June 1998 discloses general information on bacterial plasmids.

For the manufacture of nucleotide-based therapeutics the efficient production of plasmid vectors is required. However, the present inventors have found that homopolymeric sequences are deteriorating the replication process leading to decreased replication speed or even interruption of the replication process. As a consequence, the yield of fermentative production of plasmids comprising homopolymeric sequences is decreased compared to the plasmids without such sequences. Furthermore, homopolymeric sequences are often at least partially deleted during fermentative production of the plasmids.

Hence, there is a need for efficient replication of vectors comprising homopolymeric sequences with high fidelity.

## Summary of the invention

A solution to these problems is the provision of covalently closed circular recombinant DNA molecules in which the homopolymeric sequence comprising a stretch of at least 15 identical nulceotides is not located directly next to the origin of replication, in the present invention a bacterial origin of replication, in the direction of replication, but in which there is a certain distance, namely a distance of at least 500 bp, between the homopolymeric sequence and the origin of replication.

Accordingly, the present invention relates to a covalently closed circular recombinant DNA molecule comprising:

- a bacterial origin of replication,
- a promoter for a DNA-dependent RNA polymerase,
- an insert comprising a homopolymeric region,

wherein the homopolymeric region comprises at least one homopolymeric sequence comprising a stretch of at least 15 identical nucleotides and is located at a distance of least 500 bp from the bacterial origin of replication in the direction of replication, wherein the insert comprising the homopolymeric region is oriented so that the direction of transcription of the insert is the same as the direction of replication of the origin of replication.

The covalently closed circular recombinant DNA molecule in which the homopolymeric region is located at a distance of least 500 bp from the origin of replication in the direction of replication may improve the yield of the covalently closed circular recombinant DNA molecule in fermentative production compared to the yield of a covalently closed circular recombinant DNA molecule in which the homopolymeric region is located at a distance of less than 500 bp from the origin of replication in the direction of replication.

Further, the covalently closed circular recombinant DNA molecule in which the insert comprising a homopolymeric region is oriented so that the direction of transcription of the insert is the same as the direction of replication of the origin of replication improves the yield of the covalently closed circular recombinant DNA molecule in fermentative production compared to the yield of a covalently closed circular recombinant DNA molecule in which the insert comprising the homopolymeric region is oriented so that the direction of transcription of the insert is opposite to the direction of replication of the origin of replication.

The covalently closed circular recombinant DNA molecule may be a plasmid, cosmid, bacterial artificial chromosome (BAC), bacteriophage, viral vector or hybrids thereof. Preferably, the covalently closed circular recombinant DNA molecule is a plasmid.

The origin of replication is of bacterial origin. Preferably, the origin of replication is a high copy number origin. The origin of replication may be derived from the pBR322 plasmid, pUC plasmid, pMB1 plasmid, ColE1 plasmid, R6K plasmid, p15A plasmid or pSC101 plasmid. Preferably, the origin of replication is derived from the pUC plasmid.

In some embodiments, the covalently closed circular recombinant DNA molecule further comprises a primosome assembly site in the heavy strand (PAS-BH).

Typically, the covalently closed circular recombinant DNA molecule further comprises a selection marker, for example an antibiotic resistance gene or a sucrose selectable marker.

In preferred embodiments the homopolymeric region comprised in the insert of the covalently closed circular recombinant DNA molecule comprises a poly(A) sequence and/or a poly(C) sequence. In more preferred embodiments the homopolymeric region comprises a poly(C) sequence. In even more preferred embodiments the homopolymeric region comprises a poly(C) and a poly(A) sequence. Preferably, the poly(A) sequence comprises a sequence of about 20 to about 400 adenosine nucleotides more preferably of about 60 to about 250 adenosine nucleotides. Preferably, the poly(C) sequence comprises a sequence of about 20 to 40 cytidine nucleotides. In particularly preferred embodiments the homopolymeric region comprises a poly(A) sequence of about 60 to about 70 adenosine nucleotides and a poly(C) sequence of about 20 to 30 cytidine nucleotides.

A further aspect of the invention refers to the use of a covalently closed circular recombinant DNA molecule of the present invention for increasing the yield of the covalently closed circular recombinant DNA in fermentative production compared to the yield of a covalently closed circular recombinant DNA molecule in which the insert comprising the homopolymeric region is located at a distance of less than 500 bp from the origin of replication in the direction of replication.

Still a further aspect of the invention refers to a covalently closed circular recombinant DNA molecule of the present invention for increasing the yield of the covalently closed circular recombinant DNA in fermentative production compared to the yield of a covalently closed circular recombinant DNA molecule in which the insert comprising the homopolymeric region is oriented so that the direction of transcription of the insert is opposite to the direction of replication of the origin of replication.

When the closed circular recombinant DNA molecule is used in which the homopolymeric region is located at a distance of least 500 bp from the origin of replication in the direction of replication, the fermentation time may be shortened compared to the fermentation time of a covalently closed circular recombinant DNA molecule in which the insert comprising the homopolymeric region is located at a distance of less than 500 bp from the origin of replication in the direction of replication.

When the closed circular recombinant DNA molecule is used in which the insert comprising a homopolymeric region is oriented so that the direction of transcription of the insert is the same as the direction of replication of the origin of replication, the fermentation time is shortened compared to the fermentation time of a covalently closed circular recombinant DNA molecule in which the insert comprising the homopolymeric region is oriented so that the direction of transcription of the insert is opposite to the direction of replication of the origin of replication.

A further aspect of the invention refers to a method for fermentative production of a covalently closed recombinant DNA molecule comprising the steps of:

(a) providing a microorganism comprising the covalently closed circular recombinant DNA molecule according to the invention;
(b) fermenting the microorganism of step (a).

In a specific embodiment, the method further comprises the following step:
(c) isolating the covalently closed circular recombinant DNA molecule from the microorganism of step (b).

In particularly preferred embodiments the microorganism is a bacterium, preferably E. coli. In preferred embodiments the microorganism is a bacterium (e.g. E. coli) containing a covalently closed circular recombinant temperature inducible high copy DNA plasmid and step (b) comprises the following steps:

(i) growing the bacteria containing a covalently closed circular recombinant temperature inducible high copy DNA plasmid at a temperature in the range of 25°C to 32°C during the growth phase of the fed-batch phase wherein the substrate is supplied at a rate such that the growth rate is $\mu$=0.05 to 0.3$hr^{-1}$ during the fed-batch phase,
(ii) inducing production of said DNA plasmid after the growth phase by increasing the temperature to 36°C to 45°C; and
(iii) continuing fermentation at the temperature applied (ii) to accumulate the said DNA plasmid.

In a more specific embodiment, the temperature applied in step (i) is about 30°C and the temperature applied in step (ii) is about 42°C.

In the above described methods, the yield of the covalently closed circular recombinant DNA molecule in which

the homopolymeric region is located at a distance of least 500 bp from the origin of replication in the direction of replication is increased compared to the yield of a covalently closed circular recombinant DNA molecule in which the insert comprising the homopolymeric region is located at a distance of less than 500 bp from the origin of replication in the direction of replication.

The yield of the covalently closed circular recombinant DNA in which the insert comprising a homopolymeric region is oriented so that the direction of transcription of the insert is the same as the direction of replication of the origin of replication is increased compared to the yield of a covalently closed circular recombinant DNA molecule in which the insert comprising the homopolymeric region is oriented so that the direction of transcription of the insert is opposite to the direction of replication of the origin of replication. Moreover, the fermentation time is shortened, compared to the fermentation time of a covalently closed circular recombinant DNA molecule in which the insert comprising the homopolymeric region is oriented so that the direction of transcription of the insert is opposite to the direction of replication of the origin of replication.

In an embodiment of the above method, the sequence of the covalently closed circular recombinant DNA molecule remains stable during fermentative production.For example, the homopolymeric region remains stable during fermentative production.

Another aspect of the invention refers to a method for improving the yield of a covalently closed circular recombinant DNA molecule comprising the following steps:

(a) providing a covalently closed circular recombinant DNA molecule having

- a bacterial origin of replication, and
- an insert comprising a homopolymeric region, wherein the homopolymeric region comprises at least one homoplymeric sequence comprising a stretch of at least 15 nucleotides and is located at a distance of less than 500 bp from the origin of replication in the direction of replication;

(b) modifying the covalently closed circular recombinant DNA molecule of (a) so that the homopolymeric region is located at a distance of at least 500 bp from the origin of replication in the direction of replication.

The invention further provides a method for improving the yield of a covalently closed circular recombinant DNA molecule comprising the following steps:

(a) providing a covalently closed circular recombinant DNA molecule having

- a bacterial origin of replication, and
- an insert comprising a homopolymeric region comprising at least one homopolymeric sequence comprising a stretch of at least 15 nucleotides, wherein the insert comprising the homopolymeric region is oriented so that the direction of transcription of the insert is opposite to the direction of replication of the origin of replication, and wherein the homopolymeric region is located at a distance of at least 500 bp from the bacterial origin of replication in the direction of replication;

(b) modifying the covalently closed circular recombinant DNA molecule of (a) so that the insert comprising a homopolymeric region is oriented so that the direction of transcription of the insert is the same as the direction of replication of the origin of replication.

Finally, the invention refers to the use of the covalently closed circular recombinant DNA molecule according to the invention for *in vitro* transcription of RNA.

**Brief description of the figures**

**Figure 1: Vector map of P1140**
The orientation of P1140 is such that the direction of replication of the origin of replication (denoted in the vector map as pUC origin) is opposite to the direction of transcription of the insert (denoted in the vector map as RNA).

**Figure 2: Vector map of P1140-AF2**
The orientation of P1140-AF2 is such that the direction of replication of the origin of replication (denoted in the vector map as pUC origin) is the same as the direction of transcription of the insert (RNA denoted in the vector map as RNA).

**Figure 3: Vector map of P1140-AF1**
The orientation of P1140-AF1 is such that the direction of replication of the origin of replication (denoted in the vector map as pUC origin) is opposite to the direction of transcription of the insert (denoted in the vector map as RNA).

**Figure 4: Vector map of P1140-K2**
The orientation of P1140-K2 is such that the direction of replication of the origin of replication (denoted in the vector map as pUC origin) is the same as the direction of transcription of the insert (RNA denoted in the vector map as RNA).

**Figure 5: Vector map** of **P1140-K1**
The orientation of P1140-K1 is such that the direction of replication of the origin of replication (denoted in the vector map as pUC origin) is opposite to the direction of transcription of the insert (denoted in the vector map as RNA).

**Figure 6:** GC-optimized mRNA encoding H1N1(Netherlands2009)-HA (SEQ ID NO: 2)

## Definitions

For the sake of clarity and readability, the following definitions are provided. Any technical feature mentioned for these definitions may be read on each and every embodiment of the invention. Additional definitions and explanations may be specifically provided in the context of these embodiments. Unless defined otherwise, all technical and scientific terms used herein generally have the same meaning as commonly understood by one of ordinary skill in the art to which this invention belongs. Generally, the nomenclature used herein and the laboratory procedures in cell culture, molecular genetics, organic chemistry, and nucleic acid chemistry and hybridization are those well known and commonly employed in the art. Standard techniques are used for molecular biotechnology assays. The techniques and procedures are generally performed according to conventional methods in the art and various general references (e.g., Sambrook et al., 1989, Molecular Cloning: A Laboratory Manual, 2d ed. Cold Spring Harbor Laboratory Press, Cold Spring Harbor, NY), which are provided throughout this document.

Covalently closed circular recombinant DNA: Covalently closed circular recombinant DNA refers to a circular double-stranded DNA molecule which is intact, *i.e.* wherein both strands are uncut. The molecule preferably does not occur naturally but is engineered, for example by molecular cloning techniques. In the context of the present invention particularly preferred are plasmids as defined herein.

Protein: A protein typically comprises one or more peptides or polypeptides. A protein is typically folded into a 3-dimensional form, which may be required for the protein to exert its biological function. The sequence of a protein or peptide is typically understood to be the order, i.e. the succession of its amino acids.

RNA, mRNA: RNA is the usual abbreviation for ribonucleic acid. It is a nucleic acid molecule, i.e. a polymer consisting of nucleotide monomers. These nucleotides are usually adenosine-monophosphate, uridine-monophosphate, guanosine-monophosphate and cytidine-monophosphate monomers, which are connected to each other along a so-called backbone. The backbone is formed by phosphodiester bonds between the sugar, i.e. ribose, of a first and a phosphate moiety of a second, adjacent monomer. The specific order of the monomers, i.e. the order of the bases linked to the sugar/phosphate-backbone, is called the RNA-sequence. Usually RNA may be obtainable by transcription of a DNA-sequence, e.g., inside a cell. In eukaryotic cells, transcription is typically performed inside the nucleus or the mitochondria. In vivo, transcription of DNA usually results in the so-called premature RNA, which has to be processed into so-called messenger-RNA, usually abbreviated as mRNA. Processing of the premature RNA, e.g. in eukaryotic organisms, comprises a variety of different posttranscriptional-modifications such as splicing, 5'-capping, polyadenylation, export from the nucleus or the mitochondria and the like. The sum of these processes is also called maturation of RNA. The mature messenger RNA usually provides the nucleotide sequence that may be translated into an amino acid sequence of a particular peptide or protein. Typically, a mature mRNA comprises a 5'-cap, optionally a 5'UTR, an open reading frame, optionally a 3'UTR and a poly(A) sequence. Aside from messenger RNA, several non-coding types of RNA exist which may be involved in regulation of transcription and/or translation, and immunostimulation. The term "RNA" further encompass other coding RNA molecules, such as viral RNA, retroviral RNA and replicon RNA, small interfering RNA (siRNA), antisense RNA, CRISPR RNA, ribozymes, aptamers, riboswitches, immunostimulating RNA, transfer RNA (tRNA), ribosomal RNA (rRNA), small nuclear RNA (snRNA), small nucleolar RNA (snoRNA), microRNA (miRNA), and Piwi-interacting RNA (piRNA). In the context of the present invention coding RNAs such as mRNA, viral RNA, retroviral RNA and replicon RNA is particularly preferred.

The coding region of the mRNA for use according to the present invention may occur as a mono-, di-, or even multicistronic mRNA, i.e. an mRNA, which carries the coding sequences of one, two or more proteins or peptides. Such coding sequences in di-, or even multicistronic mRNA's may be separated by at least one internal ribosome entry site (IRES) sequence, e.g. as described herein or by signal peptides which induce the cleavage of the resulting polypeptide, which comprises several proteins or peptides.

bicistronic/multicistronic RNA: The term "bicistronic" or "multicistronic" RNA as used herein is typically RNA, preferably an mRNA, that typically has two (bicistronic) or more (multicistronic) open reading frames (ORF). An open reading frame in this context is a sequence of codons that is translatable into a peptide or protein.

small interfering RNA (siRNA): "small interfering RNA" (siRNA) refers to double-stranded RNA molecules which are from about 10 to about 30 nucleotides long and which are named for their ability to specifically interfere with protein expression. Preferably, siRNA molecules are 12-28 nucleotides long, more preferably 15-25 nucleotides long, still more preferably 19-23 nucleotides long and most preferably 21-23 nucleotides long. Therefore, preferred siRNA molecules are 12, 13, 14, 15, 16, 17, 18, 19, 20, 21, 22, 23, 24, 25, 26, 27 28 or 29 nucleotides in length.

antisense RNA: The term "antisense RNA" includes any RNA molecule which is suitable as an antisense RNA to regions of RNA, preferably mRNA, and most preferably to regulatory elements thereof, and which is capable of causing inhibition of gene expression by complementary binding to these regions. The antisense RNA may also comprise DNA sequences.

CRISPR RNA: The term "CRISPR RNA" is derived from CRISPRs (Clustered Regularly Interspaced Short Palindromic Repeats) evolved in bacteria as an adaptive immune system to defend against viral attack. "Clustered Regularly Interspaced Short Palindromic Repeats" or "CRISPRs", as used herein refers to loci containing multiple short direct repeats that are found in the genomes of approximately 40% of sequenced bacteria and 90% of sequenced archaea. The CRISPR system is a microbial nuclease system involved in defense against invading phages and plasmids that provides a form of acquired immunity. Upon exposure to a virus, short segments of viral DNA are integrated into the CRISPR locus. "CRISPR RNA" is transcribed from a portion of the CRISPR locus that includes the viral sequence. That RNA, which contains sequence complementary to the viral genome, mediates targeting of a Cas9 protein to the sequence in the viral genome. The Cas9 protein cleaves and thereby silences the viral target. CRISPR clusters are transcribed and processed into mature CRISPR (Clustered Regularly Interspaced Short Palindromic Repeats) RNA (CRISPR RNA).

Ribozymes: "Ribozymes" are typically RNA molecules having an enzymatic activity which is able to repeatedly cleave other separate RNA target molecules in a nucleotide base sequence specific manner, blocking their expression and affecting normal functions of other genes. Ribozymes can be targeted to virtually any RNA transcript, and achieve efficient cleavage in vitro.

Aptamers: The term "aptamers" is understood herein to refer to nucleic acid molecules having specific binding affinity to molecules through interactions other than classic Watson-Crick base pairing. The term "RNA aptamer" as used herein is an aptamer comprising ribonucleoside units. Aptamers, preferably RNA aptamers, are capable of specifically binding to selected targets and modulating the target's activity. Aptamers have a number of desirable characteristics for use as therapeutics and diagnostics including high specificity and affinity, biological efficacy, and excellent pharmacokinetic properties.

Riboswitches are RNA elements that reside in the 5' untranslated region (UTR) of genes and modulate their expression using either transcriptional or translational mechanisms (Roth and Breaker (2009) Annu Rev Biochem 78: 305-334).

small nuclear RNA (snRNA): The phrase "small nuclear RNA" (snRNA) refers to small RNA molecules which are synthesized and/or function in the nucleoplasm and/or the nucleolus of the cell.

small nucleolar RNAs (snoRNAs): "small nucleolar RNAs" (snoRNAs) are non-coding RNAs involved in RNA processing. There are two major subclasses of snoRNAs, termed box C/D and box H/ACA snoRNAs. These two classes contain guide sequences that are known to canonically pair with complementary regions on a target pre-rRNA, forming a RNA duplex and facilitating the enzymatic activity of methylases and uridylases that site-specifically modify pre-rRNA bases by either 2'-0-methylation or pseudouridylation, respectively. These modifications of the rRNA are critical to ribosome assembly and viability.

Micro RNAs (miRNAs): "Micro RNAs" (miRNAs) play an important role in regulating gene activity. Micro-RNAs are single-stranded RNAs of typically 22-nucleotides that are processed from about 70 nucleotide hairpin RNA precursors by the RNase III nuclease. Similar to siRNAs, miRNAs can silence gene activity through destruction of homologous mRNA in plants or blocking its translation in plants and animals. These 20-22 nucleotide non-coding RNAs have the ability to hybridize via base-pairing with specific target mRNAs and downregulate the expression of these transcripts, by mediating either RNA cleavage or translational repression. Recent studies have indicated that miRNAs have important functions during development. Further, miRNAs are transcribed by RNA polymerase 11 as polyadenylated and capped messages known as pri-miRNAs.

Piwi-interacting RNA (piRNA): "Piwi-interacting RNA" (piRNA) is a class of small non-coding RNA molecules that is expressed in, or can be introduced into animal cells (see, e.g., Seto et al. (2007) Molecular Cell, 26(5): 603-609; Siomi et al. (2011) Nat. Rev. Mol. Cell. Biol., 12:246-258). piRNAs form RNA-protein complexes through interactions with piwi proteins. These piRNA complexes have been linked to both epigenetic and post-transcriptional gene silencing of retrotransposons and other genetic elements.

Immunostimulating RNA: The terms "immunstimulating RNA" or "immunostimulatory RNA" (isRNA) in the context of the invention may typically be a RNA that is able to induce an innate immune response. An isRNA usually does

not have an open reading frame and thus does not provide a peptide-antigen, but elicits an innate immune response, e.g. by binding to pathogen-associated molecular patterns (PAMP) receptors (e.g. Toll-like-receptor (TLR) or other intracellular RNA sensors (e.g. RIG-I, MDA-5 or PKR).

Homopolymeric sequence: A homopolymeric sequence comprises a stretch of identical nucleotides, such a sequence of adenine nucleotides, a sequence of cytosine nucleotides, a sequence of guanine nucleotides, a sequence of thymine nucleotides, a sequence of identical modified nucleotides or a sequence of identical nucleotide analogs. The homopolymeric sequence comprises a stretch of at least 15, 20, 25, 30, 35, 40, 45, 50, or 60 identical nucleotides, e.g. a poly(A) sequence comprising at least 60 adenine nucleotides and/or a poly(C) sequence comprising at least 30 cytidine nucleotides.

Homopolymeric region: A homopolymeric region comprises at least one homopolymeric sequence. Preferably, the homopolymeric region comprises at least two homopolymeric sequences. The homopolymeric region may comprise at least one homopolymeric sequence selected from the group consisting of a poly(A) sequence, a poly(T) sequence, a poly(C)sequence or a poly(G)sequence or combinations thereof. Preferably the homopolymeric region may comprise a poly(A) sequence and a homopolymeric sequence selected from the group consisting of a poly(T) sequence, a poly(C) sequence or a poly(G) sequence. More preferably the homopolymeric region may comprise a poly(A) sequence and/or a poly(C) sequence. The homopolymeric sequences may be connected by a linker sequence which might be a heteropolymeric sequence. Therefore, the homopolymeric region may comprise different nucleotides, i.e. be a heteropolymeric sequence. Most preferably the homopolymeric region may contain a poly(A) sequence and a poly(C) sequence. In general, the homopolymeric region is a sequence of nucleotides, e.g., of up to about 1000 nucleotides, e.g. from about 20 to about 800, preferably from about 50 to about 600, more preferably from about 50 to about 500, even more preferably from about 50 to about 300, most preferably from about 60 to about 250 nucleotides, which is preferably located 3' of the open reading frame optionally comprised in the insert of the covalently closed circular recombinant DNA molecule according to the invention.

Poly(N) sequence: A poly(N) sequence, also called poly(N) tail or 3'-poly(N) tail, is usually understood to be a sequence of nucleotides, e.g., of up to about 400 nucleotides, e.g. from about 20 to about 400, preferably from about 50 to about 400, more preferably from about 50 to about 300, even more preferably from about 50 to about 250, most preferably from about 60 to about 250 nucleotides, which is preferably added to the 3'-terminus of an RNA, in particular mRNA. A poly(N) sequence is typically located at the 3'-end of an (m)RNA. In the context of the present invention, a poly(N) sequence may be located within an (m)RNA or any other nucleic acid molecule, such as, e.g., in a vector, for example, in a vector serving as template for the generation of an RNA, preferably an mRNA, e.g., by transcription of the vector. The nucleotides may be adenosine, cytidine, uridine, guanosine, modified nucleotides, nucleotide analogs and mixtures thereof.

Poly(A) sequence: A poly(A) sequence, also called poly(A) tail or 3'-poly(A) tail, is usually understood to be a sequence of adenine nucleotides, e.g., of up to about 400 adenine nucleotides, e.g. from about 20 to about 400, preferably from about 50 to about 400, more preferably from about 50 to about 300, even more preferably from about 50 to about 250, most preferably from about 60 to about 250 adenine nucleotides, which is preferably added to the 3'-terminus of a construct coding for a mRNA. A poly(A) sequence is typically located at the 3'-end of an RNA, in particular mRNA. In the context of the present invention, a poly(A) sequence may be located within an (m)RNA or any other nucleic acid molecule, such as, e.g., in a vector, for example, in a vector serving as template for the generation of an RNA, preferably an mRNA, e.g., by transcription of the vector. In general, the poly(A) sequence consists of adenosine monophosphates.

Poly(C) sequence: A poly(C) sequence is usually understood to be a sequence of cytosine nucleotides, e.g., of up to about 400 cytosine nucleotides, e.g. from about 20 to about 100, most preferably from about 20 to about 40 cytosine nucleotides, which is preferably added to the 3'-terminus of a construct coding for a mRNA. A poly(C) sequence is typically located at the 3'-end of an RNA, in particular mRNA. In the context of the present invention, a poly(C) sequence may be located within an (m)RNA or any other nucleic acid molecule, such as, e.g., in a vector, for example, in a vector serving as template for the generation of an RNA, preferably an mRNA, e.g., by transcription of the vector.

Poly(G) sequence: A poly(G) sequence, is usually understood to be a sequence of guanine nucleotides, e.g., of up to about 400 guanine nucleotides, e.g. from about 20 to about 400, preferably from about 50 to about 400, more preferably from about 50 to about 300, even more preferably from about 50 to about 250, most preferably from about 60 to about 250 guanine nucleotides, which is preferably added to the 3'-terminus of a construct coding for a mRNA. A poly(G) sequence is typically located at the 3'-end of an RNA, in particular mRNA. In the context of the present invention, a poly(G) sequence may be located within an (m)RNA or any other nucleic acid molecule, such as, e.g., in a vector, for example, in a vector serving as template for the generation of an RNA, preferably an mRNA, e.g., by transcription of the vector.

Poly(T) sequence: A poly(T) sequence, is usually understood to be a sequence of thymine nucleotides, e.g., of up to about 400 thymine nucleotides, e.g. from about 20 to about 400, preferably from about 50 to about 400, more preferably from about 50 to about 300, even more preferably from about 50 to about 250, most preferably from about 60 to about 250 thymine nucleotides, which is preferably added to the 3'-terminus of a construct coding for a mRNA. A

poly(T) sequence is typically located at the 3'-end of an RNA, in particular mRNA. In the context of the present invention, a poly(T) sequence may be located within an (m)RNA or any other nucleic acid molecule, such as, e.g., in a vector, for example, in a vector serving as template for the generation of an RNA, preferably an mRNA, e.g., by transcription of the vector.

Sugar Modifications: The modified nucleosides and nucleotides, which may be used in the context of the present invention, can be modified in the sugar moiety. For example, the 2' hydroxyl group (OH) can be modified or replaced with a number of different "oxy" or "deoxy" substituents. Examples of "oxy" -2' hydroxyl group modifications include, but are not limited to, alkoxy or aryloxy (-OR, e.g., R = H, alkyl, cycloalkyl, aryl, aralkyl, heteroaryl or sugar); polyethyleneglycols (PEG),-0(CH2CH2o)nCH2CH2OR; "locked" nucleic acids (LNA) in which the 2' hydroxyl is connected, e.g., by a methylene bridge, to the 4' carbon of the same ribose sugar; and amino groups (-O-amino, wherein the amino group, e.g., NRR, can be alkylamino, dialkylamino, heterocyclyl, arylamino, diarylamino, heteroarylamino, or diheteroaryl amino, ethylene diamine, polyamino) or aminoalkoxy.

"Deoxy" modifications include hydrogen, amino (e.g. NH2; alkylamino, dialkylamino, heterocyclyl, arylamino, diaryl amino, heteroaryl amino, diheteroaryl amino, or amino acid); or the amino group can be attached to the sugar through a linker, wherein the linker comprises one or more of the atoms C, N, and O.

The sugar group can also contain one or more carbons that possess the opposite stereochemical configuration than that of the corresponding carbon in ribose. Thus, a modified nucleotide can include nucleotides containing, for instance, arabinose as the sugar.

Backbone Modifications: The phosphate backbone may further be modified in the modified nucleosides and nucleotides. The phosphate groups of the backbone can be modified by replacing one or more of the oxygen atoms with a different substituent. Further, the modified nucleosides and nucleotides can include the full replacement of an unmodified phosphate moiety with a modified phosphate as described herein. Examples of modified phosphate groups include, but are not limited to, phosphorothioate, phosphoroselenates, borano phosphates, borano phosphate esters, hydrogen phosphonates, phosphoroamidates, alkyl or aryl phosphonates and phosphotriesters. Phosphorodithioates have both non-linking oxygens replaced by sulfur. The phosphate linker can also be modified by the replacement of a linking oxygen with nitrogen (bridged phosphoroamidates), sulfur (bridged phosphorothioates) and carbon (bridged methylene-phosphonates).

Base Modifications: The modified nucleosides and nucleotides, which may be used in the present invention, can further be modified in the nucleobase moiety. Examples of nucleobases found in RNA include, but are not limited to, adenine, guanine, cytosine and uracil. For example, the nucleosides and nucleotides described herein can be chemically modified on the major groove face. In some embodiments, the major groove chemical modifications can include an amino group, a thiol group, an alkyl group, or a halo group.

In particularly preferred embodiments of the present invention, the nucleotide analogs/modifications are selected from base modifications, which are preferably selected from 2-amino-6-chloropurineriboside-5'-triphosphate, 2-Aminopurine-riboside-5'-triphosphate; 2-aminoadenosine-5'-triphosphate, 2'-Amino-2'-deoxycytidine-triphosphate, 2-thiocytidine-5 '-triphosphate, 2-thiouridine-5'-triphosphate, 2'-Fluorothymidine-5'-triphosphate, 2'-O-Methyl inosine-5'-triphosphate 4-thiouridine-5'-triphosphate, 5-aminoallylcytidine-5'-triphosphate, 5-aminoallyluridine-5'-triphosphate, 5-bromocytidine-5'-triphosphate, 5-bromouridine-5'-triphosphate, 5-Bromo-2'-deoxycytidine-5'-triphosphate, 5-Bromo-2'-deoxyuridine-5'-triphosphate, 5-iodocytidine-5'-triphosphate, 5-Iodo-2'-deoxycytidine-5'-triphosphate, 5-iodouridine-5'-triphosphate, 5-Iodo-2'-deoxyuridine-5'-triphosphate, 5-methylcytidine-5'-triphosphate, 5-methyluridine-5'-triphosphate, 5-Propynyl-2'-deoxycytidine-5'-triphosphate, 5-Propynyl-2'-deoxyuridine-5'-triphosphate, 6-azacytidine-5'-triphosphate, 6-azauridine-5'-triphosphate, 6-chloropurineriboside-5 '-triphosphate, 7-deazaadenosine-5'-triphosphate, 7-deazaguanosine-5'-triphosphate, 8-azaadenosine-5'-triphosphate, 8-azidoadenosine-5 '-triphosphate, benzimidazole-riboside-5'-triphosphate, N1-methyladenosine-5'-triphosphate, N1-methylguanosine-5'-triphosphate, N6-methyladenosine-5'-triphosphate, O6-methylguanosine-5'-triphosphate, pseudouridine-5'-triphosphate, or puromycin-5'-triphosphate, xanthosine-5'-triphosphate. Particular preference is given to nucleotides for base modifications selected from the group of base-modified nucleotides consisting of 5-methylcytidine-5'-triphosphate, 7-deazaguanosine-5'-triphosphate, 5-bromocytidine-5'-triphosphate, and pseudouridine-5'-triphosphate.

In some embodiments, modified nucleosides include pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thio-uridine, 5-methyl-uridine, 1-methyl-pseudouridine, 4-thio- 1-methyl-pseudouridine, 2-thio-1-methyl-pseudouridine, 1-methyl-1-deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, and 4-methoxy-2-thio-pseudouridine.

In some embodiments, modified nucleosides include 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, and 4-methoxy-1-methyl-pseudoisocytidine.

In other embodiments, modified nucleosides include 2-aminopurine, 2, 6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl) adenosine, N6-glycinylcarbamoyladenosine, N6-threonylcarbamoyladenosine, 2-methylthio-N6-threonyl carbamoyladenosine, N6,N6-dimethyladenosine, 7-methyladenine, 2-methylthio-adenine, and 2-methoxy-adenine.

In other embodiments, modified nucleosides include inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, 1-methyl-6-thio-guanosine, N2-methyl-6-thio-guanosine, and N2,N2-dimethyl-6-thio-guanosine.

In some embodiments, the nucleotide can be modified on the major groove face and can include replacing hydrogen on C-5 of uracil with a methyl group or a halo group. In specific embodiments, a modified nucleoside is 5'-0-(1-Thiophosphate)-Adenosine, 5'-0-(1-Thiophosphate)-Cytidine, 5'-0-(1-Thiophosphate)-Guanosine, 5'-0-(1-Thiophosphate)-Uridine or 5'-0-(1-Thiophosphate)-Pseudouridine.

In further specific embodiments the modified nucleotides include nucleoside modifications selected from 6-aza-cytidine, 2-thio-cytidine, α-thio-cytidine, Pseudo-iso-cytidine, 5-aminoallyl-uridine, 5-iodo-uridine, N1-methyl-pseudouridine, 5,6-dihydrouridine, α-thio-uridine, 4-thio-uridine, 6-aza-uridine, 5-hydroxy-uridine, deoxy-thymidine, 5-methyl-uridine, Pyrrolo-cytidine, inosine, α-thio-guanosine, 6-methyl-guanosine, 5-methyl-cytdine, 8-oxo-guanosine, 7-deaza-guanosine, N1-methyl-adenosine, 2-amino-6-Chloro-purine, N6-methyl-2-amino-purine, Pseudo-iso-cytidine, 6-Chloro-purine, N6-methyl-adenosine, α-thio-adenosine, 8-azido-adenosine, 7-deaza-adenosine.

Further modified nucleotides have been described previously (see, e.g., WO 2013/052523 and WO 2015/089511).

5'-cap : A 5 '-cap is an entity, typically a modified nucleotide entity, which generally "caps" the 5'-end of a mature mRNA and increases its stability. A 5'-cap may typically be formed by a modified nucleotide (cap analog), particularly by a derivative of a guanine nucleotide. Preferably, the 5'-cap is linked to the 5'-terminus of the RNA via a 5'-5'-triphosphate linkage. A 5'-cap may be methylated, e.g. m7GpppN (e.g. m7G(5')ppp(5')G (m7G)), wherein N is the terminal 5' nucleotide of the nucleic acid carrying the 5'-cap, typically the 5'-end of an RNA, m7 is a methyl group attached to position 7 of the guanine (G) and ppp is the triphosphate. Further examples of 5'cap structures include glyceryl, inverted deoxy abasic residue (moiety), 4',5' methylene nucleotide, 1-(beta-D-erythrofuranosyl)nucleotide, 4'-thio nucleotide, carbocyclic nucleotide, 1,5-anhydrohexitol nucleotide, L-nucleotides, alpha-nucleotide, modified base nucleotide, threo-pentofuranosyl nucleotide, acyclic 3',4'-seco nucleotide, acyclic 3,4-dihydroxybutyl nucleotide, acyclic 3,5 dihydroxypentyl nucleotide, 3'-3 '-inverted nucleotide moiety, 3'-3'-inverted abasic moiety, 3'-2'-inverted nucleotide moiety, 3'-2'-inverted abasic moiety, 1,4-butanediol phosphate, 3'-phosphoramidate, hexylphosphate, aminohexyl phosphate, 3'-phosphate, 3'phosphorothioate, phosphorodithioate, or bridging or non-bridging methylphosphonate moiety. Further modified 5'-cap structures which may be used in the context of the present invention are cap1 (additional methylation of the ribose of the adjacent nucleotide of m7GpppN), cap2 (additional methylation of the ribose of the 2nd nucleotide downstream of the m7GpppN), cap3 (additional methylation of the ribose of the 3rd nucleotide downstream of the m7GpppN), cap4 (additional methylation of the ribose of the 4th nucleotide downstream of the m7GpppN), ARCA (anti-reverse cap analogue), modified ARCA (e.g. phosphothioate modified ARCA), inosine, N1-methyl-guanosine, 2'-fluoro-guanosine, 7-deaza-guanosine, 8-oxo-guanosine, 2-amino-guanosine, LNA-guanosine, and 2-azido-guanosine.

RNA, mRNA: RNA is the usual abbreviation for ribonucleic acid. It is a nucleic acid molecule, i.e. a polymer consisting of nucleotides. These nucleotides are usually adenosine-monophosphate, uridine-monophosphate, guanosine-monophosphate and cytidine-monophosphate monomers which are connected to each other along a so-called backbone. The backbone is formed by phosphodiester bonds between the sugar, i.e. ribose, of a first and a phosphate moiety of a second, adjacent monomer. The specific succession of the monomers is called the RNA sequence. Usually, RNA may be obtainable by transcription of a DNA sequence, e.g., inside a cell. In eukaryotic cells, transcription is typically performed inside the nucleus or the mitochondria. *In vivo,* transcription of DNA usually results in the so-called premature RNA, which has to be processed into so-called messenger RNA, usually abbreviated as mRNA. Processing of the premature RNA, e.g. in eukaryotic organisms, comprises a variety of different posttranscriptional-modifications such as splicing, 5'-capping, polyadenylation, export from the nucleus or the mitochondria and the like. The sum of these processes is also called maturation of RNA. The mature messenger RNA usually provides the nucleotide sequence that may be translated into an amino acid sequence of a particular peptide or protein. Typically, a mature mRNA comprises a 5'-cap, a 5'-UTR, an open reading frame, a 3'-UTR and a poly(A) sequence. Aside from messenger RNA, several non-coding types of RNA exist, which may be involved in the regulation of transcription and/or translation. Short RNA molecules

can be synthesized by chemical methods whereas long RNAs are typically produced by in vitro transcription reactions containing a suitable DNA template with a bacteriophage-derived promoter, an RNA polymerase, for example bacteriophage SP6, T3 or T7 RNA polymerase and ribonucleoside triphosphates (NTPs).

DNA: DNA is the usual abbreviation for deoxyribonucleic acid. It is a nucleic acid molecule, i.e. a polymer consisting of nucleotide monomers. These nucleotides are usually deoxy-adenosine-monophosphate, deoxy-thymidine-monophosphate, deoxy-guanosine-monophosphate and deoxy-cytidine-monophosphate monomers which are - by themselves - composed of a sugar moiety (deoxyribose), a base moiety and a phosphate moiety, and polymerized by a characteristic backbone structure. The backbone structure is, typically, formed by phosphodiester bonds between the sugar moiety of the nucleotide, i.e. deoxyribose, of a first and a phosphate moiety of a second, adjacent monomer. The specific order of the monomers, i.e. the order of the bases linked to the sugar/phosphate-backbone, is called the DNA-sequence. DNA may be single-stranded or double-stranded. In the double stranded form, the nucleotides of the first strand typically hybridize with the nucleotides of the second strand, e.g. by A/T-base-pairing and G/C-base-pairing.

Sequence of a nucleic acid molecule/nucleic acid sequence: The sequence of a nucleic acid molecule is typically understood to be the particular and individual order, i.e. the succession of its nucleotides.

Sequence of amino acid molecules/amino acid sequence: The sequence of a protein or peptide is typically understood to be the order, i.e. the succession of its amino acids.

Sequence identity: Two or more sequences are identical if they exhibit the same length and order of nucleotides or amino acids. The percentage of identity typically describes the extent, to which two sequences are identical, i.e. it typically describes the percentage of nucleotides that correspond in their sequence position to identical nucleotides of a reference sequence. For the determination of the degree of identity, the sequences to be compared are considered to exhibit the same length, i.e. the length of the longest sequence of the sequences to be compared. This means that a first sequence consisting of 8 nucleotides/amino acids is 80% identical to a second sequence consisting of 10 nucleotides/amino acids comprising the first sequence. In other words, in the context of the present invention, identity of sequences preferably relates to the percentage of nucleotides/amino acids of a sequence, which have the same position in two or more sequences having the same length. Gaps are usually regarded as non-identical positions, irrespective of their actual position in an alignment.

Origin of replication: The term "origin of replication" refers to a DNA sequence which is able to promote the initiation of replication of the DNA molecule (e.g., genomic DNA, covalently closed circular recombinant DNA). In the context of the invention, the term especially refers to a DNA sequence in a covalently closed circular recombinant DNA molecule which is able to promote the initiation of replication of the covalently closed circular recombinant DNA molecule. Usually, the origin of replication is unidirectional. The replication of a unidirectional origin of replication is carried out in one direction. The origin of replication may have the sequence identified in SEQ ID NO: 6 (derived from the pUC plasmid), the sequence identified in SEQ ID NO: 7 (derived from the pBR322 plasmid), the sequence identified in SEQ ID NO: 8 (derived from the pMB1 plasmid), the sequence identified in SEQ ID NO: 9 (derived from the ColE1 plasmid), the sequences of R6K gamma (SEQ ID NO: 10), R6K beta (SEQ ID NO: 11), R6K alpha (SEQ ID NO: 12) derived from the R6K plasmid, the sequence identified in SEQ ID NO: 13 (derived from the p15 A plasmid), or the sequence identified in SEQ ID NO: 14 (derived from the pSC101 plasmid). Sequences having 70%, 80%, 90%, 95 %, 98% or 99% identity to the sequences SEQ ID NOs: 6-14 are also encompassed.

Vector: The term "vector" refers to a nucleic acid molecule, preferably to an artificial nucleic acid molecule. A vector in the context of the present invention is suitable for incorporating or harboring a desired nucleic acid sequence, such as a nucleic acid sequence comprising an open reading frame. Such vectors may be storage vectors, expression vectors, cloning vectors, transfer vectors etc. A storage vector is a vector, which allows the convenient storage of a nucleic acid molecule, for example, of an mRNA molecule. Thus, the vector may comprise a sequence corresponding, e.g., to a desired mRNA sequence or a part thereof, such as a sequence corresponding to the open reading frame and the 3'-UTR of an mRNA. An expression vector may be used for production of expression products such as RNA, e.g. mRNA, or peptides, polypeptides or proteins. For example, an expression vector may comprise sequences needed for transcription of a sequence stretch of the vector, such as a promoter sequence, e.g. an RNA polymerase promoter sequence. A cloning vector is typically a vector that contains a cloning site, which may be used to incorporate nucleic acid sequences into the vector. A cloning vector may be, e.g., a plasmid vector or a bacteriophage vector. A transfer vector may be a vector, which is suitable for transferring nucleic acid molecules into cells or organisms, for example, viral vectors. A vector in the context of the present invention may be, e.g., an RNA vector or a DNA vector. Preferably, a vector is a DNA molecule. Preferably, a vector in the sense of the present application comprises a cloning site, a selection marker, such as an antibiotic resistance factor, and a sequence suitable for multiplication of the vector, such as an origin of replication. Preferably, a vector in the context of the present application is a plasmid vector.

3'-untranslated region (3'-UTR): Generally, the term "3'-UTR" refers to a part of the artificial nucleic acid molecule, which is located 3' (i.e. "downstream") of an open reading frame and which is not translated into protein. Typically, a 3'-UTR is the part of an mRNA, which is located between the protein coding region (open reading frame (ORF) or coding sequence (CDS)) and the poly(N/A) sequence of the (m)RNA. In the context of the invention, a 3'-UTR of the

artificial nucleic acid molecule may comprise more than one 3'-UTR elements, which may be of different origin, such as sequence elements derived from the 3'-UTR of several (unrelated) naturally occurring genes. Accordingly, the term 3'-UTR may also comprise elements, which are not encoded in the template, from which an RNA is transcribed, but which are added after transcription during maturation, e.g. a poly(N/A) sequence. A 3'-UTR of the mRNA is not translated into an amino acid sequence. The 3'-UTR sequence is generally encoded by the gene, which is transcribed into the respective mRNA during the gene expression process. The genomic sequence is first transcribed into pre-mature mRNA, which comprises optional introns. The pre-mature mRNA is then further processed into mature mRNA in a maturation process. This maturation process comprises the steps of 5'capping, splicing the pre-mature mRNA to excize optional introns and modifications of the 3'-end, such as polynucleotidylation/polyadenylation of the 3'-end of the pre-mature mRNA and optional endo-/ or exonuclease cleavages etc. In the context of the present invention, a 3'-UTR corresponds to the sequence of a mature mRNA which is located between the stop codon of the protein coding region, preferably immediately 3' to the stop codon of the protein coding region, and the poly(N/A) sequence of the mRNA. The term "corresponds to" means that the 3'-UTR sequence may be an RNA sequence, such as in the mRNA sequence used for defining the 3'-UTR sequence, or a DNA sequence, which corresponds to such RNA sequence. In the context of the present invention, the term "a 3'-UTR of a gene", such as "a 3'-UTR of a ribosomal protein gene", is the sequence, which corresponds to the 3'-UTR of the mature mRNA derived from this gene, i.e. the mRNA obtained by transcription of the gene and maturation of the pre-mature mRNA. The term "3'-UTR of a gene" encompasses the DNA sequence and the RNA sequence (both sense and antisense strand and both mature and immature) of the 3'-UTR. As used herein, the term "3'-UTR element" typically refers to a fragment of a 3'-UTR as defined herein. In particular, the term comprises any nucleic acid sequence element, which is located 3' to the ORF in the artificial nucleic acid molecule, preferably the mRNA, according to the invention. Accordingly, the term covers, for example, sequence elements derived from the 3'-UTR of a heterologous gene as well as elements such as a poly(A) sequence, a poly(C) sequence or a histone stem-loop.

Histone stem loop: Histone stem-loop sequences are preferably selected from histone stem-loop sequences as disclosed in WO 2012/019780.

A histone stem-loop sequence, suitable to be used within the present invention, is preferably selected from at least one of the following formulae (I) or (II):

**formula (I)** (stem-loop sequence without stem bordering elements):

$$\underbrace{[N_{0-2}GN_{3-5}]}_{\text{stem1}}\ \underbrace{[N_{0-4}(U/T)N_{0-4}]}_{\text{loop}}\ \underbrace{[N_{3-5}CN_{0-2}]}_{\text{stem2}}$$

**formula (II)** (stem-loop sequence with stem bordering elements):

$$\underbrace{N_{1-6}}_{\substack{\text{stem1}\\ \text{bordering element}}}\ \underbrace{[N_{0-2}GN_{3-5}]}_{\text{stem1}}\ \underbrace{[N_{0-4}(U/T)N_{0-4}]}_{\text{loop}}\ \underbrace{[N_{3-5}CN_{0-2}]}_{\text{stem2}}\ \underbrace{N_{1-6}}_{\substack{\text{stem2}\\ \text{bordering element}}}$$

wherein:

stem1 or stem2 bordering elements $N_{1-6}$ is a consecutive sequence of 1 to 6, preferably of 2 to 6, more preferably of 2 to 5, even more preferably of 3 to 5, most preferably of 4 to 5 or 5 N, wherein each N is independently from another selected from a nucleotide selected from A, U, T, G and C, or a nucleotide analogue thereof;

stem1 $[N_{0-2}GN_{3-5}]$ is reverse complementary or partially reverse complementary with element stem2, and is a consecutive sequence between of 5 to 7 nucleotides;
wherein $N_{0-2}$ is a consecutive sequence of 0 to 2, preferably of 0 to 1, more preferably of 1 N, wherein each N is independently from another selected from a nucleotide selected from A, U, T, G and C or a nucleotide analogue thereof;
wherein $N_{3-5}$ is a consecutive sequence of 3 to 5, preferably of 4 to 5, more preferably of 4 N, wherein each N is independently from another selected from a nucleotide selected from A, U, T, G and C or a nucleotide analogue

thereof, and
wherein G is guanosine or an analogue thereof, and may be optionally replaced by a cytidine or an analogue thereof, provided that its complementary nucleotide cytidine in stem2 is replaced by guanosine;

loop sequence $[N_{0-4}(U/T)N_{0-4}]$ is located between elements stem1 and stem2, and is a consecutive sequence of 3 to 5 nucleotides, more preferably of 4 nucleotides;
wherein each $N_{0-4}$ is independent from another a consecutive sequence of 0 to 4, preferably of 1 to 3, more preferably of 1 to 2 N, wherein each N is independently from another selected from a nucleotide selected from A, U, T, G and C or a nucleotide analogue thereof; and
wherein U/T represents uridine, or optionally thymidine;

stem2 $[N_{3-5}CN_{0-2}]$ is reverse complementary or partially reverse complementary with element stem1, and is a consecutive sequence between of 5 to 7 nucleotides;
wherein $N_{3-5}$ is a consecutive sequence of 3 to 5, preferably of 4 to 5, more preferably of 4 N, wherein each N is independently from another selected from a nucleotide selected from A, U, T, G and C or a nucleotide analogue thereof;
wherein $N_{0-2}$ is a consecutive sequence of 0 to 2, preferably of 0 to 1, more preferably of 1 N, wherein each N is independently from another selected from a nucleotide selected from A, U, T, G or C or a nucleotide analogue thereof; and
wherein C is cytidine or an analogue thereof, and may be optionally replaced by a guanosine or an analogue thereof provided that its complementary nucleoside guanosine in stem1 is replaced by cytidine;

wherein
stem1 and stem2 are capable of base pairing with each other forming a reverse complementary sequence, wherein base pairing may occur between stem1 and stem2, e.g. by Watson-Crick base pairing of nucleotides A and U/T or G and C or by non-Watson-Crick base pairing e.g. wobble base pairing, reverse Watson-Crick base pairing, Hoogsteen base pairing, reverse Hoogsteen base pairing or are capable of base pairing with each other forming a partially reverse complementary sequence, wherein an incomplete base pairing may occur between stem1 and stem2, on the basis that one or more bases in one stem do not have a complementary base in the reverse complementary sequence of the other stem.

According to a further preferred embodiment the histone stem-loop sequence may be selected according to at least one of the following specific formulae (Ia) or (IIa):

formula (Ia) (stem-loop sequence without stem bordering elements):

$$\underbrace{[N_{0-1}GN_{3-5}]}_{\text{stem1}}\ \underbrace{[N_{1-3}(U/T)N_{0-2}]}_{\text{loop}}\ \underbrace{[N_{3-5}CN_{0-1}]}_{\text{stem2}}$$

formula (IIa) (stem-loop sequence with stem bordering elements):

$$\underbrace{N_{2-5}}_{\substack{\text{stem1}\\\text{bordering element}}}\ \underbrace{[N_{0-1}GN_{3-5}]}_{\text{stem1}}\ \underbrace{[N_{1-3}(U/T)N_{0-2}]}_{\text{loop}}\ \underbrace{[N_{3-5}CN_{0-1}]}_{\text{stem2}}\ \underbrace{N_{2-5}}_{\substack{\text{stem2}\\\text{bordering element}}}$$

wherein:

N, C, G, T and U are as defined above.

According to a further more particularly preferred embodiment of the first aspect, the artificial nucleic acid molecule sequence may comprise at least one histone stem-loop sequence according to at least one of the following specific formulae (Ib) or (IIb):

formula (Ib) (stem-loop sequence without stem bordering elements):

$$\underbrace{[N_1GN_4]}_{\text{stem1}}\ \underbrace{[N_2(U/T)N_1]}_{\text{loop}}\ \underbrace{[N_4CN_1]}_{\text{stem2}}$$

formula (IIb) (stem-loop sequence with stem bordering elements):

$$\underbrace{N_{4-5}}_{\substack{\text{stem1}\\ \text{bordering element}}}\ \underbrace{[N_1GN_4]}_{\text{stem1}}\ \underbrace{[N_2(U/T)N_1]}_{\text{loop}}\ \underbrace{[N_4CN_1]}_{\text{stem2}}\ \underbrace{N_{4-5}}_{\substack{\text{stem2}\\ \text{bordering element}}}$$

wherein:
N, C, G, T and U are as defined above.

A particular preferred histone stem-loop sequence is the sequence according to SEQ ID NO: 16: CAAAGGCTCTTTTCAGAGCCACCA or more preferably the corresponding RNA sequence according to SEQ ID NO: 17: caaaggcucuuuucagagccacca

5'-untranslated region (5'-UTR): A 5'-UTR is typically understood to be a particular section of messenger RNA (mRNA). It is located 5' of the open reading frame of the mRNA. Typically, the 5'-UTR starts with the transcriptional start site and ends one nucleotide before the start codon of the open reading frame. The 5'-UTR may comprise elements for controlling gene expression, which are also called regulatory elements. Such regulatory elements may be, for example, ribosomal binding sites. The 5'-UTR may be posttranscriptionally modified, for example by addition of a 5'-cap. In the context of the present invention, a 5'-UTR corresponds to the sequence of a mature mRNA, which is located between the 5'-cap and the start codon. Preferably, the 5'-UTR corresponds to the sequence, which extends from a nucleotide located 3' to the 5'-cap, preferably from the nucleotide located immediately 3' to the 5'-cap, to a nucleotide located 5' to the start codon of the protein coding region, preferably to the nucleotide located immediately 5' to the start codon of the protein coding region. The nucleotide located immediately 3' to the 5'-CAP of a mature mRNA typically corresponds to the transcriptional start site. The term "corresponds to" means that the 5'-UTR sequence may be an RNA sequence, such as in the mRNA sequence used for defining the 5'-UTR sequence, or a DNA sequence, which corresponds to such RNA sequence. In the context of the present invention, the term "a 5'-UTR of a gene" is the sequence, which corresponds to the 5'-UTR of the mature mRNA derived from this gene, i.e. the mRNA obtained by transcription of the gene and maturation of the pre-mature mRNA. The term "5'-UTR of a gene" encompasses the DNA sequence and the RNA sequence of the 5'-UTR.

Cloning site: A cloning site is typically understood to be a segment of a nucleic acid molecule, which is suitable for insertion of a nucleic acid molecule, e.g., a nucleic acid sequence molecule comprising an open reading frame. The nucleic acid molecule may be inserted by any molecular biological method known to the one skilled in the art, e.g. by restriction and ligation. A cloning site typically comprises one or more restriction enzyme recognition sites (restriction sites). These one or more restrictions sites may be recognized by restriction enzymes which cleave the DNA at these sites. A cloning site which comprises more than one restriction site may also be termed a multiple cloning site (MCS) or a polylinker.

Open reading frame: An open reading frame (ORF) in the context of the invention may typically be a sequence of several nucleotide triplets which may be translated into a peptide or protein. An open reading frame preferably contains a start codon, i.e. a combination of three subsequent nucleotides coding usually for the amino acid methionine (ATG), at its 5'-end and a subsequent region which usually exhibits a length which is a multiple of 3 nucleotides. An ORF is preferably terminated by a stop-codon (e.g., TAA, TAG, TGA). Typically, this is the only stop-codon of the open reading frame. Thus, an open reading frame in the context of the present invention is preferably a nucleotide sequence consisting

of a number of nucleotides that may be divided by three, which starts with a start codon (e.g. ATG) and which preferably terminates with a stop codon (e.g., TAA, TGA, or TAG). The open reading frame may be isolated or it may be incorporated in a longer nucleic acid sequence, for example in a vector or an mRNA. An open reading frame may also be termed "protein coding region".

*In vitro* transcription: The term "*in vitro* transcription" relates to a process wherein RNA is synthesized in a cell-free system (*in vitro*). DNA, particularly plasmid DNA, is used as template for the generation of RNA transcripts. RNA may be obtained by DNA-dependent *in vitro* transcription of an appropriate DNA template, which according to the present invention is preferably a linearized plasmid DNA template. The promoter for controlling *in vitro* transcription can be any promoter for any DNA dependent RNA polymerase. Particular examples of DNA dependent RNA polymerases are the T7, T3, and SP6 RNA polymerases. A DNA template for *in vitro* RNA transcription may be obtained by cloning of a nucleic acid, in particular cDNA corresponding to the respective RNA to be *in vitro* transcribed, and introducing it into an appropriate vector for *in vitro* transcription, for example into plasmid DNA. Within the meaning of the present invention the DNA template is linearized by the method of the invention, before it is transcribed *in vitro.* The cDNA may be obtained by reverse transcription of mRNA or chemical synthesis. Moreover, the DNA template for *in vitro* RNA synthesis may also be obtained by gene synthesis.

Methods for *in vitro* transcription are known in the art (Geall et al. (2013) Semin. Immunol. 25(2): 152-159; Brunelle et al. (2013) Methods Enzymol. 530:101-14). Reagents used in said method typically include:

1) a linearized DNA template (as defined above) with a promoter sequence that has a high binding affinity for its respective RNA polymerase such as bacteriophage-encoded RNA polymerases;
2) ribonucleoside triphosphates (NTPs) for the four bases (adenine, cytosine, guanine and uracil);
3) optionally a cap analog as defined above (e.g. m7G(5')ppp(5')G (m7G));
4) a DNA-dependent RNA polymerase capable of binding to the promoter sequence within the linearized DNA template (e.g. T7, T3 or SP6 RNA polymerase);
5) optionally a ribonuclease (RNase) inhibitor to inactivate any contaminating RNase;
6) optionally a pyrophosphatase to degrade pyrophosphate, which may inhibit transcription;
7) $MgCl_2$, which supplies $Mg^{2+}$ ions as a co-factor for the polymerase;
8) a buffer to maintain a suitable pH value, which can also contain antioxidants (e.g. DTT) and polyamines such as spermidine at optimal concentrations.

According to a preferred embodiment, the (transcription) buffer is selected from the group consisting of 4-(2-hydroxyethyl)-1-piperazineethanesulfonic acid (HEPES) and tris(hydroxymethyl)aminomethane (Tris). Preferably the buffer is used at a concentration from 10 to 100 mM, 10 to 75 mM, 10 to 50 mM, 10 to 40 mM, 10 to 30 mM or 10 to 20 mM. The pH value of the buffer can be adjusted with, for example, NaOH, KOH or HCl. Preferably the buffer has a pH value from 6 to 8.5, from 6.5 to 8.0, from 7.0 to 7.5, even more preferred 7.5. Most preferred is a buffer selected from the group consisting of 80 mM HEPES/KOH, pH 7.5 and 40 mM Tris/HCl, pH 7.5.

According to a preferred embodiment of the invention, the RNA polymerase is selected from the group consisting of T3, T7 and SP6 RNA polymerase. Preferably, the concentration of the RNA polymerase is from about 1 to 100 nM, 1 to 90 nM, 1 to 80 nM, 1 to 70 nM, 1 to 60 nM, 1 to 50 nM, 1 to 40 nM, 1 to 30 nM, 1 to 20 nM, or about 1 to 10 nM. Even more preferred, the concentration of the RNA polymerase is from about 10 to 50 nM, 20 to 50 nM, or 30 to 50 nM. Most preferred is a RNA polymerase concentration of about 40 nM. The person skilled in the art will understand that the choice of the RNA polymerase concentration is influenced by the concentration of the DNA template. Therefore, in specific embodiments the concentration of the RNA polymerase is between 1 and 1000 U/$\mu$g template DNA, preferably between 10 and 100 U/$\mu$g DNA, particularly if plasmid DNA is used as template DNA.

According to a preferred embodiment of the invention, the concentration of the linear DNA template is in a range from about 1 to 50 nM, 1 to 40 nM, 1 to 30 nM, 1 to 20 nM, or about 1 to 10 nM. Even more preferred the concentration of the DNA template is from about 10 to 30 nM. Most preferred the concentration of the DNA template is about 20 nM. In case plasmid DNA is used as DNA template, the concentration of the DNA template is preferably between 1 to 100 $\mu$g/ml, particularly in a concentration of about 50 $\mu$g/ml.

According to a preferred embodiment of the invention, the *in vitro* transcription is performed in the presence of pyrophosphatase. Preferably, the concentration of the pyrophosphatase is from about 1 to 20 units/ml, 1 to 15 units/ml, 1 to 10 units/ml, 1 to 5 units/ml, or 1 to 2.5 units/ml. Even more preferred the concentration of the pyrophosphatase is about 5 unit/ml.

According to a preferred embodiment of the invention, the *in vitro* transcription reaction mixture comprises $Mg^{2+}$ ions. Preferably, the $Mg^{2+}$ ions are provided in the form of $MgCl_2$ or $Mg(OAc)_2$. Preferably, the initial free $Mg^{2+}$ concentration is from about 1 to 100 mM, 1 to 75 mM, 1 to 50 mM, 1 to 25 mM, or 1 to 10 mM. Even more preferred the initial free $Mg^{2+}$ concentration is from about 10 to 30 mM or about 15 to 25 mM. Most preferred is an initial free $Mg^{2+}$ concentration of about 24 mM. The person skilled in the art will understand that the choice of the $Mg^{2+}$ concentration is influenced by

the initial total NTP concentration.

According to a preferred embodiment of the invention, the *in vitro* transcription reaction mixture comprises a reducing agent (antioxidant) to keep the RNA polymerase in its active state. Preferably, the reducing agent is selected from the group consisting of dithiothreitol (DTT), dithioerythritol (DTE), Tris(2-carboxyethyl)phosphine (TCEP) and β-mercaptoethanol. Preferably the concentration of the reducing reagent is from about 1 to 50 mM, 1 to 40 mM, 1 to 30 mM, or 1 to 20 mM, or 1 to 10 mM. Even more preferred the concentration of the reducing reagent is from 10 to 50 mM or 20 to 40 mM. Most preferred is a concentration of 40 mM of DTT.

According to a preferred embodiment of the invention, the *in vitro* transcription reaction mixture comprises a polyamine. Preferably, the polyamine is selected from the group consisting of spermine and spermidine. Preferably the concentration of the polyamine is from about 1 to 25 mM, 1 to 20 mM, 1 to 15 mM, 1 to 10 mM, 1 to 5 mM, or about 1 to 2.5 mM. Even more preferred the concentration of the polyamine is about 2 mM. Most preferred is a concentration of 2 mM of spermidine.

According to a preferred embodiment of the invention, the *in vitro* transcription reaction mixture comprises a ribonuclease inhibitor. Preferably, the concentration of the ribonuclease inhibitor is from about 1 to 500 units/ml, 1 to 400 units/ml, 1 to 300 units/ml, 1 to 200 units/ml, or 1 to 100 units/ml. Even more preferred the concentration of the ribonuclease inhibitor is about 200 units/ml.

According to a preferred embodiment of the invention, the total NTP concentration in the *in vitro* transcription reaction mixture is between 1 and 100 mM, preferably between 10 and 50 mM, and most preferably between 10 and 20 mM.

According to the invention, the term total nucleotide concentration means the total concentration of NTPs, e.g. the sum of the concentrations of ATP, GTP, CTP, UTP, and/or cap analog present initially in the *in vitro* transcription when the various components of the reaction have been assembled in the final volume for carrying out the *in vitro* transcription reaction. Naturally, as the reaction proceeds, the nucleotides will be incorporated into the RNA molecule and consequently the total nucleotide concentration will be progressively reduced from its initial value.

In this context it is particularly preferred that the single nucleotides are provided in a concentration between 0.1 and 10 mM, preferably between 1 and 5 mM and most preferably in a concentration of 4 mM.

In case a 5' cap as defined above has to be generated at the 5'-end of the RNA, the in vitro transcription reaction mixture further comprises a cap analog. In this context the concentration of GTP is preferably reduced compared to the other nucleotides (ATP, CTP and UTP). Preferably the cap analog is added with an initial concentration in the range of about 1 to 20 mM, 1 to 17.5 mM, 1 to 15 mM, 1 to 12.5 mM, 1 to 10 mM, 1 to 7.5 mM. Most preferably the cap analog is added in a concentration of 5.8 mM and the GTP concentration is reduced to a concentration of 1.45 mM whereas ATP, CTP and UTP are comprised in the reaction in a concentration of 4 mM each.

The ribonucleoside triphosphates (NTPs) GTP, ATP, CTP and UTP or analogs thereof may be provided with a monovalent or divalent cation as counterion. Preferably the monovalent cation is selected from the group consisting of $Li^+$, $Na^+$, $K^+$, $NH4^+$ or tris(hydroxymethyl)-aminomethane (Tris). Preferably, the divalent cation is selected from the group consisting of $Mg^{2+}$, $Ba^{2+}$ and $Mn^{2+}$.

According to a preferred embodiment of the invention, a part or all of at least one ribonucleoside triphosphate in the *in vitro* transcription reaction mixture is replaced with a modified nucleoside triphosphate (as defined herein). In a preferred embodiment of the invention, said modified nucleoside triphosphate is selected from the group consisting of pseudouridine-5'-triphosphate, 1-methylpseudouridine-5'-triphosphate, 2-thiouridine-5'-triphosphate, 4-thiouridine-5'-triphosphate and 5-methylcytidine-5 '-triphosphate.

After *in vitro* transcription has occurred, the RNA product is subjected to purification methods. In this context any purification method may be used (e.g. DNA template digest, phenol-chloroform extraction, LiCl precipitation, HPLC, etc.).

Plasmid DNA: The term 'plasmid DNA' refers to a circular nucleic acid molecule, preferably to an artificial nucleic acid molecule. A plasmid DNA in the context of the present invention is suitable for incorporating or harboring a desired nucleic acid sequence, such as a nucleic acid sequence comprising an RNA coding sequence and/or an open reading frame encoding at least one peptide or polypeptide. Such plasmid DNA constructs may be storage vectors, expression vectors, cloning vectors, transfer vectors etc. A storage vector is a vector which allows the convenient storage of a nucleic acid molecule, for example, of an RNA molecule. Thus, the plasmid DNA may comprise a sequence corresponding to (coding for), e.g., a target RNA sequence or a part thereof, such as a sequence corresponding to the open reading frame and the 5'- and/or 3'UTR of an mRNA. An expression vector may be used for production of expression products such as RNA, e.g. mRNA in a process called *in vitro* transcription. For example, an expression vector may comprise sequences needed for *in vitro* transcription of a sequence stretch of the vector, such as a promoter sequence, e.g. an RNA promoter sequence, preferably T3, T7 or SP6 RNA promotor sequences. A cloning vector is typically a vector that contains a cloning site, which may be used to incorporate nucleic acid sequences (insert) into the vector. A cloning vector may be, e.g., a plasmid vector or a bacteriophage vector. A transfer vector may be a vector which is suitable for transferring nucleic acid molecules into cells or organisms, for example, viral vectors. Preferably, a plasmid DNA vector

within the meaning of the present invention comprises a multiple cloning site, an RNA promoter sequence, optionally a selection marker, such as an antibiotic resistance factor, and a sequence suitable for multiplication of the vector, such as an origin of replication. The DNA, which forms the template of the RNA to be transcribed *in vitro,* may be prepared by fermentative proliferation and subsequent isolation as part of a plasmid which can be replicated in bacteria. Particularly preferred in the context of the present invention are plasmid DNA vectors, or expression vectors, comprising promoters for DNA-dependent RNA polymerases such as T3, T7 and Sp6. As plasmid backbone, particularly preferred are pUC19 and pBR322.

Antigen: In the context of the present invention "antigen" refers typically to a substance, which may be recognized by the immune system, preferably by the adaptive immune system, and is capable of triggering an antigen-specific immune response, e.g. by formation of antibodies and/or antigen-specific T cells as part of an adaptive immune response. Typically, an antigen may be or may comprise a peptide or protein which may be presented by the MHC to T-cells and comprises at least one epitope. In particular the antigen may be a pathogenic antigen or a tumor antigen.

Pathogenic antigens: The mRNA may encode a protein or a peptide, which comprises a pathogenic antigen or a fragment, variant or derivative thereof. Such pathogenic antigens are derived from pathogenic organisms, in particular bacterial, viral or protozoological (multicellular) pathogenic organisms, which evoke an immunological reaction in a subject, in particular a mammalian subject, more particularly a human. More specifically, pathogenic antigens are preferably surface antigens, e.g. proteins (or fragments of proteins, e.g. the exterior portion of a surface antigen) located at the surface of the virus or the bacterial or protozoological organism.

Pathogenic antigens are peptide or protein antigens preferably derived from a pathogen associated with infectious disease, which are preferably selected from antigens derived from the pathogens Acinetobacter baumannii, Anaplasma genus, Anaplasma phagocytophilum, Ancylostoma braziliense, Ancylostoma duodenale, Arcanobacterium haemolyticum, Ascaris lumbricoides, Aspergillus genus, Astroviridae, Babesia genus, Bacillus anthracis, Bacillus cereus, Bartonella henselae, BK virus, Blastocystis hominis, Blastomyces dermatitidis, Bordetella pertussis, Borrelia burgdorferi, Borrelia genus, Borrelia spp, Brucella genus, Brugia malayi, Bunyaviridae family, Burkholderia cepacia and other Burkholderia species, Burkholderia mallei, Burkholderia pseudomallei, Caliciviridae family, Campylobacter genus, Candida albicans, Candida spp, Chlamydia trachomatis, Chlamydophila pneumoniae, Chlamydophila psittaci, CJD prion, Clonorchis sinensis, Clostridium botulinum, Clostridium difficile, Clostridium perfringens, Clostridium perfringens, Clostridium spp, Clostridium tetani, Coccidioides spp, coronaviruses, Corynebacterium diphtheriae, Coxiella burnetii, Crimean-Congo hemorrhagic fever virus, Cryptococcus neoformans, Cryptosporidium genus, Cytomegalovirus (CMV), Dengue viruses (DEN-1, DEN-2, DEN-3 and DEN-4), Dientamoeba fragilis, Ebolavirus (EBOV), Echinococcus genus, Ehrlichia chaffeensis, Ehrlichia ewingii, Ehrlichia genus, Entamoeba histolytica, Enterococcus genus, Enterovirus genus, Enteroviruses, mainly Coxsackie A virus and Enterovirus 71 (EV71), Epidermophyton spp, Epstein-Barr Virus (EBV), Escherichia coli O157:H7, O111 and O104:H4, Fasciola hepatica and Fasciola gigantica, FFI prion, Filarioidea superfamily, Flaviviruses, Francisella tularensis, Fusobacterium genus, Geotrichum candidum, Giardia intestinalis, Gnathostoma spp, GSS prion, Guanarito virus, Haemophilus ducreyi, Haemophilus influenzae, Helicobacter pylori, Henipavirus (Hendra virus Nipah virus), Hepatitis A Virus, Hepatitis B Virus (HBV), Hepatitis C Virus (HCV), Hepatitis D Virus, Hepatitis E Virus, Herpes simplex virus 1 and 2 (HSV-1 and HSV-2), Histoplasma capsulatum, HIV (Human immunodeficiency virus), Hortaea werneckii, Human bocavirus (HBoV), Human herpesvirus 6 (HHV-6) and Human herpesvirus 7 (HHV-7), Human metapneumovirus (hMPV), Human papillomavirus (HPV), Human parainfluenza viruses (HPIV), Japanese encephalitis virus, JC virus, Junin virus, Kingella kingae, Klebsiella granulomatis, Kuru prion, Lassa virus, Legionella pneumophila, Leishmania genus, Leptospira genus, Listeria monocytogenes, Lymphocytic choriomeningitis virus (LCMV), Machupo virus, Malassezia spp, Marburg virus, Measles virus, Metagonimus yokagawai, Microsporidia phylum, Molluscum contagiosum virus (MCV), Mumps virus, Mycobacterium leprae and Mycobacterium lepromatosis, Mycobacterium tuberculosis, Mycobacterium ulcerans, Mycoplasma pneumoniae, Naegleria fowleri, Necator americanus, Neisseria gonorrhoeae, Neisseria meningitidis, Nocardia asteroides, Nocardia spp, Onchocerca volvulus, Orientia tsutsugamushi, Orthomyxoviridae family (Influenza), Paracoccidioides brasiliensis, Paragonimus spp, Paragonimus westermani, Parvovirus B19, Pasteurella genus, Plasmodium genus, Pneumocystis jirovecii, Poliovirus, Rabies virus, Respiratory syncytial virus (RSV), Rhinovirus, rhinoviruses, Rickettsia akari, Rickettsia genus, Rickettsia prowazekii, Rickettsia rickettsii, Rickettsia typhi, Rift Valley fever virus, Rotavirus, Rubella virus, Sabia virus, Salmonella genus, Sarcoptes scabiei, SARS coronavirus, Schistosoma genus, Shigella genus, Sin Nombre virus, Hantavirus, Sporothrix schenckii, Staphylococcus genus, Staphylococcus genus, Streptococcus agalactiae, Streptococcus pneumoniae, Streptococcus pyogenes, Strongyloides stercoralis, Taenia genus, Taenia solium, Tick-borne encephalitis virus (TBEV), Toxocara canis or Toxocara cati, Toxoplasma gondii, Treponema pallidum, Trichinella spiralis, Trichomonas vaginalis, Trichophyton spp, Trichuris trichiura, Trypanosoma brucei, Trypanosoma cruzi, Ureaplasma urealyticum, Varicella zoster virus (VZV), Varicella zoster virus (VZV), Variola major or Variola minor, vCJD prion, Venezuelan equine encephalitis virus, Vibrio cholerae, West Nile virus, Western equine encephalitis virus, Wuchereria bancrofti, Yellow fever virus, Yersinia enterocolitica, Yersinia pestis, and Yersinia pseudotuberculosis.

In this context particularly preferred are antigens from the pathogens selected from Influenza virus, respiratory

syncytial virus (RSV), Herpes simplex virus (HSV), human Papilloma virus (HPV), Human immunodeficiency virus (HIV), Plasmodium, Staphylococcus aureus, Dengue virus, Chlamydia trachomatis, Cytomegalovirus (CMV), Hepatitis B virus (HBV), Mycobacterium tuberculosis, Rabies virus, and Yellow Fever Virus.

In a preferred embodiment, the mRNA encodes a Rabies virus protein or peptide or an antigenic fragment thereof. Preferably, the mRNA encodes an antigenic protein or peptide selected from the group consisting of glycoprotein G (RAV-G), nucleoprotein N (RAV-N), phosphoprotein P (RAV-P), matrix protein M (RAV-M) or RNA polymerase L (RAV-L) of Rabies virus, or a fragment, variant or derivative thereof.

In another preferred embodiment, the mRNA according to the invention encodes a respiratory syncytial virus (RSV) protein or peptide or an antigenic fragment thereof. Preferably, the mRNA according to the invention encodes an antigenic protein or peptide selected from the group consisting of the fusion protein F, the glycoprotein G, the short hydrophobic protein SH, the matrix protein M, the nucleoprotein N, the large polymerase L, the M2-1 protein, the M2-2 protein, the phosphoprotein P, the non-structural protein NS1 or the non-structural protein NS2 of respiratory syncytial virus (RSV), or a fragment, variant or derivative thereof.

Tumour antigens: In a further embodiment, the mRNA according to the present invention may encode a protein or a peptide, which comprises a peptide or protein comprising a tumour antigen, a fragment, variant or derivative of said tumour antigen, preferably, wherein the tumour antigen is a melanocyte-specific antigen, a cancer-testis antigen or a tumour-specific antigen, preferably a CT-X antigen, a non-X CT-antigen, a binding partner for a CT-X antigen or a binding partner for a non-X CT-antigen or a tumour-specific antigen, more preferably a CT-X antigen, a binding partner for a non-X CT-antigen or a tumour-specific antigen or a fragment, variant or derivative of said tumour antigen; and wherein each of the nucleic acid sequences encodes a different peptide or protein; and wherein at least one of the nucleic acid sequences encodes for 5T4, 707-AP, 9D7, AFP, AlbZIP HPG1, alpha-5-beta-1-integrin, alpha-5-beta-6-integrin, alpha-actinin-4/m, alpha-methylacyl-coenzyme A racemase, ART-4, ARTC1/m, B7H4, BAGE-1, BCL-2, bcr/abl, beta-catenin/m, BING-4, BRCA1/m, BRCA2/m, CA 15-3/CA 27-29, CA 19-9, CA72-4, CA125, calreticulin, CAMEL, CASP-8/m, cathepsin B, cathepsin L, CD19, CD20, CD22, CD25, CDE30, CD33, CD4, CD52, CD55, CD56, CD80, CDC27/m, CDK4/m, CDKN2A/m, CEA, CLCA2, CML28, CML66, COA-1/m, coactosin-like protein, collage XXIII, COX-2, CT-9/BRD6, Cten, cyclin B1, cyclin D1, cyp-B, CYPB1, DAM-10, DAM-6, DEK-CAN, EFTUD2/m, EGFR, ELF2/m, EMMPRIN, EpCam, EphA2, EphA3, ErbB3, ETV6-AML1, EZH2, FGF-5, FN, Frau-1, G250, GAGE-1, GAGE-2, GAGE-3, GAGE-4, GAGE-5, GAGE-6, GAGE7b, GAGE-8, GDEP, GnT-V, gp100, GPC3, GPNMB/m, HAGE, HAST-2, hepsin, Her2/neu, HERV-K-MEL, HLA-A*0201-R17I, HLA-A11/m, HLA-A2/m, HNE, homeobox NKX3.1, HOM-TES-14/SCP-1, HOM-TES-85, HPV-E6, HPV-E7, HSP70-2M, HST-2, hTERT, iCE, IGF-1R, IL-13Ra2, IL-2R, IL-5, immature laminin receptor, kallikrein-2, kallikrein-4, Ki67, KIAA0205, KIAA0205/m, KK-LC-1, K-Ras/m, LAGE-A1, LDLR-FUT, MAGE-A1, MAGE-A2, MAGE-A3, MAGE-A4, MAGE-A6, MAGE-A9, MAGE-A10, MAGE-A12, MAGE-B1, MAGE-B2, MAGE-B3, MAGE-B4, MAGE-B5, MAGE-B6, MAGE-B10, MAGE-B16, MAGE-B17, MAGE-C1, MAGE-C2, MAGE-C3, MAGE-D1, MAGE-D2, MAGE-D4, MAGE-E1, MAGE-E2, MAGE-F1, MAGE-HI, MAGEL2, mammaglobin A, MART-1/melan-A, MART-2, MART-2/m, matrix protein 22, MC1R, M-CSF, ME1/m, mesothelin, MG50/PXDN, MMP11, MN/CA IX-antigen, MRP-3, MUC-1, MUC-2, MUM-1/m, MUM-2/m, MUM-3/m, myosin class I/m, NA88-A, N-acetylglucosaminyltransferase-V, Neo-PAP, Neo-PAP/m, NFYC/m, NGEP, NMP22, NPM/ALK, N-Ras/m, NSE, NY-ESO-1, NY-ESO-B, OA1, OFA-iLRP, OGT, OGT/m, OS-9, OS-9/m, osteocalcin, osteopontin, p15, p190 minor bcr-abl, p53, p53/m, PAGE-4, PAI-1, PAI-2, PAP, PART-1, PATE, PDEF, Pim-1-Kinase, Pin-1, Pml/PARalpha, POTE, PRAME, PRDX5/m, prostein, proteinase-3, PSA, PSCA, PSGR, PSM, PSMA, PTPRK/m, RAGE-1, RBAF600/m, RHAMM/CD168, RU1, RU2, S-100, SAGE, SART-1, SART-2, SART-3, SCC, SIRT2/m, Sp17, SSX-1, SSX-2/HOM-MEL-40, SSX-4, STAMP-1, STEAP-1, survivin, survivin-2B, SYT-SSX-1, SYT-SSX-2, TA-90, TAG-72, TARP, TEL-AML1, TGFbeta, TGFbetaRII, TGM-4, TPI/m, TRAG-3, TRG, TRP-1, TRP-2/6b, TRP/INT2, TRP-p8, tyrosinase, UPA, VEGFR1, VEGFR-2/FLK-1, WT1 and a immunoglobulin idiotype of a lymphoid blood cell or a T cell receptor idiotype of a lymphoid blood cell, or a fragment, variant or derivative of said tumour antigen; preferably survivin or a homologue thereof, an antigen from the MAGE-family or a binding partner thereof or a fragment, variant or derivative of said tumour antigen.

Particularly preferred in this context are the tumour antigens NY-ESO-1, 5T4, MAGE-C1, MAGE-C2, Survivin, Muc-1, PSA, PSMA, PSCA, STEAP and PAP.

The following combinations of antigens are particularly preferred:

- Muc-1, PSA, PSMA, PSCA, and STEAP
- Muc-1, PSA, PSMA, PSCA, and PAP
- Muc-1, PSA, PSMA, STEAP and PAP
- Muc-1, PSA, PSCA, STEAP and PAP
- Muc-1, PSMA, PSCA, STEAP and PAP
- PSA, PSMA, PSCA, STEAP and PAP
- Muc-1, PSA, PSMA, PSCA, STEAP and PAP

Further, the following combinations of antigens are particularly preffered:

- NY-ESO-1, 5T4, MAGE-C1, MAGE-C2, and Survivin
- NY-ESO-1, 5T4, MAGE-C1, MAGE-C2, and Muc-1
- NY-ESO-1, 5T4, MAGE-C1,, Survivin and Muc-1
- NY-ESO-1, 5T4,, MAGE-C2, Survivin and Muc-1
- NY-ESO-1, MAGE-C 1, MAGE-C2, Survivin and Muc-1
- 5T4, MAGE-C1, MAGE-C2, Survivin and Muc-1
- NY-ESO-1, 5T4, MAGE-C1, MAGE-C2, Survivin and Muc-1

Therapeutic proteins: Therapeutic proteins as defined herein are peptides or proteins which are beneficial for the treatment of any inherited or acquired disease or which improves the condition of an individual. Particularly, therapeutic proteins plays a big role in the creation of therapeutic agents that could modify and repair genetic errors, destroy cancer cells or pathogen infected cells, treat immune system disorders, treat metabolic or endocrine disorders, among other functions. For instance, Erythropoietin (EPO), a protein hormone can be utilized in treating patients with erythrocyte deficiency, which is a common cause of kidney complications. Furthermore adjuvant proteins, therapeutic antibodies are encompassed by therapeutic proteins and also hormone replacement therapy which is e.g. used in the therapy of women in the menopause. In newer approaches somatic cells of a patient are used to reprogram them into pluripotent stem cells which replace the disputed stem cell therapy. Also these proteins used for reprogramming of somatic cells or used for differentiating of stem cells are defined herein as therapeutic proteins. Furthermore therapeutic proteins may be used for other purposes e.g. wound healing, tissue regeneration, angiogenesis, etc.

Therefore therapeutic proteins can be used for various purposes including treatment of various diseases like e.g. infectious diseases, neoplasms (e.g. cancer or tumour diseases), diseases of the blood and blood-forming organs, endocrine, nutritional and metabolic diseases, diseases of the nervous system, diseases of the circulatory system, diseases of the respiratory system, diseases of the digestive system, diseases of the skin and subcutaneous tissue, diseases of the musculoskeletal system and connective tissue, and diseases of the genitourinary system, independently if they are inherited or acquired.

In this context, particularly preferred therapeutic proteins which can be used inter alia in the treatment of metabolic or endocrine disorders are selected from: Acid sphingomyelinase (Niemann-Pick disease), Adipotide (obesity), Agalsidase-beta (human galactosidase A) (Fabry disease; prevents accumulation of lipids that could lead to renal and cardiovascular complications), Alglucosidase (Pompe disease (glycogen storage disease type II)), alpha-galactosidase A (alpha-GAL A, Agalsidase alpha) (Fabry disease), alpha-glucosidase (Glycogen storage disease (GSD), Morbus Pompe), alpha-L-iduronidase (mucopolysaccharidoses (MPS), Hurler syndrome, Scheie syndrome), alpha-N-acetylglucosaminidase (Sanfilippo syndrome), Amphiregulin (cancer, metabolic disorder), Angiopoietin ((Ang1, Ang2, Ang3, Ang4, ANGPTL2, ANGPTL3, ANGPTL4, ANGPTL5, ANGPTL6, ANGPTL7) (angiogenesis, stabilize vessels), Betacellulin (metabolic disorder), Beta-glucuronidase (Sly syndrome), Bone morphogenetic protein BMPs (BMP1, BMP2, BMP3, BMP4, BMP5, BMP6, BMP7, BMP8a, BMP8b, BMP10, BMP15) (regenerative effect, bone-related conditions, chronic kidney disease (CKD)), CLN6 protein (CLN6 disease - Atypical Late Infantile, Late Onset variant, Early Juvenile, Neuronal Ceroid Lipofuscinoses (NCL)), Epidermal growth factor (EGF) (wound healing, regulation of cell growth, proliferation, and differentiation), Epigen (metabolic disorder), Epiregulin (metabolic disorder), Fibroblast Growth Factor (FGF, FGF-1, FGF-2, FGF-3, FGF-4, FGF-5, FGF-6, FGF-7, FGF-8, FGF-9, FGF-10, FGF-11, FGF-12, FGF-13, FGF-14, FGF-16, FGF-17, FGF-17, FGF-18, FGF-19, FGF-20, FGF-21, FGF-22, FGF-23) (wound healing, angiogenesis, endocrine disorders, tissue regeneration), Galsulphase (Mucopolysaccharidosis VI), Ghrelin (irritable bowel syndrome (IBS), obesity, Prader-Willi syndrome, type II diabetes mellitus), Glucocerebrosidase (Gaucher's disease), GM-CSF (regenerative effect, production of white blood cells, cancer), Heparin-binding EGF-like growth factor (HB-EGF) (wound healing, cardiac hypertrophy and heart development and function), Hepatocyte growth factor HGF (regenerative effect, wound healing), Hepcidin (iron metabolism disorders, Beta-thalassemia), Human albumin (Decreased production of albumin (hypoproteinaemia), increased loss of albumin (nephrotic syndrome), hypovolaemia, hyperbilirubinaemia), Idursulphase (Iduronate-2-sulphatase) (Mucopolysaccharidosis II (Hunter syndrome)), Integrins $\alpha$Vb3, $\alpha$Vb5 and $\alpha$5b1 (Bind matrix macromolecules and proteinases, angiogenesis), Iuduronate sulfatase (Hunter syndrome), Laronidase (Hurler and Hurler-Scheie forms of mucopolysaccharidosis I), N-acetylgalactosamine-4-sulfatase (rhASB; galsulfase, Arylsulfatase A (ARSA), Arylsulfatase B (ARSB)) (arylsulfatase B deficiency, Maroteaux-Lamy syndrome, mucopolysaccharidosis VI), N-acetylglucosamine-6-sulfatase (Sanfilippo syndrome), Nerve growth factor (NGF, Brain-Derived Neurotrophic Factor (BDNF), Neurotrophin-3 (NT-3), and Neurotrophin 4/5 (NT-4/5) (regenerative effect, cardiovascular diseases, coronary atherosclerosis, obesity, type 2 diabetes, metabolic syndrome, acute coronary syndromes, dementia, depression, schizophrenia, autism, Rett syndrome, anorexia nervosa, bulimia nervosa, wound healing, skin ulcers, corneal ulcers, Alzheimer's disease), Neuregulin (NRG1, NRG2, NRG3, NRG4) (metabolic disorder, schizophrenia), Neuropilin (NRP-1, NRP-2) (angiogenesis, axon guidance, cell survival, migration), Obestatin (irritable bowel syndrome (IBS), obesity, Prader-Willi syndrome, type II diabetes mellitus), Platelet Derived Growth factor (PDGF (PDFF-A, PDGF-B, PDGF-C, PDGF-D) (regenerative effect, wound healing, disorder in angiogenesis, Arteriosclerosis, Fibrosis, cancer), TGF beta receptors (endoglin, TGF-beta 1 receptor, TGF-beta 2 receptor, TGF-beta 3 receptor) (renal fibrosis, kidney disease, diabetes, ultimately end-stage renal disease (ESRD), angiogenesis), Thrombopoietin (THPO) (Megakaryocyte growth and development factor (MGDF)) (platelets disorders, platelets for donation, recovery of platelet counts after myelosuppressive chemotherapy), Transforming Growth factor (TGF (TGF-a, TGF-beta (TGFbeta1, TGFbeta2, and TGFbeta3))) (regenerative effect, wound healing, immunity, cancer, heart disease, diabetes, Marfan syndrome, Loeys-Dietz syndrome), VEGF (VEGF-A, VEGF-B, VEGF-C, VEGF-D, VEGF-E, VEGF-F und PIGF) (regenerative effect, angiogenesis, wound healing, cancer, permeability), Nesiritide (Acute decompensated congestive heart failure), Trypsin (Decubitus ulcer, varicose ulcer, debridement of eschar, dehiscent wound, sunburn, meconium ileus), adrenocorticotrophic hormone (ACTH) ("Addison's disease, Small cell carcinoma, Adrenoleukodystrophy, Congenital adrenal hyperplasia, Cushing's syndrome, Nelson's syndrome, Infantile spasms), Atrial-natriuretic peptide (ANP) (endocrine disorders), Cholecystokinin (diverse), Gastrin (hypogastrinemia), Leptin (Diabetes, hypertriglyceridemia, obesity), Oxytocin (stimulate breastfeeding, non-progression of parturition), Somatostatin (symptomatic treatment of carcinoid syndrome, acute variceal bleeding, and acromegaly, polycystic diseases of the liver and kidney, acromegaly and symptoms caused by neuroendocrine tumors), Vasopressin (antidiuretic hormone) (diabetes insipidus), Calcitonin (Postmenopausal osteoporosis, Hypercalcaemia, Paget's disease, Bone metastases, Phantom limb pain, Spinal Stenosis), Exenatide (Type 2 diabetes resistant to treatment with metformin and a sulphonylurea), Growth hormone (GH), somatotropin (Growth failure due to GH deficiency or chronic renal insufficiency, Prader-Willi syndrome, Turner syndrome, AIDS wasting or cachexia with antiviral therapy), Insulin (Diabetes mellitus, diabetic ketoacidosis, hyperkalaemia), Insulin-like growth factor 1 IGF-1 (Growth failure in children with GH gene deletion or severe primary IGF1 deficiency, neurodegenerative disease, cardiovascular diseases, heart failure), Mecasermin rinfabate, IGF-1 analog (Growth failure in children with GH gene deletion or severe primary IGF1 deficiency, neurodegenerative disease, cardiovascular diseases, heart failure), Mecasermin, IGF-1 analog (Growth failure in children with GH gene deletion or severe primary IGF1 deficiency, neurodegenerative disease, cardiovascular diseases, heart failure), Pegvisomant (Acromegaly), Pramlintide (Diabetes mellitus, in combination with insulin), Teriparatide (human parathyroid hormone residues 1-34) (Severe osteoporosis), Becaplermin (Debridement adjunct for diabetic ulcers), Dibotermin-alpha (Bone morphogenetic protein 2) (Spinal fusion surgery, bone injury repair), Histrelin acetate (gonadotropin releasing hormone; GnRH) (Precocious puberty), Octreotide (Acromegaly, symptomatic relief of VIP-secreting adenoma and metastatic carcinoid tumours), and Palifermin (keratinocyte growth factor; KGF) (Severe oral mucositis in patients undergoing chemotherapy, wound healing).
(in brackets is the particular disease for which the therapeutic protein is used in the treatment).

These and other proteins are understood to be therapeutic, as they are meant to treat the subject by replacing its defective endogenous production of a functional protein in sufficient amounts. Accordingly, such therapeutic proteins are typically mammalian, in particular human proteins.

For the treatment of blood disorders, diseases of the circulatory system, diseases of the respiratory system, cancer or tumour diseases, infectious diseases or immunedeficiencies following therapeutic proteins may be used: Alteplase (tissue plasminogen activator; tPA) (Pulmonary embolism, myocardial infarction, acute ischaemic stroke, occlusion of central venous access devices), Anistreplase (Thrombolysis), Antithrombin III (AT-III) (Hereditary AT-III deficiency, Thromboembolism), Bivalirudin (Reduce blood-clotting risk in coronary angioplasty and heparin-induced thrombocytopaenia), Darbepoetin-alpha (Treatment of anaemia in patients with chronic renal insufficiency and chronic renal failure (+/- dialysis)), Drotrecogin-alpha (activated protein C) (Severe sepsis with a high risk of death), Erythropoietin, Epoetin-alpha, erythropoetin, erthropoyetin (Anaemia of chronic disease, myleodysplasia, anaemia due to renal failure or chemotherapy, preoperative preparation), Factor IX (Haemophilia B), Factor VIIa (Haemorrhage in patients with haemophilia A or B and inhibitors to factor VIII or factor IX), Factor VIII (Haemophilia A), Lepirudin (Heparin-induced thrombocytopaenia), Protein C concentrate (Venous thrombosis, Purpura fulminans), Reteplase (deletion mutein of tPA) (Management of acute myocardial infarction, improvement of ventricular function), Streptokinase (Acute evolving transmural myocardial infarction, pulmonary embolism, deep vein thrombosis, arterial thrombosis or embolism, occlusion of arteriovenous cannula), Tenecteplase (Acute myocardial infarction), Urokinase (Pulmonary embolism), Angiostatin (Cancer), Anti-CD22 immunotoxin (Relapsed CD33+ acute myeloid leukaemia), Denileukin diftitox (Cutaneous T-cell lymphoma (CTCL)), Immunocyanin (bladder and prostate cancer), MPS (Metallopanstimulin) (Cancer), Aflibercept (Non-small cell lung cancer (NSCLC), metastatic colorectal cancer (mCRC), hormone-refractory metastatic prostate cancer, wet macular degeneration), Endostatin (Cancer, inflammatory diseases like rheumatoid arthritis as well as Crohn's disease, diabetic retinopathy, psoriasis, and endometriosis), Collagenase (Debridement of chronic dermal ulcers and severely burned areas, Dupuytren's contracture, Peyronie's disease), Human deoxyribonuclease I, dornase (Cystic fibrosis; decreases respiratory tract infections in selected patients with FVC greater than 40% of predicted), Hyaluronidase (Used as an adjuvant to increase the absorption and dispersion of injected drugs, particularly anaesthetics in ophthalmic surgery and certain imaging agents), Papain (Debridement of necrotic tissue or liquefication of slough in acute and chronic lesions, such as pressure ulcers, varicose and diabetic ulcers, burns, postoperative wounds, pilonidal cyst

wounds, carbuncles, and other wounds), L-Asparaginase (Acute lymphocytic leukaemia, which requires exogenous asparagine for proliferation), Peg-asparaginase (Acute lymphocytic leukaemia, which requires exogenous asparagine for proliferation), Rasburicase (Paediatric patients with leukaemia, lymphoma, and solid tumours who are undergoing anticancer therapy that may cause tumour lysis syndrome), Human chorionic gonadotropin (HCG) (Assisted reproduction), Human follicle-stimulating hormone (FSH) (Assisted reproduction), Lutropin-alpha (Infertility with luteinizing hormone deficiency), Prolactin (Hypoprolactinemia, serum prolactin deficiency, ovarian dysfunction in women, anxiety, arteriogenic erectile dysfunction, premature ejaculation, oligozoospermia, asthenospermia, hypofunction of seminal vesicles, hypoandrogenism in men), alpha-1-Proteinase inhibitor (Congenital antitrypsin deficiency), Lactase (Gas, bloating, cramps and diarrhoea due to inability to digest lactose), Pancreatic enzymes (lipase, amylase, protease) (Cystic fibrosis, chronic pancreatitis, pancreatic insufficiency, post-Billroth II gastric bypass surgery, pancreatic duct obstruction, steatorrhoea, poor digestion, gas, bloating), Adenosine deaminase (pegademase bovine, PEG-ADA) (Severe combined immunodeficiency disease due to adenosine deaminase deficiency), Abatacept (Rheumatoid arthritis (especially when refractory to TNFa inhibition)), Alefacept (Plaque Psoriasis), Anakinra (Rheumatoid arthritis), Etanercept (Rheumatoid arthritis, polyarticular-course juvenile rheumatoid arthritis, psoriatic arthritis, ankylosing spondylitis, plaque psoriasis, ankylosing spondylitis), Interleukin-1 (IL-1) receptor antagonist, Anakinra (inflammation and cartilage degradation associated with rheumatoid arthritis), Thymulin (neurodegenerative diseases, rheumatism, anorexia nervosa), TNF-alpha antagonist (autoimmune disorders such as rheumatoid arthritis, ankylosing spondylitis, Crohn's disease, psoriasis, hidradenitis suppurativa, refractory asthma), Enfuvirtide (HIV-1 infection), and Thymosin $\alpha$1 (Hepatitis B and C).
(in brackets is the particular disease, for which the therapeutic protein is used in the treatment)

Furthermore, adjuvant or immunostimulating proteins are also encompassed in the term therapeutic proteins. Adjuvant or immunostimulating proteins may be used in this context to induce, alter or improve an immune response in an individual to treat a particular disease or to ameliorate the condition of the individual.

In this context, adjuvant proteins may be selected from mammalian, in particular human adjuvant proteins, which typically comprise any human protein or peptide, which is capable of eliciting an innate immune response (in a mammal), e.g. as a reaction of the binding of an exogenous TLR ligand to a TLR. More preferably, human adjuvant proteins are selected from the group consisting of proteins, which are components and ligands of the signalling networks of the pattern recognition receptors including TLR, NLR and RLH, including TLR1, TLR2, TLR3, TLR4, TLR5, TLR6, TLR7, TLR8, TLR9, TLR10, TLR11; NOD1, NOD2, NOD3, NOD4, NOD5, NALP1, NALP2, NALP3, NALP4, NALP5, NALP6, NALP6, NALP7, NALP7, NALP8, NALP9, NALP10, NALP11, NALP12, NALP13, NALP14,1 IPAF, NAIP, CIITA, RIG-I, MDA5 and LGP2, the signal transducers of TLR signaling including adaptor proteins including e.g. Trif and Cardif; components of the Small-GTPases signalling (RhoA, Ras, Rac1, Cdc42, Rab etc.), components of the PIP signalling (PI3K, Src-Kinases, etc.), components of the MyD88-dependent signalling (MyD88, IRAK1, IRAK2, IRAK4, TIRAP, TRAF6 etc.), components of the MyD88-independent signalling (TICAM1, TICAM2, TRAF6, TBK1, IRF3, TAK1, IRAK1 etc.); the activated kinases including e.g. Akt, MEKK1, MKK1, MKK3, MKK4, MKK6, MKK7, ERK1, ERK2, GSK3, PKC kinases, PKD kinases, GSK3 kinases, JNK, p38MAPK, TAK1, IKK, and TAK1; the activated transcription factors including e.g. NF-kB, c-Fos, c-Jun, c-Myc, CREB, AP-1, Elk-1, ATF2, IRF-3, IRF-7.

Mammalian, in particular human adjuvant proteins may furthermore be selected from the group consisting of heat shock proteins, such as HSP10, HSP60, HSP65, HSP70, HSP75 and HSP90, gp96, Fibrinogen, TypIII repeat extra domain A of fibronectin; or components of the complement system including C1q, MBL, C1r, C1s, C2b, Bb, D, MASP-1, MASP-2, C4b, C3b, C5a, C3a, C4a, C5b, C6, C7, C8, C9, CR1, CR2, CR3, CR4, C1qR, C1INH, C4bp, MCP, DAF, H, I, P and CD59, or induced target genes including e.g. Beta-Defensin, cell surface proteins; or human adjuvant proteins including trif, flt-3 ligand, Gp96 or fibronectin, etc., or any species homolog of any of the above human adjuvant proteins.

Mammalian, in particular human adjuvant proteins may furthermore comprise cytokines which induce or enhance an innate immune response, including IL-1 alpha, IL1 beta, IL-2, IL-6, IL-7, IL-8, IL-9, IL-12, IL-13, IL-15, IL-16, IL-17, IL-18, IL-21, IL-23, TNFalpha, IFNalpha, IFNbeta, IFNgamma, GM-CSF, G-CSF, M-CSF; chemokines including IL-8, IP-10, MCP-1, MIP-1alpha, RANTES, Eotaxin, CCL21; cytokines which are released from macrophages, including IL-1, IL-6, IL-8, IL-12 and TNF-alpha; as well as IL-1R1 and IL-1 alpha.

Therapeutic proteins for the treatment of blood disorders, diseases of the circulatory system, diseases of the respiratory system, cancer or tumour diseases, infectious diseases or immunedeficiencies or adjuvant proteins are typically proteins of mammalian origin, preferably of human origin, depending on which animal shall be treated. A human subject, for example, is preferably treated by a therapeutic protein of human origin.

Pathogenic adjuvant proteins, typically comprise a pathogenic adjuvant protein, which is capable of eliciting an innate immune response (in a mammal), more preferably selected from pathogenic adjuvant proteins derived from bacteria, protozoa, viruses, or fungi, etc., e.g., bacterial (adjuvant) proteins, protozoan (adjuvant) proteins (e.g. profilin - like protein of Toxoplasma gondii), viral (adjuvant) proteins, or fungal (adjuvant) proteins, etc.

Particularly, bacterial (adjuvant) proteins may be selected from the group consisting of bacterial heat shock proteins or chaperons, including Hsp60, Hsp70, Hsp90, Hsp100; OmpA (Outer membrane protein) from gram-negative bacteria; bacterial porins, including OmpF; bacterial toxins, including pertussis toxin (PT) from *Bordetella pertussis,*

pertussis adenylate cyclase toxin CyaA and CyaC from *Bordetella pertussis,* PT-9K/129G mutant from pertussis toxin, pertussis adenylate cyclase toxin CyaA and CyaC from *Bordetella pertussis,* tetanus toxin, cholera toxin (CT), cholera toxin B-subunit, CTK63 mutant from cholera toxin, CTE112K mutant from CT, *Escherichia coli* heat-labile enterotoxin (LT), B subunit from heat-labile enterotoxin (LTB) *Escherichia coli* heat-labile enterotoxin mutants with reduced toxicity, including LTK63, LTR72; phenol-soluble modulin; neutrophil-activating protein (HP-NAP) from *Helicobacter pylori;* Surfactant protein D; Outer surface protein A lipoprotein from *Borrelia burgdorferi,* Ag38 (38 kDa antigen) from *Mycobacterium tuberculosis;* proteins from bacterial fimbriae; Enterotoxin CT of Vibrio cholerae, Pilin from pili from gram negative bacteria, and Surfactant protein A; etc., or any species homolog of any of the above bacterial (adjuvant) proteins.

Bacterial (adjuvant) proteins may also comprise bacterial flagellins. In the context of the present invention, bacterial flagellins may be selected from flagellins from organisms including, without being limited thereto, *Agrobacterium, Aquifex, Azospirillum, Bacillus, Bartonella, Bordetella, Borrelia, Burkholderia, Campylobacter, Caulobacte, Clostridium, Escherichia, Helicobacter, Lachnospiraceae, Legionella, Listeria, Proteus, Pseudomonas, Rhizobium, Rhodobacter, Roseburia, Salmonella, Serpulina, Serratia, Shigella, Treponema, Vibrio, Wolinella, Yersinia,* more preferably from flagellins from the species including, without being limited thereto, *Agrobacterium tumefaciens, Aquifex pyrophilus, Azospirillum brasilense, Bacillus subtilis, Bacillus thuringiensis, Bartonella bacilliformis, Bordetella bronchiseptica, Borrelia burgdorferi, Burkholderia cepacia, Campylobacter jejuni, Caulobacter crescentus, Clostridium botulinum strain Bennett clone 1, Escherichia coli, Helicobacter pylori, Lachnospiraceae bacterium, Legionella pneumophila, Listeria monocytogenes, Proteus mirabilis, Pseudomonas aeroguinosa, Pseudomonas syringae, Rhizobium meliloti, Rhodobacter sphaeroides, Roseburia cecicola, Roseburis hominis, Salmonella typhimurium, Salmonella bongori, Salmonella typhi, Salmonella enteritidis, Serpulina hyodysenteriae, Serratia marcescens, Shigella boydii, Treponema phagedenis, Vibrio alginolyticus, Vibrio cholerae, Vibrio parahaemolyticus, Wolinella succinogenes* and *Yersinia enterocolitica.*

Protozoan (adjuvant) proteins are a further example of pathogenic adjuvant proteins. Protozoan (adjuvant) proteins may be selected in this context from any protozoan protein showing adjuvant properties, more preferably, from the group consisting of, without being limited thereto, Tc52 from Trypanosoma cruzi, PFTG from Trypanosoma gondii, Protozoan heat shock proteins, LeIF from Leishmania spp., profiling-like protein from Toxoplasma gondii, etc.

Viral (adjuvant) proteins are another example of pathogenic adjuvant proteins. In this context, viral (adjuvant) proteins may be selected from any viral protein showing adjuvant properties, more preferably, from the group consisting of, without being limited thereto, Respiratory Syncytial Virus fusion glycoprotein (F-protein), envelope protein from MMT virus, mouse leukemia virus protein, Hemagglutinin protein of wild-type measles virus, etc.

Fungal (adjuvant) proteins are even a further example of pathogenic adjuvant proteins. In the context of the present invention, fungal (adjuvant) proteins may be selected from any fungal protein showing adjuvant properties, more preferably, from the group consisting of, fungal immunomodulatory protein (FIP; LZ-8), etc.

Finally, adjuvant proteins may furthermore be selected from the group consisting of, Keyhole limpet hemocyanin (KLH), OspA, etc.

In a further embodiment, therapeutic proteins may be used for hormone replacement therapy, particularly for the therapy of women in the menopause. These therapeutic proteins are preferably selected from oestrogens, progesterone or progestins, and sometimes testosterone.

Furthermore, therapeutic proteins may be used for reprogramming of somatic cells into pluri- or omnipotent stem cells. For this purpose, several factors are described, particularly Oct-3/4, Sox gene family (Sox1, Sox2, Sox3, and Sox15), Klf family (Klf1, Klf2, Klf4, and Klf5), Myc family (c-myc, L-myc, and N-myc), Nanog, and LIN28.

As mentioned above, also therapeutic antibodies are defined herein as therapeutic proteins. These therapeutic antibodies are preferably selected from antibodies, which are used inter alia for the treatment of cancer or tumour diseases, e.g. 131I-tositumomab (Follicular lymphoma, B cell lymphomas, leukemias), 3F8 (Neuroblastoma), 8H9, Abagovomab (Ovarian cancer), Adecatumumab (Prostate and breast cancer), Afutuzumab (Lymphoma), Alacizumab pegol, Alemtuzumab (B-cell chronic lymphocytic leukaemia, T-cell-Lymphoma), Amatuximab, AME-133v (Follicular lymphoma, cancer), AMG 102 (Advanced Renal Cell Carcinoma), Anatumomab mafenatox (Non-small cell lung carcinoma), Apolizumab (Solid Tumors, Leukemia, Non-Hodgkin-Lymphoma, Lymphoma), Bavituximab (Cancer, viral infections), Bectumomab (Non-Hodgkin's lymphoma), Belimumab (Non-Hodgkin lymphoma), Bevacizumab (Colon Cancer, Breast Cancer, Brain and Central Nervous System Tumors, Lung Cancer, Hepatocellular Carcinoma, Kidney Cancer, Breast Cancer, Pancreatic Cancer, Bladder Cancer, Sarcoma, Melanoma, Esophageal Cancer; Stomach Cancer, Metastatic Renal Cell Carcinoma; Kidney Cancer, Glioblastoma, Liver Cancer, Proliferative Diabetic Retinopathy, Macular Degeneration), Bivatuzumab mertansine (Squamous cell carcinoma), Blinatumomab, Brentuximab vedotin (Hematologic cancers), Cantuzumab (Colon Cancer, Gastric Cancer, Pancreatic Cancer, NSCLC), Cantuzumab mertansine (Colorectal cancer), Cantuzumab ravtansine (Cancers), Capromab pendetide (Prostate cancer), Carlumab, Catumaxomab (Ovarian Cancer, Fallopian Tube Neoplasms, Peritoneal Neoplasms), Cetuximab (Metastatic colorectal cancer and head and neck cancer), Citatuzumab bogatox (Ovarian cancer and other solid tumors), Cixutumumab (Solid tumors), Clivatuzumab tetraxetan (Pancreatic cancer), CNTO 328 (B-Cell Non-Hodgkin's Lymphoma, Multiple Myeloma, Castleman's Disease, ovarian cancer), CNTO 95 (Melanoma), Conatumumab, Dacetuzumab (Hematologic cancers), Dalotuzumab, Denosumab (Myeloma, Giant Cell Tumor of Bone, Breast Cancer, Prostate Cancer, Osteoporosis), Detumomab (Lymphoma), Drozitumab, Ecromeximab (Malignant melanoma), Edrecolomab (Colorectal carcinoma), Elotuzumab (Multiple myeloma), Elsilimomab, Enavatuzumab, Ensituximab, Epratuzumab (Autoimmune diseases, Systemic Lupus Erythematosus, Non-Hodgkin-Lymphoma, Leukemia), Ertumaxomab (Breast cancer), Ertumaxomab (Breast Cancer), Etaracizumab (Melanoma, prostate cancer, ovarian cancer), Farletuzumab (Ovarian cancer), FBTA05 (Chronic lymphocytic leukaemia), Ficlatuzumab (Cancer), Figitumumab (Adrenocortical carcinoma, non-small cell lung carcinoma), Flanvotumab (Melanoma), Galiximab (B-cell lymphoma), Galiximab (Non-Hodgkin-Lymphoma), Ganitumab, GC1008 (Advanced Renal Cell Carcinoma; Malignant Melanoma, Pulmonary Fibrosis), Gemtuzumab (Leukemia), Gemtuzumab ozogamicin (Acute myelogenous leukemia), Girentuximab (Clear cell renal cell carcinoma), Glembatumumab vedotin (Melanoma, breast cancer), GS6624 (Idiopathic pulmonary fibrosis and solid tumors), HuC242-DM4 (Colon Cancer, Gastric Cancer, Pancreatic Cancer), HuHMFG1 (Breast Cancer), HuN901-DM1 (Myeloma), Ibritumomab (Relapsed or refractory low-grade, follicular, or transformed B-cell non-Hodgkin's lymphoma (NHL)), Icrucumab, ID09C3 (Non-Hodgkin-Lymphoma), Indatuximab ravtansine, Inotuzumab ozogamicin, Intetumumab (Solid tumors (Prostate cancer, melanoma)), Ipilimumab (Sarcoma, Melanoma, Lung cancer, Ovarian Cancer leucemia, Lymphoma, Brain and Central Nervous System Tumors, Testicular Cancer, Prostate Cancer, Pancreatic Cancer, Breast Cancer), Iratumumab (Hodgkin's lymphoma), Labetuzumab (Colorectal cancer), Lexatumumab, Lintuzumab, Lorvotuzumab mertansine, Lucatumumab (Multiple myeloma, non-Hodgkin's lymphoma, Hodgkin's lymphoma), Lumiliximab (Chronic lymphocytic leukemia), Mapatumumab (Colon Cancer, Myeloma), Matuzumab (Lung Cancer, Cervical Cancer, Esophageal Cancer), MDX-060 (Hodgkin-Lymphoma, Lymphoma), MEDI 522 (Solid Tumors, Leukemia, Lymphoma, Small Intestine Cancer, Melanoma), Mitumomab (Small cell lung carcinoma), Mogamulizumab, MORab-003 (Ovarian Cancer, Fallopian Tube Cancer, Peritoneal Cancer), MORab-009 (Pancreatic Cancer, Mesothelioma, Ovarian Cancer, Non-Small Cell Lung Cancer, Fallopian Tube Cancer, Peritoneal Cavity Cancer), Moxetumomab pasudotox, MT103 (Non-Hodgkin-Lymphoma), Nacolomab tafenatox (Colorectal cancer), Naptumomab estafenatox (Non-small cell lung carcinoma, renal cell carcinoma), Narnatumab, Necitumumab (Non-small cell lung carcinoma), Nimotuzumab (Squamous cell carcinoma, head and neck cancer, nasopharyngeal cancer, glioma), Nimotuzumab (Squamous cell carcinomas, Glioma, Solid Tumors, Lung Cancer), Olaratumab, Onartuzumab (Cancer), Oportuzumab monatox, Oregovomab (Ovarian cancer), Oregovomab (Ovarian Cancer, Fallopian Tube Cancer, Peritoneal Cavity Cancer), PAM4 (Pancreatic Cancer), Panitumumab (Colon Cancer, Lung Cancer, Breast Cancer; Bladder Cancer; Ovarian Cancer), Patritumab, Pemtumomab, Pertuzumab (Breast Cancer, Ovarian Cancer, Lung Cancer, Prostate Cancer), Pritumumab (Brain cancer), Racotumomab, Radretumab, Ramucirumab (Solid tumors), Rilotumumab (Solid tumors), Rituximab (Urticaria, Rheumatoid Arthritis, Ulcerative Colitis, Chronic Focal Encephalitis, Non-Hodgkin-Lymphoma, Lymphoma, Chronic Lymphocytic Leukemia), Robatumumab, Samalizumab, SGN-30 (Hodgkin-Lymphoma, Lymphoma), SGN-40 (Non-Hodgkin-Lymphoma, Myeloma, Leukemia, Chronic Lymphocytic Leukemia), Sibrotuzumab, Siltuximab, Tabalumab (B-cell cancers), Tacatuzumab tetraxetan, Taplitumomab paptox, Tenatumomab, Teprotumumab (Hematologic tumors), TGN1412 (Chronic lymphocytic leukemia, rheumatoid arthritis), Ticilimumab (= tremelimumab), Tigatuzumab, TNX-650 (Hodgkin's lymphoma), Tositumomab (Follicular lymphoma, B cell lymphomas, Leukemias, Myeloma), Trastuzumab (Breast Cancer, Endometrial Cancer, Solid Tumors), TRBS07 (Melanoma), Tremelimumab, TRU-016 (Chronic lymphocytic leukemia), TRU-016 (Non-Hodgkin lymphoma), Tucotuzumab celmoleukin, Ublituximab, Urelumab, Veltuzumab (Non-Hodgkin's lymphoma), Veltuzumab (IMMU-106) (Non-Hodgkin's lymphoma), Volociximab (Renal Cell Carcinoma, Pancreatic Cancer, Melanoma), Votumumab (Colorectal tumors), WX-G250 (Renal Cell Carcinoma), Zalutumumab (Head and Neck Cancer, Squamous Cell Cancer), and Zanolimumab (T-Cell-Lymphoma);

antibodies, which are used inter alia for the treatment of immune disorders, e.g. Efalizumab (Psoriasis), Epratuzumab (Autoimmune diseases, Systemic Lupus Erythematosus, Non-Hodgkin-Lymphoma, Leukemia), Etrolizumab (inflammatory bowel disease), Fontolizumab (Crohn's disease), Ixekizumab (autoimmune diseases), Mepolizumab (Hypereosinophilie-Syndrom, Asthma, Eosinophilic Gastroenteritis, Churg-Strauss Syndrome, Eosinophilic Esophagitis), Milatuzumab (multiple myeloma and other hematological malignancies), Pooled immunoglobulins (Primary immunodeficiencies), Priliximab (Crohn's disease, multiple sclerosis), Rituximab (Urticaria, Rheumatoid Arthritis, Ulcerative Colitis, Chronic Focal Encephalitis, Non-Hodgkin-Lymphoma, Lymphoma, Chronic Lymphocytic Leukemia), Rontalizumab (systemic lupus erythematosus), Ruplizumab (rheumatic diseases), Sarilumab (rheumatoid arthritis, ankylosing spondylitis), Vedolizumab (Crohn's disease, ulcerative colitis), Visilizumab (Crohn's disease, ulcerative colitis), Reslizumab (inflammations of the airways, skin and gastrointestinal tract), Adalimumab (Rheumatoid arthritis, Crohn's disease, Ankylosing spondylitis, Psoriatic arthritis), Aselizumab (severely injured patients), Atinumab (treatment of neurologic systems), Atlizumab (rheumatoid arthritis, systemic juvenile idiopathic arthritis), Bertilimumab (severe allergic disorders), Besilesomab (inflammatory lesions and metastases), BMS-945429, ALD518 (cancer and rheumatoid arthritis), Briakinumab (psoriasis, rheumatoid arthritis, inflammatory bowel diseases, multiple sclerosis), Brodalumab (inflammatory diseases), Canakinumab (rheumatoid arthritis), Canakinumab (cryopyrin-associated periodic syndromes (CAPS), rheumatoid arthritis, chronic obstructive pulmonary disease), Certolizumab pegol (Crohn's disease), Erlizumab (heart attack, stroke, traumatic shock), Fezakinumab (rheumatoid arthritis, psoriasis), Golimumab (rheumatoid arthritis, psoriatic arthritis,

ankylosing spondylitis), Gomiliximab (allergic asthma), Infliximab (Rheumatoid arthritis, Crohn's disease, ankylosing spondylitis, psoriatic arthritis, plaque psoriasis, Morbus Bechterew, Colitis ulcerosa), Mavrilimumab (rheumatoid arthritis), Natalizumab (Multiple sclerosis), Ocrelizumab (multiple sclerosis, rheumatoid arthritis, lupus erythematosus, hematological cancer), Odulimomab (prevention of organ transplant rejections, immunological diseases), Ofatumumab (Chronic lymphocytic leukemia, follicular non-Hodgkin's lymphoma, B cell lymphoma, rheumatoid arthritis, relapsing remitting multiple sclerosis, Lymphoma, B-Cell Chronic Lymphocytic Leukemia), Ozoralizumab (inflammation), Pexelizumab (reduction of side effects of cardiac surgery), Rovelizumab (haemorrhagic shock), SBI-087 (Rheumatoid arthritis), SBI-087 (Systemic lupus erythematosus), Secukinumab (uveitis, rheumatoid arthritis psoriasis), Sirukumab (rheumatoid arthritis), Talizumab (allergic reaction), Tocilizumab (rheumatoid arthritis, systemic juvenile idiopathic arthritis, Castleman's disease), Toralizumab (rheumatoid arthritis, lupus nephriti), TRU-015 (Rheumatoid arthritis), TRU-016 (Autoimmune disease and inflammation), Ustekinumab (multiple sclerosis, psoriasis, psoriatic arthritis), Ustekinumab (IL-12/IL-23 blocker) (Plaque-Psoriasis, psoriatic arthritis, multiple sclerosis, sarcoidosis, the latter versus), Vepalimomab (inflammation), Zolimomab aritox (systemic lupus erythematosus, graft-versus-host disease), Sifalimumab (SLE, dermatomyositis, polymyositis), Lumiliximab (Allergies), and Rho(D) Immune Globulin (Rhesus disease); or are selected from antibodies used for the treatment of infectious diseases, e.g. Afelimomab (sepsis), CR6261 (infectious disease/influenza A), Edobacomab (sepsis caused by gram-negative bacteria), Efungumab (invasive Candida infection), Exbivirumab (hepatitis B), Felvizumab (respiratory syncytial virus infection), Foravirumab (rabies (prophylaxis)), Ibalizumab (HIV infection), Libivirumab (hepatitis B), Motavizumab (respiratory syncytial virus (prevention)), Nebacumab (sepsis), Tuvirumab (chronic hepatitis B), Urtoxazumab (diarrhoea caused by E. coli), Bavituximab (diverse viral infections), Pagibaximab (sepsis (e.g. Staphylococcus)), Palivizumab (prevention of respiratory syncytial virus infection in high-risk paediatric patients), Panobacumab (Pseudomonas aeruginosa infection), PRO 140 (HIV infection), Rafivirumab (rabies (prophylaxis)), Raxibacumab (anthrax (prophylaxis and treatment)), Regavirumab (cytomegalovirus infection), Sevirumab (cytomegalovirus infection), Suvizumab (viral infections), and Tefibazumab (Staphylococcus aureus infection);
antibodies, which are used inter alia for the treatment of blood disorders, e.g. Abciximab (percutaneous coronary intervention), Atorolimumab (hemolytic disease of the newborn), Eculizumab (Paroxysmal nocturnal haemoglobinuria), Mepolizumab (Hypereosinophilie-Syndrom, Asthma, Eosinophilic Gastroenteritis, Churg-Strauss Syndrome, Eosinophilic Esophagitis), and Milatuzumab (multiple myeloma and other hematological malignancies);
antibodies, which are used inter alia for immunoregulation, e.g. Antithymocyte globulin (Acute kidney transplant rejection, aplastic anaemia), Basiliximab (Prophylaxis against allograft rejection in renal transplant patients receiving an immunosuppressive regimen including cyclosporine and corticosteroids), Cedelizumab (prevention of organ transplant rejections, treatment of autoimmune diseases), Daclizumab (Prophylaxis against acute allograft rejection in patients receiving renal transplants, Multiple Sclerosis), Gavilimomab (graft versus host disease), Inolimomab (graft versus host disease), Muromonab-CD3 (prevention of organ transplant rejections), Muromonab-CD3 (Acute renal allograft rejection or steroid-resistant cardiac or hepatic allograft rejection), Odulimomab (prevention of organ transplant rejections, immunological diseases), and Siplizumab (psoriasis, graft-versus-host disease (prevention));
antibodies used for the treatment of diabetes, e.g. Gevokizumab (diabetes), Otelixizumab (diabetes mellitus type 1), and Teplizumab (diabetes mellitus type 1);
antibodies, which are used for the treatment of the Alzheimer's disease, e.g. Bapineuzumab, Crenezumab, Gantenerumab, Ponezumab, R1450, and Solanezumab;
antibodies, which are used for the treatment of asthma, e.g. Benralizumab, Enokizumab, Keliximab, Lebrikizumab, Omalizumab, Oxelumab, Pascolizumab, and Tralokinumab;
and antibodies, which are used for the treatment of diverse disorders, e.g. Blosozumab (osteoporosis), CaroRx (Tooth decay), Fresolimumab (idiopathic pulmonary fibrosis, focal segmental glomerulosclerosis, cancer), Fulranumab (pain), Romosozumab (osteoporosis), Stamulumab (muscular dystrophy), Tanezumab (pain), and Ranibizumab (Neovascular age-related macular degeneration).

Epitope: Epitopes (also called 'antigen determinant') can be distinguished in T cell epitopes and B cell epitopes. T cell epitopes or parts of the proteins in the context of the present invention may comprise fragments preferably having a length of about 6 to about 20 or even more amino acids, e.g. fragments as processed and presented by MHC class I molecules, preferably having a length of about 8 to about 10 amino acids, e.g. 8, 9, or 10, (or even 11, or 12 amino acids), or fragments as processed and presented by MHC class II molecules, preferably having a length of about 13 or more amino acids, e.g. 13, 14, 15, 16, 17, 18, 19, 20 or even more amino acids, wherein these fragments may be selected from any part of the amino acid sequence. These fragments are typically recognized by T cells in form of a complex consisting of the peptide fragment and an MHC molecule, i.e. the fragments are typically not recognized in their native form. B cell epitopes are typically fragments located on the outer surface of (native) protein or peptide antigens as defined herein, preferably having 5 to 15 amino acids, more preferably having 5 to 12 amino acids, even more preferably having 6 to 9 amino acids, which may be recognized by antibodies, i.e. in their native form.

Such epitopes of proteins or peptides may furthermore be selected from any of the herein mentioned variants of such proteins or peptides. In this context, antigenic determinants can be conformational or discontinuous epitopes,

which are composed of segments of the proteins or peptides as defined herein that are discontinuous in the amino acid sequence of the proteins or peptides as defined herein, but are brought together in the three-dimensional structure or continuous or linear epitopes, which are composed of a single polypeptide chain.

Protein: A protein typically comprises one or more peptides or polypeptides. A protein is typically folded into a 3-dimensional form, which may be required for the protein to exert its biological function.

Peptide: A peptide or polypeptide is typically a polymer of amino acid monomers, linked by peptide bonds. It typically contains less than 50 monomer units. Nevertheless, the term peptide is not a disclaimer for molecules having more than 50 monomer units. Long peptides are also called polypeptides, typically having between 50 and 600 monomeric units.

Fragment or part of a protein: Fragments or parts of a protein in the context of the present invention are typically understood to be peptides corresponding to a continuous part of the amino acid sequence of a protein, preferably having a length of about 6 to about 20 or even more amino acids, e.g. parts as processed and presented by MHC class I molecules, preferably having a length of about 8 to about 10 amino acids, e.g. 8, 9, or 10, (or even 11, or 12 amino acids), or fragments as processed and presented by MHC class II molecules, preferably having a length of about 13 or more amino acids, e.g. 13, 14, 15, 16, 17, 18, 19, 20 or even more amino acids, wherein these fragments may be selected from any part of the amino acid sequence. These fragments are typically recognized by T cells in form of a complex consisting of the peptide fragment and an MHC molecule, i.e. the fragments are typically not recognized in their native form. Fragments or parts of the proteins as defined herein may also comprise epitopes or functional sites of those proteins. Preferably, fragments or parts of a proteins in the context of the invention are antigens, particularly immunogens, e.g. antigen determinants (also called 'epitopes'), or do have antigenic characteristics, eliciting an adaptive immune response. Therefore, fragments of proteins or peptides may comprise at least one epitope of those proteins or peptides. Furthermore, also domains of a protein, like the extracellular domain, the intracellular domain or the transmembrane domain, and shortened or truncated versions of a protein may be understood to comprise a fragment of a protein.

Microorganism: A microorganism is a microscopic living organism, which may be single celled or multicellular. Microorganisms are very diverse and include all the bacteria and archaea and almost all the protozoa. They also include some fungi, algae, and certain animals, such as rotifers. In the context of the present invention, microorganism are particularly preferred which are able to replicate a covalently closed circular recombinant DNA molecule comprising an origin of replication e.g. a plasmid. Particularly preferred are bacteria, e.g. Escherichia coli (E. coli). Particularly preferred in this context are E. coli strains used for plasmid production e.g. DH1, DH5$\alpha$, DH10B, BL21 (e.g. BL21$\Delta$recA$\Delta$endA), or JM101. In some embodiments particularly DH5alpha strains are preferred, e.g. DH5$\alpha$ dcm-;and DH5$\alpha$ dcm- att$\lambda$::P5/6 6/6-RNA-IN- SacB.

Insert: An insert is a DNA sequence optionally comprised in the covalently closed circular DNA molecule according to the invention, encoding a target RNA sequence or a target peptide or protein sequence. In case the insert encodes a peptide or protein the insert comprises an open reading frame.

Fermentation: The terms "fermenting", "fermentation" or "fermentative production" refer to the bulk growth of microorganisms on a growth medium under aerobic or anaerobic conditions. Preferably, the fermentation reported herein refers to bacteria under anaerobic conditions. In the context of the present invention the terms particularly refer to fermentation processes in which covalently a closed circular recombinant DNA molecule comprising an origin of replication e.g. a plasmid is replicated by the microorganism.

Fed-batch fermentation: Fed batch fermentation is a process that comprises at least one step of predetermined or controlled addition of nutrients into the fermentation culture. This step of predetermined or controlled addition of nutrients is also named fed-batch phase and allows for control of growth rate at rates $<\mu_{max}$.

Primosome assembly site: The primosome assembly site is a nucleotide sequence which supports the assembly of the primosome. The primosome comprises the proteins DnaG primase, DnaB helicase, DnaC helicase assistant, DnaT, PriA, Pri B, and PriC. At each replication fork, the primosome is utilized once on the leading strand of DNA and repeatedly, initiating each Okazaki fragment, on the lagging DNA strand.

## Detailed Description of the Invention

To solve the above-mentioned problem, the present invention employs covalently closed circular DNA molecules as claimed in claim 1.

Therefore, in a first aspect, the invention relates to covalently closed circular recombinant DNA molecule comprising:

- a bacterial origin of replication,
- an insert comprising a homopolymeric region,

wherein the homopolymeric region comprises at least one homopolymeric sequence comprising a stretch of at least 15

identical nucleotides and is located at a distance of at least 500 bp from the origin of replication in the direction of replication.

In an embodiment thereof, the insert comprising a homopolymeric region is oriented so that the direction of transcription of the insert is the same as the direction of replication of the origin of replication.

The covalently closed circular recombinant DNA molecule may be may be selected from the group consisting of plasmid, cosmid, bacterial artificial chromosome (BAC), bacteriophage, viral vector or hybrids thereof. Preferably, the covalently closed circular recombinant DNA molecule is a plasmid. The plasmid may be any plasmid that is useful for the expression of RNA, such as pUC, pBluescript, pGEM, pTZ, pBR322, pACYC, pSC101, pET, pGEX, PColE1, PR6K, PSC101.

The homopolymeric region is located at a distance of at least 500 bp, at least 600 bp, at least 700 bp, at least 800 bp, at least 900 bp, at least 1000 bp, at least 1100 bp, at least 1200 bp, at least 1300 bp, at least 1400 bp, at least 1500 bp, at least 1600 bp, at least 1700 bp, at least 1800 bp, at least 1900 bp, at least 2000 bp, at least 2100 bp, at least 2200 bp, at least 2300 bp, at least 2400 bp from the origin of replication in the direction of replication. Preferably, the homopolymeric region is located at a distance of at least at least 550 bp, at least 600 bp, at least 650 bp, at least 700 bp, at least 750 bp, at least 800 bp, at least 850 bp, at least 900 bp, at least 950 bp, at least 1000 bp, at least 1050 bp, at least 1100 bp, at least 1200 bp, at least 1300 bp, at least 1400 bp, at least 1500 bp, at least 1600 bp, at least 1700 bp, at least 1800 bp, at least 1850 bp, at least 1900 bp, at least 1950 bp, at least 2000bp, at least 2050 bp, at least 2100 bp, at least 2150 bp, at least 2200 bp from the origin of replication in the direction of replication.

the covalently closed circular recombinant DNA molecule in which the insert comprising a homopolymeric region is oriented so that the direction of transcription of the insert is the same as the direction of replication of the origin of replication improves the yield of the covalently closed circular recombinant DNA molecule in fermentation production compared to the yield of a covalently closed circular recombinant DNA molecule in which the insert comprising the homopolymeric region is oriented so that the direction of transcription of the insert is opposite to the direction of replication of the origin of replication.

The distance from the origin of replication refers to the distance between the last nucleotide (3'-end) of the origin of replication and the first nucleotide (5'-end) of the homopolymeric region. The skilled person is aware of the sequences of commonly used origins of replication, therefore the last nucleotide (3'-end) of the origin of replication can be easily identified.

The plasmids used in the examples of the present application contain an origin derived from a pUC plasmid. In some examples, the origin of replication has the sequence identified in SEQ ID NO: 6. Hence, the last nucleotide of the sequence identified in SEQ ID NO: 6 is the last nucleotide (3'-end) of the origin of replication derived from a pUC plasmid as identified in SEQ ID NO: 6.

The covalently closed circular recombinant DNA molecule may for example be derived from the P1140-AF2 or from the P1140-K2 vector.

Thus, the covalently closed circular recombinant DNA molecule may comprise a sequence which is at least 90 % identical to SEQ ID NO: 4 or SEQ ID NO: 5. Preferably the covalently closed circular recombinant DNA molecule may comprise a sequence which is set forth in SEQ ID NO: 4 or SEQ ID NO: 5.

The "direction of replication" of an origin of replication denotes the direction in which the replication is performed. While the leading strand is continuously assembled in the direction of replication, the lagging strand is discontinuously assembled, i.e. the nucleotide polymers that are assembled in 5' to 3' direction are joined in order to allow an overall growth in 3' to 5' direction of the lagging strand. The direction of replication is usually indicated in the vector map of the plasmids.

The insert is a double strand DNA sequence coding for a target RNA comprising a homopolymeric region. Accordingly, the insert comprises a homopolymeric region. Preferably the insert comprises a nucleic acid sequence coding for a targert RNA and a homopolymeric region. The homopolymeric region is located at the 3' end of the insert. Preferably a poly(A) sequence and/or poly(C) sequence is located at the 3' end of the insert. More, preferably, a poly(A) sequence and or poly(C) sequence is located at the 3' end of the insert.

The sequence of the target RNA may comprises at least 20 bp, at least 30 bp, at least 50 bp, at least 100 bp, at least 200 bp, a least 300 bp, a at least 400 bp, at least 500 bp, at least 600 bp, at least 700 bp, at least 800 bp, at least 900 bp, at least 1000 bp, at least 1100 bp, at least 1200 bp, at least 1300 bp, at least 1400 bp, at least 1500 bp, at least 1600 bp, at least 1700 bp, at least 1800 bp, at least 1900 bp, at least 2000 bp, at least 2100 bp, at least 2200 bp, at least 2300 bp, at least 2400 bp.

The target RNA may be any RNA type described herein or may code for therapeutically active proteins or peptides, adjuvant proteins, antigens (tumor antigens, pathogenic antigens (e.g. selected, from animal antigens, from viral antigens, from protozoal antigens, from bacterial antigens), allergenic antigens, autoimmune antigens, or further antigens), allergens, antibodies, immunostimulatory proteins or peptides, antigen-specific T-cell receptors, cell penetrating peptides, secreted proteins, plasma membrane proteins, cytoplasmic or cytoskeletal proteins, intracellular membrane bound proteins, nuclear proteins, proteins associated with human disease, targeting moieties or those proteins encoded by the human genome, for which no therapeutic indication has been identified but which nonetheless have

utility in areas of research and discovery. Preferably the target RNA is an immunostimulating RNA or a coding RNA. The coding RNAs may be e.g. mRNAs, viral RNAs, or replicon RNAs.

The insert may comprise further elements as described herein, such as a histone stem-loop sequence, stabilization sequences, UTRs, IRES sequences, etc.

The poly(C) sequence may be a sequence of at least 15 cytidines, preferably at least 20 cytidines, more preferably at least 30 cytidines. Particularly, the insert may contain or code for a poly(C) sequence of typically about 20 to 50 or even 20 to 30 cytidine nucleotides. This poly(C) sequence is preferably located in the 3' region of the insert, i.e. at the 3' end of the coding region comprised

The homopolymeric region comprised in the insert of the covalently closed circular recombinant DNA molecule comprises at least one poly(A) sequence and/or at least one poly(C) sequence. In preferred embodiments the homopolymeric region comprises at least one poly(C) sequence. In more preferred embodiments the homopolymeric region comprises a poly (C) sequence. In even more preferred embodiments the homopolymeric region comprises a poly(C) and a poly(A) sequence. Preferably, the poly(A) sequence comprises a sequence of about 20 to about 400 adenosine nucleotides more preferably of about 60 to about 250 adenosine nucleotides. Preferably, the poly(C) sequence comprises a sequence of about 20 to 40 cytidine nucleotides. In particularly preferred embodiments the homopolymeric region comprises a poly(A) sequence of about 60 to about 70 adenosine nucleotides and a poly(C) sequence of about 20 to 30 cytidine nucleotides.

In a specific embodiment, the covalently closed circular recombinant DNA molecule comprises a poly(A) sequence and a poly(C) sequence.

The poly(A) sequence and the poly (C) sequence may be connected by a linker sequence. The linker sequence may be a heteropolymeric sequence. The linker sequence may comprises 1 to 400, preferably 1 to 100, more preferably 2 to 50, even more preferably 3 to 50, most preferably 3 to 100 nucleotides. The linker sequence may comprise a sequence such as 'tgcat'. Accordingly, the homopolymeric region comprising a poly(A) sequence and a poly(C) sequence may have for example a sequence as set forth in SEQ ID NO: 1.

Another aspect of the invention refers to a covalently closed circular recombinant DNA molecule according to the invention comprising an origin of replication and an insert comprising a homopolymeric region, wherein the insert comprising a homopolymeric region is oriented so that the direction of transcription of the insert is the same as the direction of replication of the origin of replication.

Particularly, the invention refers to a covalently closed circular recombinant DNA molecule according to the invention comprising an origin of replication and an insert comprising a homopolymeric region at the 3' end of the insert, wherein the insert is oriented so that the direction of transcription of the insert is the same as the direction of replication of the origin of replication.

The term "the direction of transcription of the insert is the same as the direction of replication of the origin of replication" means that the sequence is replicated in the same direction as it is transcribed, i.e. that the 5' end of the insert is closer to the origin of replication in the direction of the replication than the 3' end of the insert.

Thus between the origin of replication and the homopolymeric region there is the sequence coding for a target RNA.

Hence, in a covalently closed circular recombinant DNA molecule, in which the insert comprising the homopolymeric region is oriented so that the direction of the transcription of the insert is the same as the direction of replication of the origin of replication, the distance between the origin of replication and the homopolymeric region is larger compared a covalently closed circular recombinant DNA molecule, in which the insert comprising the homopolymeric region is oriented so that the direction of the transcription of the insert is opposite to the direction of replication of the origin or replication.

The yield of the covalently closed circular recombinant DNA molecule in which the homopolymeric region is located at a distance of least 500 bp from the origin of replication in the direction of replication is improved in fermentative production compared to the yield of a covalently closed circular recombinant DNA molecule in which the homopolymeric region is located at a distance of less than 500 bp from the origin of replication in the direction of replication. It is understood that the fermentation conditions of both covalently closed circular recombinant DNA molecules are the same.

Further, the covalently closed circular recombinant DNA molecule in which the insert comprising a homopolymeric region is oriented so that the direction of transcription of the insert is the same as the direction of replication of the origin of replication improves the yield of the covalently closed circular recombinant DNA molecule in fermentation production compared to the yield of a covalently closed circular recombinant DNA molecule in which the insert comprising the homopolymeric region is oriented so that the direction of transcription of the insert is opposite to the direction of replication of the origin of replication. It is understood that the fermentation conditions of both covalently closed circular recombinant DNA molecules are the same.

When the yield and/or fermentation time of a covalently closed circular recombinant DNA molecule in which the homopolymeric region is located at a distance of least 500 bp from the origin of replication in the direction of replication is compared to the yield of a covalently closed circular recombinant DNA molecule in which the homopolymeric region

is located at a distance of less than 500 bp from the origin of replication in the direction of replication, it is understood that the fermentation conditions of both covalently closed circular recombinant DNA molecules are the same.

Correspondingly, when the yield and/or fermentation time of a covalently closed circular recombinant DNA molecule in which the insert comprising a homopolymeric region is oriented so that the direction of transcription of the insert is the same as the direction of replication of the origin of replication is compared to the yield of a covalently closed circular recombinant DNA molecule in which the insert comprising the homopolymeric region is oriented so that the direction of transcription of the insert is opposite to the direction of replication of the origin of replication, it is understood, that the insert of both covalently closed circular recombinant DNA molecules is the same.

As can be seen from the vector map depicted in Figure 2, the orientation of P1140-AF2 is such that the direction of replication of the origin of application is the same as the direction of transcription of the insert. The vector map of P1140-AF1 in Figure 3 shows that the direction of transcription of the insert is opposite to the direction of replication of the origin of replication. As can be seen from Table 1 plasmid yield for the construct P1140-AF1 is 3.0 mg/L/$OD_{600}$, while the plasmid yield increases to 7.5 mg/L/$OD_{600}$ when the plasmid P1140-AF2 is used. Thus, the plasmid yield is increased 2.5 times for plasmid P1140-AF2 compared to P1140-AF1.

As can be seen from the vector map depicted in Figure 4, the orientation of P1140-K2 is such that the direction of replication of the origin of application is the same as the direction of transcription of the insert. The vector map of P1140-K1 in Figure 5 shows that the direction of the transcription of insert is opposite to the direction of replication of the origin of replication. As can be seen from Table 1 plasmid yield is in for the construct P1140-K1 1.9 mg/L/$OD_{600}$, while the plasmid yield increases to 9.3 mg/L/$OD_{600}$ when the plasmid P1140-K2 is used. Thus, the plasmid yield is increased 4.9 times for plasmid P1140-K2 compared to P1140-K1.

The yield of the covalently closed circular recombinant DNA molecule in which the insert comprising a homopolymeric region is oriented so that the direction of transcription of the insert is the same as the direction of replication of the origin of replication may be increased in fermentative production by at least 1.1 times, at least 1.2 times, at least 1.3 times, at least 1.4 times, at least 1.5 times, at least 1.6 times, at least 1.7 times, at least 1.8 times, at least 1.9 times, at least 2.0 times, at least 2.1 times, at least 2.2 times, at least 2.3 times, at least 2.4 times, at least 2.5 times, at least 2.6 times, at least 2.7 times, at least 2.8 times, at least 2.9 times, at least 3.0 times, at least 3.1 times, at least 3.2 times, at least 3.3 times, at least 3.4 times, at least 3.5 times, at least 3.6 times, at least 3.7 times, at least 3.8 times, at least 3.9 times, at least 4.0 times, at least 4.1 times, at least 4.2 times, at least 4.3 times, at least 4.4 times, at least 4.5 times, at least 4.6 times, at least 4.7 times, at least 4.8 times, at least 4.9 times, at least 5.0 times, at least 5.1 times, at least 5.2 times, at least 5.3 times, at least 5.4 times, at least 5.5 times or more in fermentative production compared to the yield of a covalently closed circular recombinant DNA molecule in which the insert comprising the homopolymeric region is oriented so that the direction of transcription of the insert is opposite to the direction of replication of the origin of replication. It is understood that the fermentation conditions of both covalently closed circular recombinant DNA molecules are the same.

The yield of the covalently closed circular recombinant DNA molecule in which the homopolymeric region is located at a distance of least 500 bp from the origin of replication in the direction of replication may increase the yield of the covalently closed circular recombinant DNA molecule in fermentative production by at least 1.1 times, at least at least 1.2 times, at least 1.3 times, at least 1.4 times, at least 1.5 times, at least 1.6 times, at least 1.7 times, at least 1.8 times, at least 1.9 times, at least 2.0 times, at least 2.1 times, at least 2.2 times, at least 2.3 times, at least 2.4 times, at least 2.5 times, at least 2.6 times, at least 2.7 times, at least 2.8 times, at least 2.9 times, at least 3.0 times, at least 3.1 times, at least 3.2 times, at least 3.3 times, at least 3.4 times, at least 3.5 times, at least 3.6 times, at least 3.7 times, at least 3.8 times, at least 3.9 times, at least 4.0 times, at least 4.1 times, at least 4.2 times, at least 4.3 times, at least 4.4 times, at least 4.5 times, at least 4.6 times, at least 4.7 times, at least 4.8 times, at least 4.9 times, at least 5.0 times, at least 5.1 times, at least 5.2 times, at least 5.3 times, at least 5.4 times, at least 5.5 times or more in fermentation production compared to the yield of a covalently closed circular recombinant DNA molecule in which the homopolymeric region is located at a distance of less than 500 bp from the origin of replication in the direction of replication. It is understood that the fermentation conditions of both covalently closed circular recombinant DNA molecules are the same.

The origin of replication is of bacterial origin. Usually, the origin of replication is unidirectional. The replication of a unidirectional origin of replication is carried out in one direction. Preferably, the origin of replication is a high copy number origin. The origin of replication may be derived from the pBR322 plasmid, pUC plasmid, pMB1 plasmid, ColE1 plasmid, R6K plasmid, p15 A plasmid, or pSC101 plasmid. The different origins of replication are known to the skilled in the art. Some of the above mentioned origins of replication are related. For example, the origin of replication derived from the pUC plasmid is a variant of the origin of replication derived from the pMB1 plasmid differing from the pMB1 origin in only two mutations which lead to higher copy numbers compared to the pMB1 plasmid. Preferably, the origin of replication is derived from the pUC plasmid.

In some embodiments, the covalently closed circular recombinant DNA molecule further comprises a primosome assembly site, for example a primosome assembly site in the leading strand (PAS-BH). The primosome assembly site may for example comprise the sequence SEQ ID NO: 3.

Typically, the covalently closed circular recombinant DNA molecule further comprises a sequence encoding a selection marker.

The term "sequence encoding a selection marker" refers to a sequence that encodes an RNA or polypeptide that provides a phenotype to the cell containing the sequence encoding a selection marker allowing either a positive or negative selection of cells containing the sequence encoding a selection marker. The sequence encoding a selection marker may be used to distinguish between transformed and non-transformed cells or may be used to identify cells having undergone recombination or other kinds of genetic modifications.

The skilled person is aware of different selection markers such as antibiotic resistance genes, for example the ampicillin or kanamycine resistance genes. Alternatively, antibiotic free selection systems may be used. Such antibiotic selection systems which can be used in the context of the present invention are described in Luke J et al.;Vaccine. 2009 Oct 30;27(46):6454-9). For example, a sucrose selectable marker may be used as selection marker. A sucrose selectable marker is e.g. the RNA-OUT system from Nature Technology Corporation (described in Luke J et al.;Vaccine. 2009 Oct 30;27(46):6454-9). Vectors contain and express a 150 bp RNA-OUT antisense RNA. RNA-OUT represses expression of a chromosomally integrated constitutively expressed counter-selectable marker (sacB), allowing plasmid selection on sucrose. SacB encodes a levansucrase, which is toxic in the presence of sucrose.

A further aspect refers to the use of a covalently closed circular recombinant DNA molecule according to the invention, in which the homopolymeric region is located at a distance of at least 500 bp from the origin of replication in the direction of replication for increasing the yield of the covalently closed circular recombinant DNA in fermentative production compared to the yield of a covalently closed circular recombinant DNA molecule in which the insert comprising the homopolymeric region is located at a distance of less than 500 bp from the origin of replication in the direction of replication.

The covalently closed circular recombinant DNA molecule may comprise at least one promotor for *in vitro* transcription, such as T3, Sp6 or T7 as well as a transcription terminator, for example trpA. Further, the covalently closed circular recombinant DNA molecule may comprise a plurality of insert sites and the insert sites may be clustered as part of a multiple cloning site. The covalently closed circular recombinant DNA molecule may also comprise more than one multiple cloning sites, which may be identical.

Another aspect refers to the use of a covalently closed circular recombinant DNA molecule according to the invention in which the insert comprising a homopolymeric region is oriented so that the direction of transcription of the insert is the same as the direction of replication of the origin of replication for increasing the yield of the covalently closed circular recombinant DNA in fermentation production compared to the yield of a covalently closed circular recombinant DNA molecule in which the insert comprising the homopolymeric region is oriented so that the direction of transcription of the insert is opposite to the direction of replication of the origin of replication.

In specific embodiments the fermentation time of a covalently closed circular recombinant DNA molecule, in which the homopolymeric region is located at a distance of least 500 bp from the origin of replication in the direction of replication compared to the fermentation time of a covalently closed circular recombinant DNA molecule in which the insert comprising the homopolymeric region is located at a distance of less than 500 bp from the origin of replication in the direction of replication is preferably reduced.

Reduced fermentation time means that the same yield is achieved within a shorter fermentation time.

The fermentation time of a covalently closed circular recombinant DNA molecule in which the insert comprising a homopolymeric region is oriented so that the direction of transcription of the insert is the same as the direction of replication of the origin of replication compared to the fermentation time of a covalently closed circular recombinant DNA molecule in which the insert comprising the homopolymeric region is oriented so that the direction of transcription of the insert is opposite to the direction of replication of the origin of replication is preferably reduced.

This means, for example, that the fermentation time is preferably reduced by more than 5%, by more than 10%, by more than 15%, by more than 16%, by more than 17%, by more than 18%, by more than 19%, by more than 20%, by more than 21%, by more than 22%, by more than 23%, by more than 24%,by more than 25%, by more than 26%, y more than 27%, by more than 28%, by more than 29%, by more than 30%, by more than 31%, by more than 32%. Preferably the fermentation time is reduced between 5% and 70%, more preferably between 10% and 50%, even more preferably between 15% and 35%.

A further aspect of the invention refers to a method for fermentative production of a covalently closed recombinant DNA molecule comprising the steps of:

(a) providing a microorganism comprising the covalently closed circular recombinant DNA molecule according to the invention;
(b) fermenting the microorganism of step (a).

In a specific embodiment, the method further comprises the following step:
(c) isolating the covalently closed circular recombinant DNA molecule from the microorganism of step(b).

The skilled person is aware of the standard techniques used for isolation of covalently closed circular recombinant DNA molecules.

In preferred embodiments the microorganism is a bacterium (e.g. E. coli) containing a covalently closed circular recombinant temperature inducible high copy DNA plasmid and step (b) comprises the following steps:

(i) growing the bacteria containing a covalently closed circular recombinant temperature inducible high copy DNA plasmid at a temperature in the range of 25°C to 32°C during the growth phase of the fed-batch phase wherein the substrate is supplied at a rate such that the growth rate is $\mu$=0.05 to 0.3hr$^{-1}$ during the fed-batch phase,
(ii) inducing production of said DNA plasmid after the growth phase by increasing the temperature to 36°C to 45°C; and
(iii) continuing fermentation at the temperature applied (ii) to accumulate the said DNA plasmid.

In more specific embodiments, step (b) comprises the following steps:

(i) growing the bacteria containing a covalently closed circular recombinant temperature inducible high copy DNA plasmid at a temperature of about 30°C during the growth phase of the fed-batch phase wherein the substrate is supplied at a rate such that the growth rate is $\mu$=0.05 to 0.3hr$^{-1}$ during the fed-batch phase,
(ii) inducing production of said DNA plasmid after the growth phase by increasing the temperature to about 42°C; and
(iii) continuing fermentation at the temperature applied (ii) to accumulate the said DNA plasmid.

This method of fed-batch fermentation is described in detail in EP 1 781 800 B1 and is termed herein as HyperGRO fermentation. Usually the method is carried out with temperature inducible covalently closed circular recombinant DNA molecules (e.g. pUC or pMM1 origin containing plasmids) at restricted cell growth rate and reduced temperature during the growth phase; plasmid production is then induced by increasing the temperature. Thereby the plasmid DNA fermentation yield increases, while the stability of the covalently closed circular recombinant DNA molecule is maintained or improved.

The growth phase in step (b) (i) can be performed at temperatures from 25-37°C, preferably at 30-37°C. For high copy plasmids, the growth phase is preferably performed at 30-32°C, more preferably at 30°C. The induction phase in step (b) (ii) may be carried out at temperatures from 33-45°C, preferably 37-42°C, most preferably at 42°C.

The skilled person is aware of different limiting feeding strategies in step (b) (i). For example a carbon limiting exponential feeding strategy may be employed. Briefly, an initial amount of carbon substrate is consumed during the batch phase at a specific growth rate $\mu_{max}$. Upon exhaustion of the carbon substrate, the fed-batch phase begins and feed nutrient is added automatically at a rate determined by the following equation

$$F(t) = \frac{\mu X_B V_B}{S_f Y_{X/S}} e^{\mu t}$$

Where $\mu$ = desired specific growth rate during fed-batch phase,
$X_B$ = biomass concentration at the end of the batch phase, g DCW/L
$V_B$ = initial liquid volume of culture, L;
$S_f$ = limiting substrate concentration in the nutrient feed medium, g/L:
$Y_{X/S}$ = yield coefficient of biomass from substrate, g/g,
t = time since beginning of fed-batch phase

It is an advantage of the covalently closed circular recombinant DNA molecule of the invention, that the sequence of the covalently closed circular recombinant DNA molecule remains stable during fermentation production.
For example, the homopolymeric region remains stable during fermentation production. In particular, the poly(A) sequence and/or poly(C) sequence remains stable during the fermentation production. The term "remains stable" as used herein means that there are no mutations, i.e. such as nucleotide substitutions, nucleotide deletions or nucleotide additions introduced during the fermentation production.

Another aspect of the invention refers to a method for improving the yield of covalently closed circular recombinant DNA molecule comprising the following steps:

(a) providing a covalently closed circular recombinant DNA molecule comprising

- a bacterial origin of replication, and
- an insert comprising a homopolymeric region comprising at least one homopolymeric sequence comprising a stretch of at least 15 identical nucleotides, wherein the homopolymeric sequence is located at a distance of

less than 500 bp from the origin of replication in the direction of replication;

(b) modifying the covalently closed circular recombinant DNA molecule of (a) so that the insert comprising a homopolymeric region (e.g. the 3'-end of the insert) is located at a distance of at least 500 bp from the origin of replication (e.g. the 5'-end of the origin of replication) in the direction of replication.

Modifying the covalently closed circular recombinant DNA molecule so that the insert comprising a homopolymeric region is located at a distance of at least 500 bp from the origin of replication in the direction of replication means for example, without limitation that a spacer sequence is inserted between the origin of replication and the insert comprising a homopolymeric region. Alternatively, the insert is removed from the covalently closed circular recombinant DNA and introduced in the opposite orientation. The covalently closed circular recombinant DNA may be modified by common cloning techniques known to skilled person in the art.

The invention further provides a method for improving the yield of a covalently closed circular recombinant DNA molecule comprising the following steps:

(a) providing a covalently closed circular recombinant DNA molecule comprising

- a bacterial origin of replication, and
- an insert comprising a homopolymeric region comprising at least one homopolymeric sequence comprising a stretch of at least 15 identical nucleotides, wherein the homopolymeric sequence is located at a distance of less than 500 bp from the origin of replication in the direction of replication, and wherein the insert comprising the homopolymeric region is oriented so that the direction of transcription of the insert is opposite to the direction of replication of the origin of replication;

(b) modifying the covalently closed circular recombinant DNA molecule of (a) so that the insert comprising a homopolymeric region is oriented so that the direction of transcription of the insert is the same as the direction of replication of the origin of replication.

Modifying the covalently closed circular recombinant DNA molecule so that the insert comprising a homopolymeric region is oriented so that the direction of transcription of the insert is the same as the direction of replication of the origin of replication, means for example, that the insert is removed from the covalently closed circular recombinant DNA and introduced in the opposite orientation. The skilled person is aware of the common cloning techniques necessary to remove and introduce nucleic acid sequences from/ into covalently closed circular recombinant DNA molecules.

Finally the invention refers the use of the covalently closed circular recombinant DNA molecule according the invention for in vitro transcription of RNA.

## Examples

The Examples shown in the following are merely illustrative and shall describe the present invention in a further way. These Examples shall not be construed to limit the present invention thereto.

### Plasmids:

Different vectors encoding GC-optimized mRNA encoding H1N1(Netherlands2009)-HA (as shown in Figure 6, SEQ ID NO: 2) were generated.

P1140 (shown in Figure 1):

The orientation of P1140 is such that the direction of replication of the origin of replication (denoted in the vector map as pUC origin) is opposite to the direction of transcription of the insert (denoted in the vector map as RNA).

As selection marker the vector encodes kanamycin (denoted in the vector map as KanR).

P1140-AF1 (shown in Figure 3):

The orientation of P1140-AF1 is such that the direction of replication of the origin of replication (denoted in the vector map as pUC origin) is opposite to the direction of transcription of the insert (denoted in the vector map as RNA).

As selection marker the vector encodes the RNA-OUT, an antisense RNA which represses expression of a chromosomally integrated constitutively expressed counter-selectable marker (sacB), allowing plasmid selection on

sucrose (denoted in the vector map as RNA-OUT).

P1140-AF2 (shown in Figure 2):

The orientation of P1140-AF2 is such that the direction of replication of the origin of replication (denoted in the vector map as pUC origin) is the same as the direction of transcription of the insert (RNA denoted in the vector map as RNA).

As selection marker the vector encodes the RNA-OUT, an antisense RNA which represses expression of a chromosomally integrated constitutively expressed counter-selectable marker (sacB), allowing plasmid selection on sucrose (denoted in the vector map as RNA-OUT).

P1140-K1 (as shown in Figure 5):

The orientation of P1140-K1 is such that the direction of replication of the origin of replication (denoted in the vector map as pUC origin) is opposite to the direction of transcription of the insert (denoted in the vector map as RNA).

As selection marker the vector encodes kanamycin (denoted in the vector map as KanR).

P1140-K2 (shown in Figure 4):

The orientation of P1140-K2 is such that the direction of replication of the origin of replication (denoted in the vector map as pUC origin) is the same as the direction of transcription of the insert (RNA denoted in the vector map as RNA).

As selection marker the vector encodes kanamycin (denoted in the vector map as KanR).

**HyperGRO fermentation:**

The plasmids P1140 (Figure 1), P1140-AF1 (Figure 3), P1140-AF2 (Figure 2), P1140-K1 (Figure 5), and P1140-K2 (Figure 4) were transformed into DH5α derived E. coli strains DH5α, DH5α dcm- or DH5α dcm- attλ::P5/6 6/6-RNA-IN- SacB (as indicated in Table 1) and propagated by HyperGRO fermentation. This method of fed-batch fermentation is described in detail in EP 1 781 800. The P1140 vector was slow growing and poor yielding in HyperGRO fermentation. The P1140-AF1 and the P1140-K1 cell line were also slow growing and poor yielding in HyperGRO fermentation (see **Table 1**). By contrast, the P1140-AF2 and P1140-K2 cell lines had normal growth and high production yields (see **Table 1**) By sequencing of the plasmids it could be confirmed that the poly(A) sequence was stable during fermentation in all tested plasmids.

**Table 1: Summary of HyperGRO fermentation**

| **Plasmid** | **Cell line** | **42°C plasmid yield (mg/L)** | **Harvest OD600** | **Fermentation duration (hours)** |
|---|---|---|---|---|
| P1140 | DH5α dcm- | 75 | 68 | 53 |
| P1140-AF1 | DH5α dcm-attλ::P5/6 6/6-RNA-IN- SacB | 223 | 75 | 45 |
| P1140-AF2 | DH5α dcm-attλ::P5/6 6/6-RNA-IN- SacB | 684 | 91 | 38 |
| P1140-K1 | DH5α | 130 | 68 | 38 |
| P1140-K2 | DH5α | 798 | 86 | 36 |

**Batch fermentation:**

These results have been confirmed using standard batch fermentation at 37°C showing that increasing the plasmid yield is independent of the specific fermentation protocol.

## Claims

**1.** Covalently closed circular recombinant DNA molecule comprising:

- a bacterial origin of replication,
- a promoter for a DNA-dependent RNA polymerase,
- an insert comprising a homopolymeric region,

wherein the homopolymeric region comprises at least one homopolymeric sequence comprising a stretch of at least 15 identical nucleotides and is located at a distance of at least 500 bp from the bacterial origin of replication in the direction of replication,
wherein the insert comprising a homopolymeric region is oriented so that the direction of transcription of the insert is the same as the direction of replication of the origin of replication.

**2.** Covalently closed circular recombinant DNA molecule of claim 1, wherein the homopolymeric region comprises at least one poly(C) sequence and/or at least one poly(A) sequence.

**3.** Covalently closed circular recombinant DNA molecule according to any one of the previous claims, wherein the covalently closed circular recombinant DNA molecule is selected from the group consisting of plasmid, cosmid, bacterial artificial chromosome (BAC), bacteriophage, viral vector or hybrids thereof, wherein preferably the covalently closed circular recombinant DNA molecule is a plasmid, more preferably a plasmid derived from the backbone having the sequence identified in SEQ ID NO: 4 or having the sequence identified in SEQ ID NO: 5.

**4.** Covalently closed circular recombinant DNA molecule according to any one of the previous claims, wherein the origin of replication is a high copy number origin, wherein preferably the origin of replication is derived from the pBR322 plasmid, pUC plasmid, pMB1 plasmid, ColE1 plasmid, R6K plasmid, p15A plasmid or pSC101 plasmid, more preferably from the pUC plasmid.

**5.** Covalently closed circular recombinant DNA molecule according to any one of the previous claims, wherein the covalently closed circular recombinant DNA molecule further comprises a primosome assembly site in the heavy strand (PAS-BH).

**6.** Covalently closed circular recombinant DNA molecule according to claims 2 to 5, wherein the poly(A) sequence comprises a sequence of about 20 to about 400 adenosine nucleotides, more preferably a sequence of about 60 to about 250 adenosine nucleotides.

**7.** Covalently closed circular recombinant DNA molecule according to claim 6, wherein the poly(C) sequence comprises a sequence of about 15 to 200 cytidine nucleotides, preferably of about 20 to 40 cytidine nucleotides.

**8.** Use of a covalently closed circular recombinant DNA molecule, according to any one of claims 1 to 7 for increasing the yield of the covalently closed circular recombinant DNA in fermentative production compared to the yield of a covalently closed circular recombinant DNA molecule in which the insert comprising the homopolymeric region is located at a distance of less than 500 bp from the origin of replication in the direction of replication.

**9.** Use of a covalently closed circular recombinant DNA molecule, according to any one of claims 3 to 7 for increasing the yield of the covalently closed circular recombinant DNA in fermentative production compared to the yield of a covalently closed circular recombinant DNA molecule in which the insert comprising the homopolymeric region is oriented so that the direction of transcription of the insert is opposite to the direction of replication of the origin of replication.

**10.** Method for fermentative production of a covalently closed recombinant DNA molecule comprising the steps of:

(a) providing a microorganism comprising the covalently closed circular recombinant DNA molecule according to claims 1 to 7;
(b) fermenting the microorganism of step (a),optionally further comprising a step of
(c) isolating the covalently closed circular recombinant DNA molecule from the microorganism of step (b).

**11.** Method of claim 10, wherein the microorganism is a bacterium containing a covalently closed circular recombinant temperature inducible high copy DNA plasmid and step (b) comprises the following steps:

(i) growing the bacteria containing a covalently closed circular recombinant temperature inducible high copy DNA plasmid at a temperature in the range of 25°C to 32°C, preferably at about 30°C, during the growth phase of the fed-batch phase wherein the substrate is supplied at a rate such that the growth rate is $\mu$=0.05 to 0.3$hr^{-1}$ during the fed-batch phase,
(ii) inducing production of said DNA plasmid after the growth phase by increasing the temperature to 36°C to 45°C, preferably to about 42°C; and
(iii) continuing fermentation at the temperature applied (ii) to accumulate the said DNA plasmid.

**12.** Method according to any one of claim 10 or 11, wherein the yield of the covalently closed circular recombinant DNA molecule according to claims 1 to 7 is increased compared to the yield of a covalently closed circular recombinant DNA molecule in which the insert comprising homopolymeric region is located at a distance of less than 500 bp from the origin of replication in the direction of replication or compared to the yield of a covalently closed circular recombinant DNA molecule in which the insert comprising the homopolymeric region is oriented so that the direction of transcription of the insert is opposite to the direction of replication of the origin of replication.

**13.** Method according to any one of claims 10 to 12, wherein the homopolymeric region sequence or the sequence of the covalently closed circular recombinant DNA molecule remains stable during the fermentation production.

14. Method for improving the yield of a covalently closed circular recombinant DNA molecule comprising the following steps:

(a) providing a covalently closed circular recombinant DNA molecule having

- a bacterial origin of replication, and
- an insert comprising a homopolymeric region comprising at least one homopolymeric sequence comprising a stretch of at least 15 identical nucleotides, wherein the homopolymeric region is located at a distance of less than 500 bp from the bacterial origin of replication in the direction of replication;

(b) modifying the covalently closed circular recombinant DNA molecule of (a) so that the insert comprising a homopolymeric region sequence is located at a distance of at least 500 bp from the bacterial origin of replication in the direction of replication.

15. Method for improving the yield of a covalently closed circular recombinant DNA molecule comprising the following steps:

(a) providing a covalently closed circular recombinant DNA molecule having

- a bacterial origin of replication, and
- an insert comprising a homopolymeric region comprising at least one homopolymeric sequence comprising a stretch of at least 15 identical nucleotides, wherein the insert comprising the homopolymeric region sequence is oriented so that the direction of transcription of the insert is opposite to the direction of replication of the bacterial origin of replication, and wherein the homopolymeric region is located at a distance of at least 500 bp from the bacterial origin of replication in the direction of replication;

(b) modifying the covalently closed circular recombinant DNA molecule of (a) so that the insert comprising a homopolymeric region is oriented so that the direction of transcription of the insert is the same as the direction of replication of the bacterial origin of replication.

16. Use of the covalently closed circular recombinant DNA molecule according to claims 1 to 7 for the *in vitro* transcription of RNA.

## Patentansprüche

1. Kovalent geschlossenes zirkuläres rekombinantes DNA-Molekül, umfassend:

- einen bakteriellen Replikationsursprung,
- einen Promotor für eine DNA-abhängige RNA-Polymerase,
- ein Insert, das eine homopolymere Region umfasst,

wobei die homopolymere Region mindestens eine homopolymere Sequenz umfasst, die einen Abschnitt von mindestens 15 identischen Nukleotiden umfasst und sich in einem Abstand von mindestens 500 bp in Replikationsrichtung von dem bakteriellen Replikationsursprung befindet, wobei das Insert, das eine homopolymere Region umfasst, so ausgerichtet ist, dass die Transkriptionsrichtung des Inserts mit der Replikationsrichtung des Replikationsursprungs identisch ist.

2. Kovalent geschlossenes zirkuläres rekombinantes DNA-Molekül nach Anspruch 1, wobei die homopolymere Region mindestens eine Poly(C)-Sequenz und/oder mindestens eine Poly(A)-Sequenz umfasst.

3. Kovalent geschlossenes zirkuläres rekombinantes DNA-Molekül nach einem der vorhergehenden Ansprüche, wobei das kovalent geschlossene zirkuläre rekombinante DNA-Molekül ausgewählt ist aus der Gruppe bestehend aus Plasmid, Cosmid, bakteriellem künstlichem Chromosom (BAC), Bakteriophagen, viralem Vektor oder Hybriden davon, wobei vorzugsweise das kovalent geschlossene zirkuläre rekombinante DNA-Molekül ein Plasmid ist, bevorzugter ein Plasmid, das von dem Grundgerüst, das die in SEQ ID NO: 4 identifizierte Sequenz hat, abgeleitet ist oder das die in SEQ ID NO: 5 identifizierte Sequenz hat.

4. Kovalent geschlossenes zirkuläres rekombinantes DNA-Molekül nach einem der vorhergehenden Ansprüche, wobei der Replikationsursprung ein Ursprung mit hoher Kopiezahl ist, wobei vorzugsweise der Replikationsursprung von dem pBR322-Plasmid, pUC-Plasmid, pMB1-Plasmid, ColE1-Plasmid, R6K-Plasmid, p15A-Plasmid oder pSC101-Plasmid abgeleitet ist, bevorzugter von dem pUC-Plasmid.

5. Kovalent geschlossenes zirkuläres rekombinantes DNA-Molekül nach einem der vorhergehenden Ansprüche, wobei das kovalent geschlossene zirkuläre rekombinante DNA-Molekül weiterhin eine Primosomenassemblierungsstelle auf dem schweren Strang (PAS-BH) umfasst.

6. Kovalent geschlossenes zirkuläres rekombinantes DNA-Molekül nach den Ansprüchen 2 bis 5, wobei die Poly(A)-Sequenz eine Sequenz von etwa 20 bis etwa 400 Adenosinnukleotiden umfasst, bevorzugter eine Sequenz von etwa 60 bis etwa 250 Adenosinnukleotiden.

7. Kovalent geschlossenes zirkuläres rekombinantes DNA-Molekül nach Anspruch 6, wobei die Poly(C)-Sequenz eine Sequenz von etwa 15 bis 200 Cytidinnukleotiden, vorzugsweise von etwa 20 bis 40 Cytidinnukleotiden, umfasst.

8. Verwendung eines kovalent geschlossenen zirkulären rekombinanten DNA-Moleküls nach einem der Ansprüche 1 bis 7 zur Erhöhung der Ausbeute der kovalent geschlossenen zirkulären rekombinanten DNA bei der fermentativen Produktion im Vergleich zur Ausbeute eines kovalent geschlossenen zirkulären rekombinanten DNA-Moleküls, in dem das Insert, das die homopolymere Region umfasst, sich in einem Abstand von weniger als 500 bp in Replikationsrichtung von dem Replikationsursprung befindet.

9. Verwendung eines kovalent geschlossenen zirkulären rekombinanten DNA-Moleküls nach einem der Ansprüche 3 bis 7 zur Erhöhung der Ausbeute der kovalent geschlossenen zirkulären rekombinanten DNA bei der fermentativen Produktion im Vergleich zur Ausbeute eines kovalent geschlossenen zirkulären rekombinanten DNA-Moleküls, in dem das Insert, das die homopolymere Region umfasst, so ausgerichtet ist, dass die Transkriptionsrichtung des Inserts der Replikationsrichtung des Replikationsursprungs entgegengesetzt ist.

10. Verfahren zur fermentativen Herstellung eines kovalent geschlossenen rekombinanten DNA-Moleküls, umfassend die Schritte:

(a) Bereitstellen eines Mikroorganismus, der das kovalent geschlossene zirkuläre rekombinante DNA-Molekül gemäß den Ansprüchen 1 bis 7 umfasst;
(b) Fermentieren des Mikroorganismus aus Schritt (a), optional weiterhin umfassend einen Schritt von
(c) Isolieren des kovalent geschlossenen zirkulären rekombinanten DNA-Moleküls aus dem Mikroorganismus aus Schritt (b).

11. Verfahren nach Anspruch 10, wobei der Mikroorganismus ein Bakterium ist, das ein kovalent geschlossenes zirkuläres rekombinantes temperaturinduzierbares DNA-Plasmid mit hoher Kopiezahl enthält, und Schritt (b) die folgenden Schritte umfasst:

(i) Züchten der Bakterien, die ein kovalent geschlossenes zirkuläres rekombinantes temperaturinduzierbares DNA-Plasmid mit hoher Kopiezahl enthalten, bei einer Temperatur im Bereich von 25°C bis 32°C, vorzugsweise bei etwa 30°C, während der Wachstumsphase der Fed-Batch-Phase wobei das Substrat mit einer solchen Geschwindigkeit zugeführt wird, dass die Wachstumsrate während der Fed-Batch-Phase $\mu$=0,05 bis 0,3 Stunden$^{-1}$ beträgt;
(ii) Induzieren der Produktion des DNA-Plasmids nach der Wachstumsphase durch Erhöhen der Temperatur auf 36°C bis 45°C, vorzugsweise auf etwa 42°C; und
(iii) Fortsetzen der Fermentation bei der angewendeten Temperatur (ii) zur Anhäufung des DNA-Plasmids.

12. Verfahren nach einem der Ansprüche 10 oder 11, wobei die Ausbeute des kovalent geschlossenen zirkulären rekombinanten DNA-Moleküls nach den Ansprüchen 1 bis 7 im Vergleich zu der Ausbeute eines kovalent geschlossenen zirkulären rekombinanten DNA-Moleküls, in dem das Insert, das die homopolymere Region umfasst, sich in einem Abstand von weniger als 500 bp in Replikationsrichtung von dem Replikationsursprung befindet, oder im Vergleich zu der Ausbeute eines kovalent geschlossenen zirkulären rekombinanten DNA-Moleküls, in dem das Insert, das die homopolymere Region umfasst, so ausgerichtet ist, dass die Transkriptionsrichtung des Inserts der Replikationsrichtung des Replikationsursprungs entgegengesetzt ist erhöht ist.

**13.** Verfahren nach einem der Ansprüche 10 bis 12, wobei die Sequenz der homopolymeren Region oder die Sequenz des kovalent geschlossenen zirkulären rekombinanten DNA-Moleküls während der Fermentationsproduktion stabil bleibt.

**14.** Verfahren zur Verbesserung der Ausbeute eines kovalent geschlossenen zirkulären rekombinanten DNA-Moleküls, umfassend die folgenden Schritte:

(a) Bereitstellen eines kovalent geschlossenen zirkulären rekombinanten DNA-Moleküls mit

- einem bakteriellen Replikationsursprung und
- einem Insert umfassend eine homopolymere Region, umfassend mindestens eine homopolymere Sequenz umfassend einen Abschnitt von mindestens 15 identischen Nukleotiden, wobei sich die homopolymere Region in einem Abstand von weniger als 500 bp in Replikationsrichtung von dem bakteriellen Replikationsursprung befindet;

(b) Modifizieren des kovalent geschlossenen zirkulären rekombinanten DNA-Moleküls aus (a), so dass sich das Insert, das eine Sequenz der homopolymeren Region umfasst, in einem Abstand von mindestens 500 bp in Replikationsrichtung von dem bakteriellen Replikationsursprung befindet.

**15.** Verfahren zur Verbesserung der Ausbeute eines kovalent geschlossenen zirkulären rekombinanten DNA-Moleküls, umfassend die folgenden Schritte:

(a) Bereitstellen eines kovalent geschlossenen zirkulären rekombinanten DNA-Moleküls mit

- einem bakteriellen Replikationsursprung und
- einem Insert umfassend eine homopolymere Region umfassend mindestens eine homopolymere Sequenz umfassend einen Abschnitt von mindestens 15 identischen Nukleotiden, wobei das Insert, das die Sequenz der homopolymeren Region umfasst, so ausgerichtet ist, dass die Transkriptionsrichtung des Inserts der Replikationsrichtung des bakteriellen Replikationsursprungs entgegengesetzt ist, und wobei sich die homopolymere Region in einem Abstand von mindestens 500 bp in Replikationsrichtung von dem bakteriellen Replikationsursprung befindet;

(b) Modifizieren des kovalent geschlossenen zirkulären rekombinanten DNA-Moleküls aus (a), so dass das Insert, das eine homopolymere Region umfasst, so ausgerichtet ist, dass die Transkriptionsrichtung des Inserts mit der Replikationsrichtung des bakteriellen Replikationsursprungs identisch ist.

**16.** Verwendung des kovalent geschlossenen zirkulären rekombinanten DNA-Moleküls nach den Ansprüchen 1 bis 7 für die *in-vitro-* Transkription von RNA.

## Revendications

**1.** Molécule d'ADN recombinant circulaire fermée de manière covalente comprenant :

- une origine de réplication bactérienne,
- un promoteur pour une ARN polymérase dépendante de l'ADN,
- un insert comprenant une région homopolymère,

la région homopolymère comprenant au moins une séquence homopolymère comprenant un segment d'au moins 15 nucléotides identiques et est située à une distance d'au moins 500 pb de l'origine de réplication bactérienne dans la direction de réplication, l'insert comprenant une région homopolymère étant orienté de manière à ce que la direction de transcription de l'insert soit la même que la direction de réplication de l'origine de réplication.

**2.** Molécule d'ADN recombinant circulaire fermée de manière covalente selon la revendication 1, dans laquelle la région homopolymère comprend au moins une séquence poly(C) et/ou au moins une séquence poly(A).

**3.** Molécule d'ADN recombinant circulaire fermée de manière covalente selon l'une quelconque des revendications précédentes, dans laquelle la molécule d'ADN recombinant circulaire fermée de manière covalente est choisie dans

le groupe constitué par un plasmide, un cosmide, un chromosome artificiel bactérien (BAC), un bactériophage, un vecteur viral ou ses hybrides, de préférence la molécule d'ADN recombinant circulaire fermée de manière covalente étant un plasmide, davantage de préférence un plasmide dérivé du squelette ayant la séquence identifiée dans SEQ ID n° 4 ou ayant la séquence identifiée dans SEQ ID n° 5.

**4.** Molécule d'ADN recombinant circulaire fermée de manière covalente selon l'une quelconque des revendications précédentes, dans laquelle l'origine de réplication est une origine à haut nombre de copies de préférence l'origine de réplication étant dérivée du plasmide pBR322, du plasmide pUC, du plasmide pMB1, du plasmide ColE1, du plasmide R6K, du plasmide p15A ou du plasmide pSC101, davantage de préférence du plasmide pUC.

**5.** Molécule d'ADN recombinant circulaire fermée de manière covalente selon l'une quelconque des revendications précédentes, dans laquelle la molécule d'ADN recombinant circulaire fermée de manière covalente comprend en outre un site d'assemblage de primosome dans la chaîne lourde (PAS-BH) .

**6.** Molécule d'ADN recombinant circulaire fermée de manière covalente selon les revendications 2 à 5, dans laquelle la séquence poly(A) comprend une séquence d'environ 20 à environ 400 nucléotides d'adénosine, davantage de préférence une séquence d'environ 60 à environ 250 nucléotides d'adénosine.

**7.** Molécule d'ADN recombinant circulaire fermée de manière covalente selon la revendication 6, dans laquelle la séquence poly(C) comprend une séquence d'environ 15 à 200 nucléotides de cytidine, de préférence d'environ 20 à 40 nucléotides de cytidine.

**8.** Utilisation d'une molécule d'ADN recombinant circulaire fermée de manière covalente, selon l'une quelconque des revendications 1 à 7 pour augmenter le rendement de la molécule d'ADN recombinant circulaire fermée de manière covalente dans une production fermentaire par rapport au rendement d'une molécule d'ADN recombinant circulaire fermée de manière covalente dans laquelle l'insert comprenant la région homopolymère est situé à une distance de moins de 500 pb de l'origine de réplication dans la direction de réplication.

**9.** Utilisation d'une molécule d'ADN recombinant circulaire fermée de manière covalente, selon l'une quelconque des revendications 3 à 7 pour augmenter le rendement de la molécule d ADN recombinant circulaire fermée de manière covalente dans une production fermentaire par rapport au rendement d'une molécule d'ADN recombinant circulaire fermée de manière covalente dans laquelle l'insert comprenant la région homopolymère est orienté de sorte que la direction de transcription de l'insert est opposée à la direction de réplication de l'origine de réplication.

**10.** Procédé de production fermentaire d'une molécule d'ADN recombinant fermée de manière covalente comprenant les étapes consistant à :

(a) préparer un microorganisme comprenant la molécule d'ADN recombinant circulaire fermée de manière covalente selon les revendications 1 à 7 ;
(b) fermenter le microorganisme de l'étape (a), éventuellement comprenant en outre une étape de
(c) isoler la molécule d'ADN recombinant circulaire fermée de manière covalente du microorganisme de l'étape (b).

**11.** Procédé selon la revendication 10, dans lequel le microorganisme est une bactérie contenant un plasmide d'ADN à haut nombre de copies inductible à la température recombinant circulaire fermé de manière covalente et l'étape (b) comprend les étapes suivantes consistant à :

(i) faire croître les bactéries contenant un plasmide à haut nombre de copies inductible à la température recombinant circulaire fermé de manière covalente dans la plage de 25 °C à 32 °C, de préférence à environ 30 °C, durant la phase de croissance de la phase de cuvée alimentée le substrat étant préparé à une vitesse de telle manière que la vitesse de croissance est m = 0,05 à 0,3 heure$^{-1}$ durant la phase de cuvée alimentée,
(ii) induire la production dudit plasmide d'ADN après la phase de croissance en augmentant la température à 36 °C à 45 °C, de préférence à environ 42 °C ; et
(iii) poursuivre la fermentation à la température appliquée (ii) pour accumuler ledit plasmide d'ADN.

**12.** Procédé selon l'une quelconque des revendications 10 ou 11, dans lequel le rendement de la molécule d'ADN recombinant circulaire fermée de manière covalente selon les revendications 1 à 7 est augmenté par rapport au rendement d'une molécule d'ADN recombinant circulaire fermée de manière covalente dans laquelle l'insert comprenant la région homopolymère est situé à une distance de moins de 500 pb de l'origine de réplication dans la direction de réplication ou par rapport au rendement d'une molécule d'ADN recombinant circulaire fermée de manière covalente dans laquelle l'insert comprenant la région homopolymère est orienté de sorte que la direction de transcription de l'insert est opposée à la direction de réplication de l'origine de réplication.

**13.** Procédé selon l'une quelconque des revendications 10 à 12, dans lequel la séquence de région homopolymère de la molécule d'ADN recombinant circulaire fermée de manière covalente reste stable durant la production de fermentation.

**14.** Procédé pour améliorer le rendement d'une molécule d'ADN recombinant circulaire fermée de manière covalente comprenant les étapes suivantes consistant à :

(a) préparer une molécule d'ADN recombinant circulaire fermée de manière covalente ayant

- une origine de réplication bactérienne, et
- un insert comprenant une séquence homopolymère comprenant un segment d'au moins 15 nucléotides identiques, la région homopolymère étant située à une distance de moins de 500 pb de l'origine de réplication dans la direction de réplication ;

(b) modifier la molécule d'ADN recombinant circulaire fermée de manière covalente de (a)

de sorte que l'insert comprenant une séquence de région homopolymère est situé à une distance d'au moins 500 pb de l'origine de réplication bactérienne dans la direction de réplication.

**15.** Procédé pour améliorer le rendement d'une molécule d'ADN recombinant circulaire fermée de manière covalente comprenant les étapes suivantes consistant à :

(a) préparer une molécule d'ADN recombinant circulaire fermée de manière covalente ayant

- une origine de réplication bactérienne, et
- un insert comprenant une séquence homopolymère comprenant au moins une séquence homopolymère comprenant un segment d'au moins 15 nucléotides identiques, l'insert comprenant la séquence de région homopolymère étant orienté de sorte que la direction de transcription de l'insert est opposée à la direction de réplication de l'origine bactérienne de réplication et la région homopolymère étant située à une distance d'au moins 500 pb de l'origine de réplication bactérienne dans la direction de réplication ;

(b) modifier la molécule d'ADN recombinant circulaire fermée de manière covalente de sorte que l'insert comprenant une région homopolymère est orienté de sorte que la direction de transcription de l'insert est la même que la direction de réplication de l'origine de réplication bactérienne.

**16.** Utilisation de la molécule d'ADN recombinant circulaire fermée de manière covalente selon les revendications 1 à 7 pour la transcription *in vitro* de l'ARN.

Figure 1

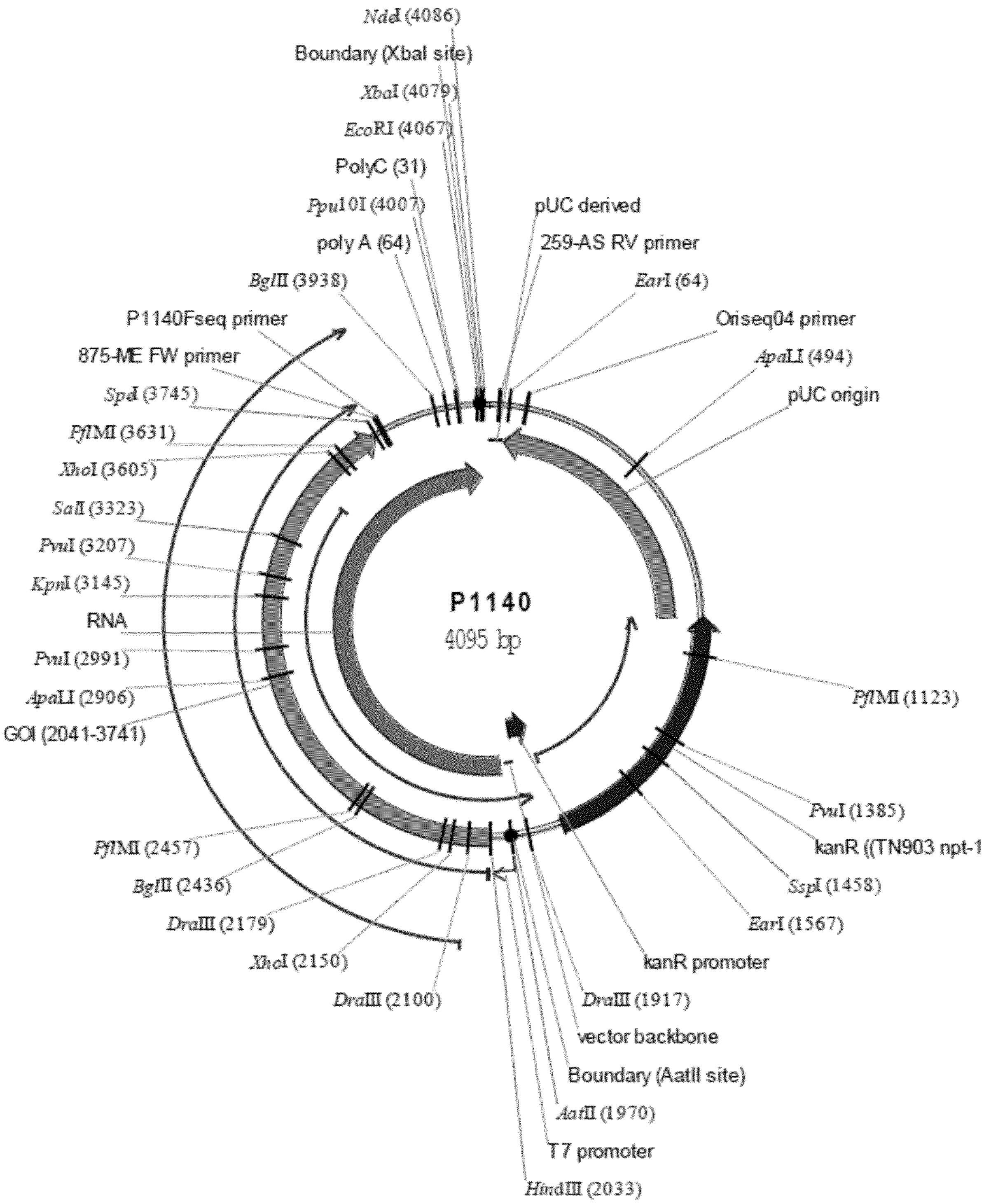
NdeI (4086)
Boundary (XbaI site)
XbaI (4079)
EcoRI (4067)
PolyC (31)
Ppu10I (4007)
poly A (64)
BglII (3938)
P1140Fseq primer
875-ME FW primer
SpeI (3745)
PflMI (3631)
XhoI (3605)
SalI (3323)
PvuI (3207)
KpnI (3145)
RNA
PvuI (2991)
ApaLI (2906)
GOI (2041-3741)
PflMI (2457)
BglII (2436)
DraIII (2179)
XhoI (2150)
DraIII (2100)
pUC derived
259-AS RV primer
EarI (64)
Oriseq04 primer
ApaLI (494)
pUC origin
P1140
4095 bp
PflMI (1123)
PvuI (1385)
kanR ((TN903 npt-1
SspI (1458)
EarI (1567)
kanR promoter
DraIII (1917)
vector backbone
Boundary (AatII site)
AatII (1970)
T7 promoter
HindIII (2033)

Figure 2

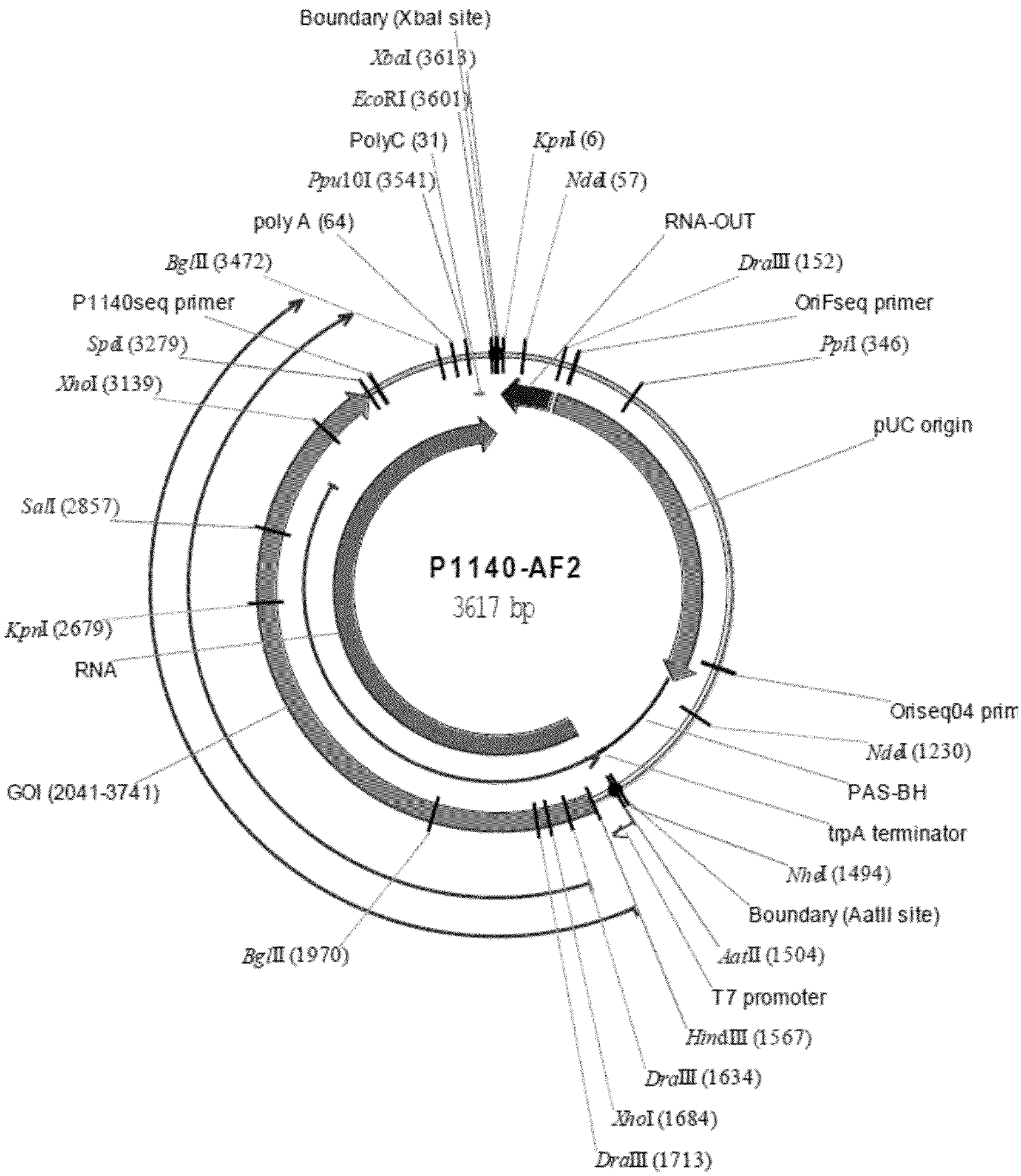

P1140-AF2
3617 bp
Boundary (XbaI site)
XbaI (3613)
EcoRI (3601)
PolyC (31)
KpnI (6)
Ppu10I (3541)
NdeI (57)
poly A (64)
RNA-OUT
BglII (3472)
DraIII (152)
P1140seq primer
OriFseq primer
SpeI (3279)
PpiI (346)
XhoI (3139)
pUC origin
SalI (2857)
KpnI (2679)
RNA
Oriseq04 prim
NdeI (1230)
PAS-BH
GOI (2041-3741)
trpA terminator
NheI (1494)
Boundary (AatII site)
BglII (1970)
AatII (1504)
T7 promoter
HindIII (1567)
DraIII (1634)
XhoI (1684)
DraIII (1713)

Figure 3

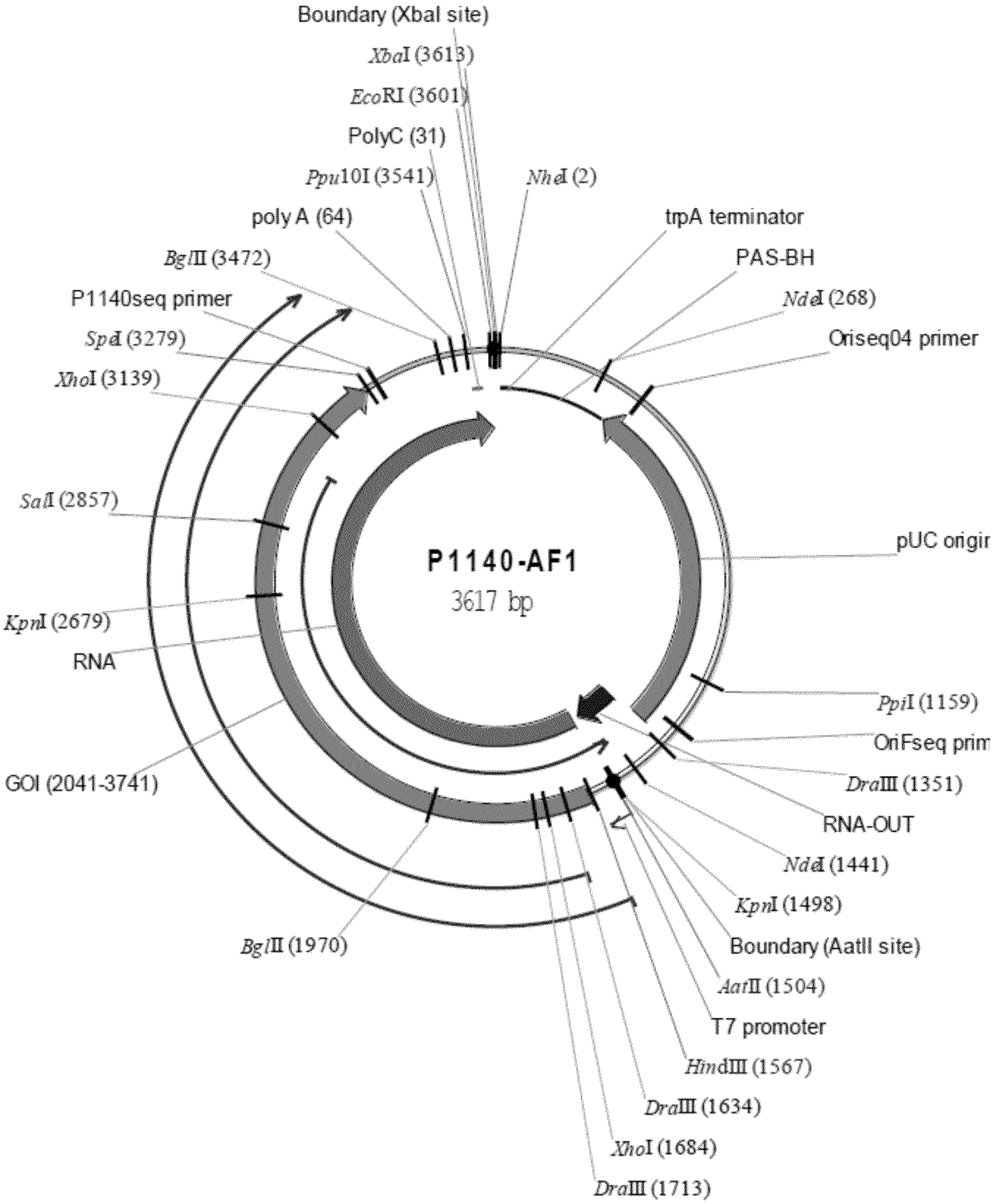
Boundary (XbaI site)
XbaI (3613)
EcoRI (3601)
PolyC (31)
Ppu10I (3541)
NheI (2)
poly A (64)
trpA terminator
BglII (3472)
PAS-BH
P1140seq primer
NdeI (268)
SpeI (3279)
Oriseq04 primer
XhoI (3139)
SalI (2857)
P1140-AF1
3617 bp
pUC origin
KpnI (2679)
RNA
PpiI (1159)
OriFseq prim
DraIII (1351)
GOI (2041-3741)
RNA-OUT
NdeI (1441)
KpnI (1498)
BglII (1970)
Boundary (AatII site)
AatII (1504)
T7 promoter
HindIII (1567)
DraIII (1634)
XhoI (1684)
DraIII (1713)

Figure 4

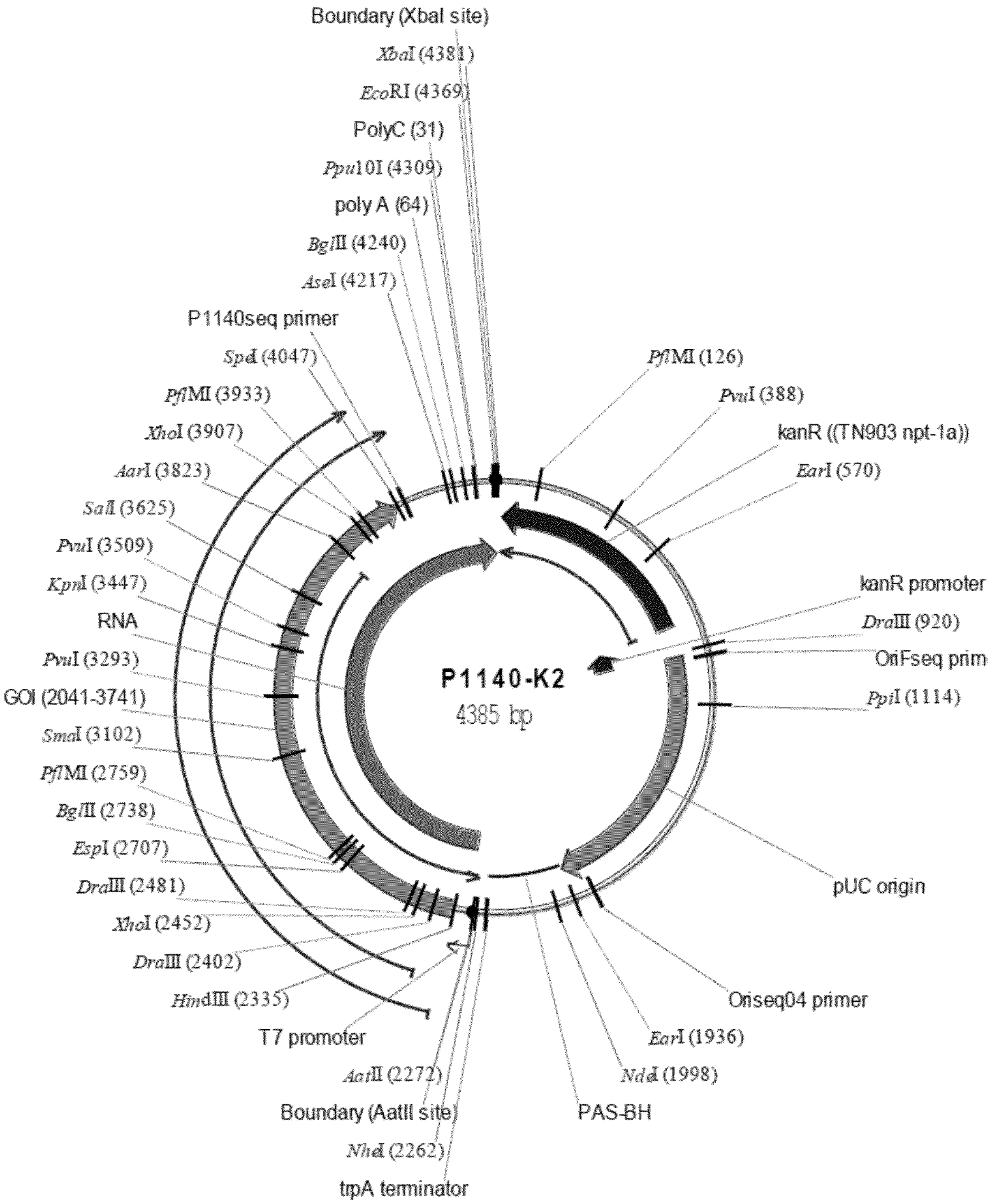
Boundary (XbaI site)
XbaI (4381)
EcoRI (4369)
PolyC (31)
Ppu10I (4309)
poly A (64)
BglII (4240)
AseI (4217)
P1140seq primer
SpeI (4047)
PflMI (3933)
XhoI (3907)
AarI (3823)
SalI (3625)
PvuI (3509)
KpnI (3447)
RNA
PvuI (3293)
GOI (2041-3741)
SmaI (3102)
PflMI (2759)
BglII (2738)
EspI (2707)
DraIII (2481)
XhoI (2452)
DraIII (2402)
HindIII (2335)
T7 promoter
AatII (2272)
Boundary (AatII site)
NheI (2262)
trpA terminator
PflMI (126)
PvuI (388)
kanR ((TN903 npt-1a))
EarI (570)
kanR promoter
DraIII (920)
OriFseq prim
PpiI (1114)
P1140-K2
4385 bp
pUC origin
Oriseq04 primer
EarI (1936)
NdeI (1998)
PAS-BH

Figure 5

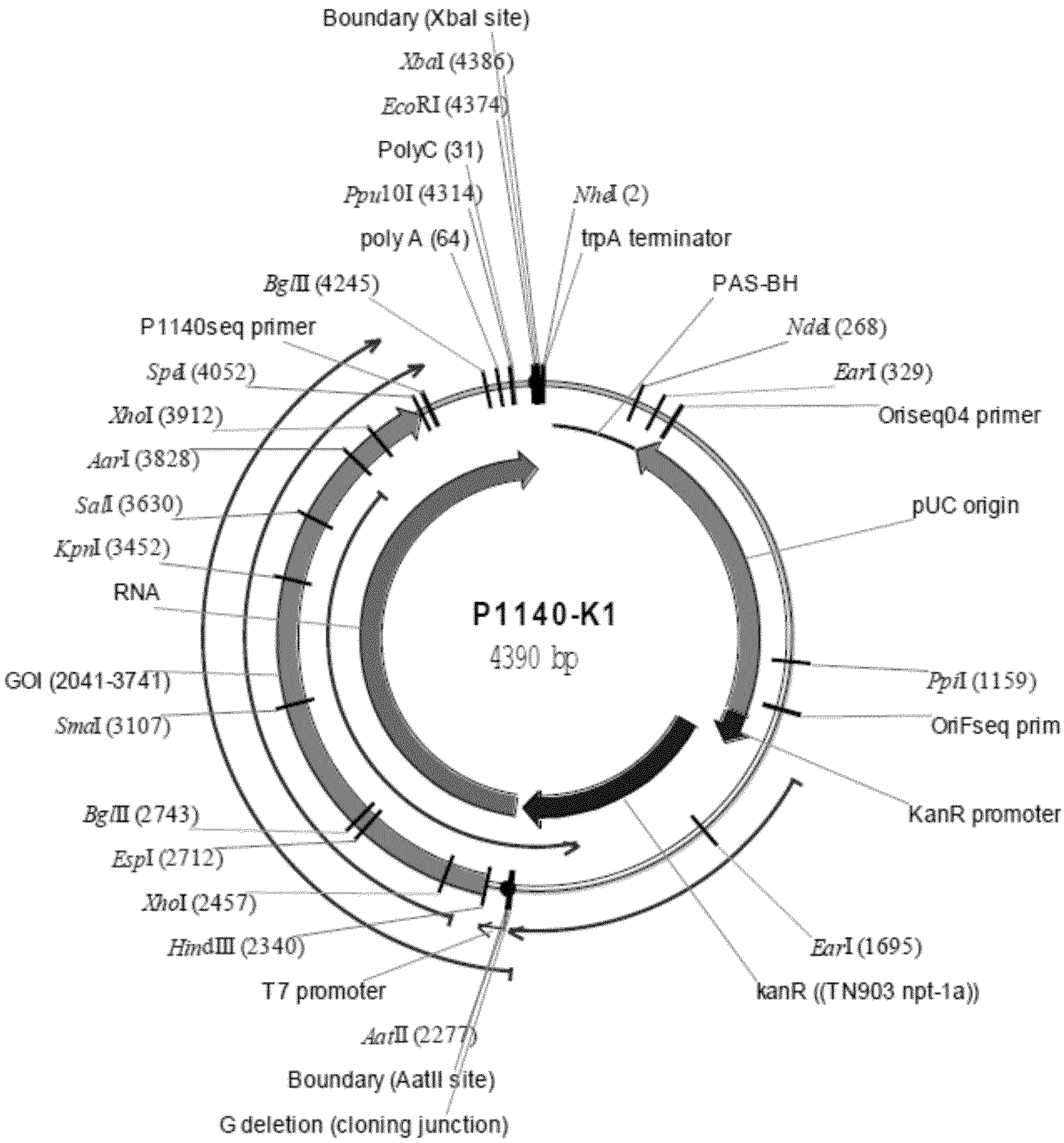
Boundary (XbaI site)
XbaI (4386)
EcoRI (4374)
PolyC (31)
Ppu10I (4314)
NheI (2)
poly A (64)
trpA terminator
BglII (4245)
PAS-BH
P1140seq primer
NdeI (268)
SphI (4052)
EarI (329)
XhoI (3912)
Oriseq04 primer
AarI (3828)
SalI (3630)
pUC origin
KpnI (3452)
RNA
P1140-K1
4390 bp
GOI (2041-3741)
PpiI (1159)
SmaI (3107)
OriFseq prim
BglII (2743)
KanR promoter
EspI (2712)
XhoI (2457)
HindIII (2340)
EarI (1695)
T7 promoter
kanR ((TN903 npt-1a))
AatII (2277)
Boundary (AatII site)
G deletion (cloning junction)

Figure 6

GC-optimized mRNA encoding H1N1(Netherlands2009)-HA

GGGGCGCUGCCUACGGAGGUGGCAGCCAUCUCCUUCUCGGCAUCAAGCUUACCAUGAAGGCC
AUCCUGGUGGUCCUCCUGUACACCUUCGCCACCGCGAACGCCGACACGCUGUGCAUCGGCUA
CCACGCCAACAACAGCACCGACACCGUGGACACCGUGCUCGAGAAGAACGUCACGGUGACCC
ACUCCGUGAACCUGCUGGAGGACAAGCACAACGGGAAGCUCUGCAAGCUGCGGGGCGUCGCC
CCGCUGCACCUCGGGAAGUGCAACAUCGCCGGCUGGAUCCUGGGGAACCCGGAGUGCGAGAG
CCUGUCCACCGCGAGCUCCUGGAGCUACAUCGUGGAGACCUCCAGCUCCGACAACGGCACGU
GCUACCCCGGCGACUUCAUCGACUACGAGGAGCUCCGCGAGCAGCUGAGCUCCGUGAGCUCC
UUCGAGCGGUUCGAGAUCUUCCCCAAGACCAGCUCCUGGCCCAACCACGACAGCAACAAGGG
GGUCACCGCCGCCUGCCCGCACGCCGGCGCGAAGUCCUUCUACAAGAACCUGAUCUGGCUCG
UGAAGAAGGGGAACAGCUACCCCAAGCUGUCCAAGAGCUACAUCAACGACAAGGGCAAGGAG
GUGCUGGUCCUCUGGGGGAUCCACCACCCCAGCACCUCCGCCGACCAGCAGAGCCUGUACCA
GAACGCCGACGCCUACGUGUUCGUGGGCUCCAGCCGCUACUCCAAGAAGUUCAAGCCCGAGA
UCGCCAUCCGGCCGAAGGUCCGCGACCAGGAGGGCCGGAUGAACUACUACUGGACGCUGGUG
GAGCCCGGGGACAAGAUCACCUUCGAGGCGACCGGCAACCUCGUGGUCCCCCGCUACGCCUU
CGCCAUGGAGCGGAACGCCGGGAGCGGCAUCAUCAUCUCCGACACCCCCGUGCACGACUGCA
ACACGACCUGCCAGACCCCGAAGGGCGCCAUCAACACCAGCCUGCCCUUCCAGAACAUCCAC
CCCAUCACGAUCGGGAAGUGCCCCAAGUACGUGAAGUCCACCAAGCUGCGCCUCGCGACCGG
CCUGCGGAACGUCCCGAGCAUCCAGUCCCGCGGGCUGUUCGGCGCCAUCGCCGGGUUCAUCG
AGGGCGGCUGGACCGGGAUGGUGGACGGCUGGUACGGGUACCACCACCAGAACGAGCAGGGC
AGCGGGUACGCCGCCGACCUCAAGUCCACGCAGAACGCGAUCGACGAGAUCACCAACAAGGU
GAACAGCGUCAUCGAGAAGAUGAACACCCAGUUCACCGCCGUGGGCAAGGAGUUCAACCACC
UGGAGAAGCGGAUCGAGAACCUGAACAAGAAGGUCGACGACGGCUUCCUCGACAUCUGGACG
UACAACGCCGAGCUGCUGGUGCUCCUGGAGAACGAGCGCACCCUGGACUACCACGACUCCAA
CGUGAAGAACCUCUACGAGAAGGUCCGGAGCCAGCUGAAGAACAACGCCAAGGAGAUCGGGA
ACGGCUGCUUCGAGUUCUACCACAAGUGCGACAACACCUGCAUGGAGUCCGUGAAGAACGGG
ACCUACGACUACCCCAAGUACAGCGAGGAGGCCAAGCUGAACCGCGAGGAGAUCGACGGCGU
GAAGCUCGAGUCCACGCGGAUCUACCAGAUCCUGGCGAUCUACAGCACCGUCGCCAGCUCCC
UGGUGCUCGUGGUCAGCCUGGGGGCCAUCUCCUUCUGGAUGUGCAGCAACGGCUCCCUGCAG
UGCCGCAUCUGCAUCUGACCACUAGUGCAUCACAUUUAAAAGCAUCUCAGCCUACCAUGAGA
AUAAGAGAAAGAAAAUGAAGAUCAAUAGCUUAUUCAUCUCUUUUUCUUUUUCGUUGGUGUAA
AGCCAACACCCUGUCUAAAAAACAUAAAUUUCUUUAAUCAUUUUGCCUCUUUUCUCUGUGCU
UCAAUUAAUAAAAAAUGGAAAGAACCUAGAUCUAAAAAAAAAAAAAAAAAAAAAAAAAAAAA
AAAAAAAAAAAAAAAAAAAAAAAAAAAAAAAAAAAUGCAUCCCCCCCCCCCCCCCCCCCCCC
CCCCCCCCCAAAGGCUCUUUUCAGAGCCACCAGAAUU

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013052523 A
- WO 2015089511 A
- WO 2012019780 A
- EP 1781800 B1
- EP 1781800 A
- EP 2015068398 W


### Non-patent literature cited in the description


- **BOCZKOWSKI et al.** *Cancer Research,* 2000, vol. 60, 1028-1034
- **FOTIN-MLECZEK et al.** *J. Gene Med.,* 2012, vol. 14 (6), 428-439
- **KARIKO et al.** *Mol. Ther.,* 2012, vol. 20 (5), 948-953
- **KORMANN et al.** *Nat. Biotechnol.,* 2012, vol. 29 (2), 154-157
- **HEIDENREICH et al.** *Int J Cancer,* 21 December 2014
- **ESTELLER.** *Nat. Rev. Genet.,* 2011, vol. 15 (12), 861-74
- **MEYER, S. ; C. TEMME.** *Crit Rev Biochem Mol Biol,* 2004, vol. 39 (4), 197-126
- **DUBIN et al.** A modified gateway cloning strategy for overexpressing tagged proteins in plants. *Plant methods, Biomed Central,* 22 January 2008, vol. 4 (1
- **ZIBERT et al.** Infectious foot-and-mouth disease virus derived from a cloned full-length cDNA. *Journal of virology,* 1990, vol. 64 (6
- **LAHIJANI et al.** High-yield production of pBR322-derived plasmids intended for human gene therapy by employing a termperature-controllable point mutation. *Human gene therapy, May Ann Liebert,* 20 October 1996, vol. 7 (7
- **BERT et al.** Generation of an improved luciferase reporter gene plasmid that employs a novel mechanism for high-copy replication. *Plasmid,* 01 September 2000, vol. 44 (2
- Impact of targeted vector design on Co/E1 plasmid replication. **GRABHERR ; BAYER.** Trends in biotechnology. Elsevier publications, 01 June 2002, vol. 20
- Improved cloning vectors and transformation procedure for Lactococcus lactis. **WELLS et al.** Journal of applied bacteriology. Blackwell Publishing LTD, 01 January 1993, vol. 74
- **TOMIZAWA.** Control of ColEl plasmid replication: binding of RNA I to RNA II and inhibition of primer formation. *Cell,* 10 October 1986, vol. 47 (1
- **CONRAD ; CAMPBELL.** Characterization of an improved in vitro DNA replication system for Escherichia coli plasmids. *Nucleic Acids Research,* 01 January 1979, vol. 6 (10
- **SOLAR et al.** Replication and control of circular bacterial plasmids. *Microbiology and molecular biology reviews,* 01 June 1998, vol. 62 (2
- **SAMBROOK et al.** Molecular Cloning: A Laboratory Manual. Cold Spring Harbor Laboratory Press, 1989
- **ROTH ; BREAKER.** *Annu Rev Biochem,* 2009, vol. 78, 305-334
- **SETO et al.** *Molecular Cell,* 2007, vol. 26 (5), 603-609
- **SIOMI et al.** *Nat. Rev. Mol. Cell. Biol.,* 2011, vol. 12, 246-258
- **GEALL et al.** *Semin. Immunol.,* 2013, vol. 25 (2), 152-159
- **BRUNELLE et al.** *Methods Enzymol.,* 2013, vol. 530, 101-14
- **LUKE J et al.** *Vaccine,* 30 October 2009, vol. 27 (46), 6454-9

SEQUENCE LISTING

<110> CureVac GmbH

<120> Method for increasing replication of a circular DNA molecule

<130> C 9828WO2/MH

<150> PCT/EP2015/068398
<151> 2015-08-10

<160> 17

<170> PatentIn version 3.5

<210> 1
<211> 100
<212> DNA
<213> Artificial Sequence

<220>
<223> poly(A) and poly(c) sequence

<400> 1

```
aaaaaaaaaa aaaaaaaaaa aaaaaaaaaa aaaaaaaaaa aaaaaaaaaa aaaaaaaaaa        60

aaaatgcatc cccccccccc cccccccccc cccccccccc                              100
```

<210> 2
<211> 2083
<212> RNA
<213> Artificial Sequence

<220>
<223> GC-optimized mRNA H1N1

<400> 2

```
gggcgcugc cuacggaggu ggcagccauc uccuucucgg caucaagcuu accaugaagg 60
ccauccuggu gguccuccug uacaccuucg ccaccgcgaa cgccgacacg cugugcaucg 120
gcuaccacgc caacaacagc accgacaccg uggacaccgu gcucgagaag aacgucacgg 180
ugacccacuc cgugaaccug cuggaggaca agcacaacgg gaagcucugc aagcugcggg 240
gcgucgcccc gcugcaccuc gggaagugca acaucgccgg cuggauccug gggaacccgg 300
agugcgagag ccuguccacc gcgagcuccu ggagcuacau cguggagacc uccagcuccg 360
acaacggcac gugcuacccc ggcgacuuca ucgacuacga ggagcuccgc gagcagcuga 420
gcuccgugag cuccuucgag cgguucgaga ucuuccccaa gaccagcucc uggcccaacc 480
acgacagcaa caagggggguc accgccgccu gcccgcacgc cggcgcgaag uccuucuaca 540
agaaccugau cuggcucgug aagaagggga acagcuaccc caagcugucc aagagcuaca 600
ucaacgacaa gggcaaggag gugcuggucc ucugggggau ccaccacccc agcaccuccg 660
ccgaccagca gagccuguac cagaacgccg acgccuacgu guucgugggc uccagccgcu 720
acuccaagaa guucaagccc gagaucgcca uccggccgaa gguccgcgac caggagggcc 780
```

ggaugaacua cuacuggacg cugguggagc ccggggacaa gaucaccuuc gaggcgaccg 840
gcaaccucgu gguccccgc uacgccuucg ccauggagcg gaacgccggg agcggcauca 900
ucaucuccga caccccgug cacgacugca acacgaccug ccagaccccg aagggcgcca 960
ucaacaccag ccugcccuuc cagaacaucc accccaucac gaucgggaag ugccccaagu 1020
acgugaaguc caccaagcug cgccucgcga ccggccugcg gaacgucccg agcauccagu 1080
cccgcgggcu guucggcgcc aucgccgggu ucaucgaggg cggcuggacc gggauggugg 1140
acggcuggua cggguaccac caccagaacg agcagggcag cggguacgcc gccgaccuca 1200
aguccacgca gaacgcgauc gacgagauca ccaacaaggu gaacagcguc aucgagaaga 1260
ugaacaccca guucaccgcc gugggcaagg aguucaacca ccuggagaag cggaucgaga 1320
accugaacaa gaaggucgac gacggcuucc ucgacaucug gacguacaac gccgagcugc 1380
uggugcuccu ggagaacgag cgcacccugg acuaccacga cuccaacgug aagaaccucu 1440
acgagaaggu ccggagccag cugaagaaca acgccaagga gaucgggaac ggcugcuucg 1500
aguucuacca caagugcgac aacaccugca uggaguccgu gaagaacggg accuacgacu 1560
accccaagua cagcgaggag gccaagcuga accgcgagga gaucgacggc gugaagcucg 1620
aguccacgcg gaucuaccag auccuggcga ucuacagcac cgucgccagc ucccuggugc 1680
ucguggucag ccugggggcc aucuccuucu ggaugugcag caacggcucc cugcagugcc 1740
gcaucugcau cugaccacua gugcaucaca uuuaaaagca ucucagccua ccaugagaau 1800
aagagaaaga aaaugaagau caauagcuua uucaucucuu uuucuuuuuc guugguguaa 1860
agccaacacc cugucuaaaa aacauaaauu ucuuuaauca uuuugccucu uuucucugug 1920
cuucaauuaa uaaaaaaugg aaagaaccua gaucuaaaaa aaaaaaaaaa aaaaaaaaaa 1980
aaaaaaaaaa aaaaaaaaaa aaaaaaaaaa aaaaaaaaau gcaucccccc cccccccccc 2040
cccccccccc cccccaaagg cucuuuucag agccaccaga auu 2083

<210> 3
<211> 285
<212> DNA
<213> Artificial Sequence

<220>
<223> PAS-BH

<400> 3

ctgcctcgcg cgtttcggtg atgacggtga aaacctctga cacatgcagc tcccggagac 60

ggtcacagct tgtctgtaag cggatgccgg gagcagacaa gcccgtcagg gcgcgtcagc 120

gggtgttggc gggtgtcggg gcgcagccat gacccagtca cgtagcgata gcggagtgta 180

tactggctta actatgcggc atcagagcag attgtactga gagtgcacca tatgcggtgt 240

gaaataccgc acagatgcgt aaggagaaaa taccgcatca ggcgc 285

<210> 4
<211> 1520
<212> DNA
<213> Artificial Sequence

<220>
<223> P1140-AF-backbone

<400> 4

ggtacctaat gatttttatc aaaatcatta agttaaggta gatacacatc ttgtcatatg 60
atcaaatggt ttcgccaaaa atcaataatc agacaacaag atgtgcgaac tcgatatttt 120
acacgactct ctttaccaat tctaccacaa cgtggtttgc aggagtcagg caactatgga 180
tgaacgaaat agacagatcg ctgagatagg tgcctcactg attaagcatt ggtaactgtc 240
agaccaagtt tactcatata tactttagat tgatttaaaa cttcattttt aatttaaaag 300
gatctaggtg aagatccttt ttgataatct catgaccaaa atcccttaac gtgagttttc 360
gttccactga gcgtcagacc ccgtagaaaa gatcaaagga tcttcttgag atcctttttt 420
tctgcgcgta atctgctgct tgcaaacaaa aaaaccaccg ctaccagcgg tggtttgttt 480
gccggatcaa gagctaccaa ctctttttcc gaaggtaact ggcttcagca gagcgcagat 540
accaaatact gttcttctag tgtagccgta gttaggccac cacttcaaga actctgtagc 600
accgcctaca tacctcgctc tgctaatcct gttaccagtg gctgctgcca gtggcgataa 660
gtcgtgtctt accgggttgg actcaagacg atagttaccg gataaggcgc agcggtcggg 720
ctgaacgggg ggttcgtgca cacagcccag cttggagcga acgacctaca ccgaactgag 780
atacctacag cgtgagctat gagaaagcgc cacgcttccc gaagggagaa aggcggacag 840
gtatccggta agcggcaggg tcggaacagg agagcgcacg agggagcttc caggggggaaa 900
cgcctggtat ctttatagtc ctgtcgggtt tcgccacctc tgacttgagc gtcgattttt 960
gtgatgctcg tcaggggggc ggagcctatg gaaaaacgcc agcaacgcgg cctttttacg 1020
gttcctggcc ttttgctggc cttttgctca catgttcttt cctgcgttat cccctgattc 1080
tgtggataac cgtattaccg cctttgagtg agctgatacc gctcgccgca gccgaacgac 1140
cgagcgcagc gagtcagtga gcgaggaagc ggaagagcgc ctgatgcggt attttctcct 1200
tacgcatctg tgcggtattt cacaccgcat atggtgcact ctcagtacaa tctgctctga 1260
tgccgcatag ttaagccagt atacactccg ctatcgctac gtgactgggt catggctgcg 1320
ccccgacacc cgccaacacc cgctgacgcg ccctgacggg cttgtctgct cccggcatcc 1380
gcttacagac aagctgtgac cgtctccggg agctgcatgt gtcagaggtt ttcaccgtca 1440
tcaccgaaac gcgcgaggca gcctaagaaa aaaagcccg ctcattaggc gggctagcga 1500
cgtctaatac gactcactat 1520

<210> 5
<211> 2284
<212> DNA
<213> Artificial Sequence

<220>
<223> P1140-K-backbone

<400> 5

gaaaaactca tcgagcatca aatgaaactg caatttattc atatcaggat tatcaatacc 60
atatttttga aaaagccgtt tctgtaatga aggagaaaac tcaccgaggc agttccatag 120
gatggcaaga tcctggtatc ggtctgcgat tccgactcgt ccaacatcaa tacaacctat 180
caatttcccc tcgtcaaaaa taaggttatc aagtgagaaa tcaccatgag tgacgactga 240
atccggtgag aatggcaaaa gtttgtgcat ttctttccag acttgttcaa caggccagcc 300
attacgctcg tcatcaaaat cactcgcatc aaccaaaccg ttattcattc gtgattgcgc 360
ctgagcgaga cgaaatacgc gatcgctgtt aaaaggacaa ttacaaacag gaatcgaatg 420
taaccggcgc aggaacactg ccagcgcatc aacaatattt tcacctgaat caggatattc 480
ttctaatacc tggaaggctg ttttcccagg gatcgcagtg gtgagtaacc aagcatcatc 540
aggagtacgg ataaaatgct tgatggtcgg aagaggcata aattccgtca gccagtttag 600
tctgaccatc tcatctgtaa catcattggc aacgctacct ttgccatgtt tcagaaacaa 660
ctctggcgca tcgggcttcc catacaatcg gtagattgtc gcacctgatt gcccgacatt 720
atcgcgagcc catttatacc catataaatc agcatccatg ttggaattta atcgcggcct 780
agagcaagac gtttcccgtt gaatatggct cataacaccc cttgtattac tgtttatgta 840
agcagacagt tttattgttc atgatgatat atttttatct tgtgcaatgt aacatcagag 900
attttgagac acaacgtggt ttgcaggagt caggcaacta tggatgaacg aaatagacag 960
atcgctgaga taggtgcctc actgattaag cattggtaac tgtcagacca agtttactca 1020
tatatacttt agattgattt aaaacttcat ttttaattta aaaggatcta ggtgaagatc 1080
ctttttgata atctcatgac caaaatccct taacgtgagt tttcgttcca ctgagcgtca 1140
gaccccgtag aaaagatcaa aggatcttct tgagatcctt tttttctgcg cgtaatctgc 1200
tgcttgcaaa caaaaaaacc accgctacca gcggtggttt gtttgccgga tcaagagcta 1260
ccaactcttt ttccgaaggt aactggcttc agcagagcgc agataccaaa tactgttctt 1320
ctagtgtagc cgtagttagg ccaccacttc aagaactctg tagcaccgcc tacatacctc 1380
gctctgctaa tcctgttacc agtggctgct gccagtggcg ataagtcgtg tcttaccggg 1440
ttggactcaa gacgatagtt accggataag gcgcagcggt cgggctgaac ggggggttcg 1500
tgcacacagc ccagcttgga gcgaacgacc tacaccgaac tgagatacct acagcgtgag 1560

ctatgagaaa gcgccacgct tcccgaaggg agaaaggcgg acaggtatcc ggtaagcggc 1620

agggtcggaa caggagagcg cacgagggag cttccagggg gaaacgcctg gtatctttat 1680

agtcctgtcg ggtttcgcca cctctgactt gagcgtcgat ttttgtgatg ctcgtcaggg 1740

gggcggagcc tatggaaaaa cgccagcaac gcggcctttt tacggttcct ggccttttgc 1800

tggccttttg ctcacatgtt ctttcctgcg ttatcccctg attctgtgga taaccgtatt 1860

accgcctttg agtgagctga taccgctcgc cgcagccgaa cgaccgagcg cagcgagtca 1920

gtgagcgagg aagcggaaga gcgcctgatg cggtattttc tccttacgca tctgtgcggt 1980

atttcacacc gcatatggtg cactctcagt acaatctgct ctgatgccgc atagttaagc 2040

cagtatacac tccgctatcg ctacgtgact gggtcatggc tgcgccccga cacccgccaa 2100

cacccgctga cgcgccctga cgggcttgtc tgctcccggc atccgcttac agacaagctg 2160

tgaccgtctc cgggagctgc atgtgtcaga ggttttcacc gtcatcaccg aaacgcgcga 2220

ggcagcctaa gaaaaaaaag cccgctcatt aggcgggcta gcgacgtcta atacgactca 2280

ctat 2284

<210> 6
<211> 1015
<212> DNA
<213> Artificial Sequence

<220>
<223> pUC origin

<400> 6

```
ggagtcaggc aactatggat gaacgaaata gacagatcgc tgagataggt gcctcactga      60
ttaagcattg gtaactgtca gaccaagttt actcatatat actttagatt gatttaaaac     120
ttcattttta atttaaaagg atctaggtga agatcctttt tgataatctc atgaccaaaa     180
tcccttaacg tgagttttcg ttccactgag cgtcagaccc cgtagaaaag atcaaaggat     240
cttcttgaga tccttttttt ctgcgcgtaa tctgctgctt gcaaacaaaa aaaccaccgc     300
taccagcggt ggtttgtttg ccggatcaag agctaccaac tctttttccg aaggtaactg     360
gcttcagcag agcgcagata ccaaatactg ttcttctagt gtagccgtag ttaggccacc     420
acttcaagaa ctctgtagca ccgcctacat acctcgctct gctaatcctg ttaccagtgg     480
ctgctgccag tggcgataag tcgtgtctta ccgggttgga ctcaagacga tagttaccgg     540
ataaggcgca gcggtcgggc tgaacggggg gttcgtgcac acagcccagc ttggagcgaa     600
cgacctacac cgaactgaga tacctacagc gtgagctatg agaaagcgcc acgcttcccg     660
aagggagaaa ggcggacagg tatccggtaa gcggcagggt cggaacagga gagcgcacga     720
gggagcttcc agggggaaac gcctggtatc tttatagtcc tgtcgggttt cgccacctct     780
gacttgagcg tcgatttttg tgatgctcgt caggggggcg gagcctatgg aaaaacgcca     840
gcaacgcggc ctttttacgg ttcctggcct tttgctggcc ttttgctcac atgttctttc     900
ctgcgttatc ccctgattct gtggataacc gtattaccgc ctttgagtga gctgataccg     960
ctcgccgcag ccgaacgacc gagcgcagcg agtcagtgag cgaggaagcg gaaga         1015
```

<210> 7
<211> 589
<212> DNA
<213> Artificial Sequence

<220>
<223> pBR322 origin

<400> 7

ttttccatag gctccgcccc cctgacgagc atcacaaaaa tcgacgctca agtcagaggt 60

ggcgaaaccc gacaggacta taaagatacc aggcgtttcc ccctggaagc tccctcgtgc 120

gctctcctgt tccgaccctg ccgcttaccg gatacctgtc cgcctttctc ccttcgggaa 180

gcgtggcgct ttctcatagc tcacgctgta ggtatctcag ttcggtgtag gtcgttcgct 240

ccaagctggg ctgtgtgcac gaaccccccg ttcagcccga ccgctgcgcc ttatccggta 300

actatcgtct tgagtccaac ccggtaagac acgacttatc gccactggca gcagccactg 360

gtaacaggat tagcagagcg aggtatgtag gcggtgctac agagttcttg aagtggtggc 420

ctaactacgg ctacactaga aggacagtat ttggtatctg cgctctgctg aagccagtta 480

ccttcggaaa aagagttggt agctcttgat ccggcaaaca aaccaccgct ggtagcggtg 540

gtttttttgt ttgcaagcag cagattacgc gcagaaaaaa aggatctca 589

<210> 8
<211> 582
<212> DNA
<213> Artificial Sequence

<220>
<223> pMB1 origin

<400> 8

gcgttgctgg cgtttttcca taggctccgc ccccctgacg agcatcacaa aaatcgacgc 60

tcaagtcaga ggtggcgaaa cccgacagga ctataaagat accaggcgtt tccccctgga 120

agctccctcg tgcgctctcc tgttccgacc ctgccgctta ccggatacct gtccgccttt 180

ctcccttcgg gaagcgtggc gctttctcat agctcacgct gtaggtatct cagttcggtg 240

taggtcgttc gctccaagct gggctgtgtg cacgaacccc ccgttcagcc cgaccgctgc 300

gccttatccg gtaactatcg tcttgagtcc aacccggtaa gacacgactt atcgccactg 360

gcagcagcca ctggtaacag gattagcaga gcgaggtatg taggcggtgc tacagagttc 420

ttgaagtggt ggcctaacta cggctacact agaaggacag tatttggtat ctgcgctctg 480

ctgaagccag ttccttcgga aaaagagttg gtagctcttg atccggcaaa caaaccaccg 540

ctggtagcgg tggttttttt gtttgcaagc agcagattac gc 582

<210> 9
<211> 602
<212> DNA
<213> Artificial Sequence

<220>
<223> ColE1 origin

<400> 9

```
aggatcttct tgagatcctt tttttctgcg cgtaatctgc tgcttgcaaa caaaaaaacc        60
accgctacca acggtggttt gtttgccgga tcaagagcta ccaactcttt ttccgaaggt       120
aactggcttc agcagagcgc agataccaaa tactgtcctt ctagtgtagc cgtagtcggg       180
ccactacttc aagaactctg tagcaccgtt tgtgccatca tcgctctgct aatccggtta       240
ccagtggctg ctgccagtgg cgttaaggcg tgccttaccg ggttggactc aagacgatag       300
ttaccggata aggcgcagcg gtcgggctga acgggggtt cgtgcacaca gcccagcttg        360
gagcgaacga cctacaccga actgagatac caacagcgtg agctatgaga aagcgccacg       420
cttcccgaag ggagaaaggc ggacaggtat ccggtaagcg gcagggtcgg aacaggagag       480
cgcacgaggg agcttccagg gggaaacgcc tggtatcttt atagtcctgt cgggtttcgc       540
cacctctgac ttgagcgtct atttttgtga tgctcgtcag gggggcggag cctatggaaa       600
aa                                                                      602
```

<210> 10
<211> 282
<212> DNA
<213> Artificial Sequence

<220>
<223> R6K_gamma origin

<400> 10

```
aagctttaaa agccttatat attctttttt ttcttataaa acttaaaacc ttagaggcta        60
tttaagttgc tgatttatat taattttatt gttcaaacat gagagcttag tacgtgaaac       120
atgagagctt agtacgttag ccatgagagc ttagtacgtt agccatgagg gtttagttcg       180
ttaaacatga gagcttagta cgttaaacat gagagcttag tacgtgaaac atgagagctt       240
agtacgtact atcaacaggt tgaactgctg atcttcagat ct                          282
```

<210> 11
<211> 532
<212> DNA
<213> Artificial Sequence

<220>
<223> R6K_beta origin

<400> 11

agatctatgg atgttgagaa tgtagtatgg ttatactgcg atgcaggata gggcaaacgc 60

cgtaaaatga cgtctttgac gttatttttc agcgcttgcc cggtgaccta cagtcggttc 120

ttgttgggag attttatgaa gtttatagta aaggatttta tcagtgataa atatgcaaag 180

gctattaaca ttttaaatga taaccttaaa gaaaactact atgtttttta tggtgtaagg 240

ttaagtgaaa ttctttttcc tgcaagtgat tatggtacag atgatttttt taaggagttt 300

gaggaaataa acaacgttac cttgctttag ttgtttttga aataaatgaa cgtgaacctg 360

tgattgtaat tggttttgat gaaataaatg gtgcgattct tatagagaaa tccggtataa 420

aggttttagt aatcgataat ctatcggcgt tgctgaccaa tgaaacattg ggtgtcttct 480

tcaagtaaca gatctagctg aaaatcgtca tcaaaactgt gcaccttatc tg 532

<210> 12
<211> 639
<212> DNA
<213> Artificial Sequence

<220>
<223> R6K alpha origin

<400> 12

tcttacttct ttgcgtagct gttaaataca gcgttgtttt gataaaatca tcattatcat 60

cgataatgct ttcttcaatt tttttatcct tactctttaa taaagcactt gctaataact 120

tcataccttt tgcaactgtc aaatttggtt catcagggta aatgctttta aggcatacta 180

acaaataatc atggtcttca tcttcaactc taaactgaat ttttttcatc ataactccca 240

acaagaaccg actgtaggtc accgggcaaa cgctgaaaaa taacgtcgaa tgacgtcatt 300

ttgcggcgtt tgccctatcc tgcatcgcag tagaaaatgc cacaactgaa attgtgcttc 360

agtatgtaca gaaatgcaaa atctgaggga tttcgtagct gaaagatcgc cagtcttcga 420

ccgtaaggat aggagttgct gtaagacctg tgcggggcgt tcgcttcgcg aacgggtctg 480

gcagggggca caagcgctgt gctgtgatat atgcaaaaga agccacccac gaacgggagg 540

gcttcggcga atcgactata gtgatctatt tacccggctg attgtcgcct tctagccctc 600

gcgggcatca tgcaaccagt gcctgaattt agttatatg 639

<210> 13
<211> 914
<212> DNA
<213> Artificial Sequence

<220>
<223> p15A origin

<400> 13

gcgctagcgg agtgtatact ggcttactat gttggcactg atgagggtgt cagtgaagtg 60

cttcatgtgg caggagaaaa aaggctgcac cggtgcgtca gcagaatatg tgatacagga 120

tatattccgc ttcctcgctc actgactcgc tacgctcggt cgttcgactg cggcgagcgg 180

aaatggctta cgaacggggc ggagatttcc tggaagatgc caggaagata cttaacaggg 240

aagtgagagg gccgcggcaa agccgttttt ccataggctc cgcccccctg acaagcatca 300

cgaaatctga cgctcaaatc agtggtggcg aaacccgaca ggactataaa gataccaggc 360

gtttccccct ggcggctccc tcgtgcgctc tcctgttcct gcctttcggt ttaccggtgt 420

cattccgctg ttatggccgc gtttgtctca ttccacgcct gacactcagt tccgggtagg 480

cagttcgctc caagctggac tgtatgcacg aaccccccgt tcagtccgac cgctgcgcct 540

tatccggtaa ctatcgtctt gagtccaacc cggaaagaca tgcaaaagca ccactggcag 600

cagccactgg taattgattt agaggagtta gtcttgaagt catgcgccgg ttaaggctaa 660

actgaaagga caagttttgg tgactgcgct cctccaagcc agttacctcg gttcaaagag 720

ttggtagctc agagaacctt cgaaaaaccg ccctgcaagg cggttttttc gttttcagag 780

caagagatta cgcgcagacc aaaacgatct caagaagatc atcttattaa tcagataaaa 840

tatttctagt tttcagtgca atttatctct tcaaatgtag cacctgaagt cagccccata 900

cgatataagt tgta 914

<210> 14
<211> 300
<212> DNA
<213> Artificial Sequence

<220>
<223> pSC101 origin

<400> 14

gcggaactga ctaaagtagt gagttataca cagggctggg atctattctt tttatctttt 60

tttattcttt ctttattcta taaattataa ccacttgaat ataaacaaaa aaaacacaca 120

aaggtctagc ggaatttaca gagggtctag cagaatttac aagttttcca gcaaaggtct 180

agcagaattt acagataccc acaactcaaa ggaaaaggac tagtaattat cattgactag 240

cccatctcaa ttggtatagt gattaaaatc acctagacca attgagatgt atgtctgaat 300

<210> 15
<211> 307
<212> DNA
<213> Artificial Sequence

<220>
<223> F1 phagemid origin

<400> 15

```
ttgttccagt ttggaacaag agtccactat taaagaacgt ggactccaac gtcaaagggc        60

gaaaaaccgt ctatcagggc gatggcccac tacgtgaacc atcaccctaa tcaagttttt       120

tggggtcgag gtgccgtaaa gcactaaatc ggaaccctaa agggagcccc cgatttagag       180

cttgacgggg aaagccggcg aacgtggcga gaaaggaagg gaagaaagcg aaaggagcgg       240

gcgctagggc gctggcaagt gtagcggtca cgctgcgcgt aaccaccaca cccgccgcgc       300

ttaatgc                                                                 307
```

<210> 16
<211> 24
<212> DNA
<213> Artificial Sequence

<220>
<223> histone stem-loop

<400> 16
caaaggctct tttcagagcc acca 24

<210> 17
<211> 24
<212> RNA
<213> Artificial Sequence

<220>
<223> histone stem loop

<400> 17
caaaggcucu uuucagagcc acca 24